# EUROPEAN PATENT SPECIFICATION

(11) **EP 1 492 519 B1**
(45) Date of publication and mention of the grant of the patent: **15.11.2006**
(21) Application number: 04757949.5
(22) Date of filing: 22.03.2004
(51) Int. Cl.: A61K 31/195, A61K 31/216, A61K 31/197, A61K 31/137, A61K 31/445, A61K 31/4725, A61P 13/00

(54) **METHODS FOR TREATING LOWER URINARY TRACT DISORDERS USING ANTIMUSCARINICS AND ALPHA-2-DELTA SUBUNIT CALCIUM CHANNEL MODULATORS**
VERFAHREN ZUR BEHANDLUNG VON ERKRANKUNGEN DER UNTEREN HARNWEGE MIT ANTIMUSKARINIKA UND MIT MODULATOREN DER ALPHA-2-DELTA UNTEREINHEIT DES KALZIUMKANALS
METHODES DE TRAITEMENT DE TROUBLES DES VOIES URINAIRES INFERIEURES UTILISANT DES DES AGENTS ANTIMUSCARINIQUES ET DES MODULATEURS DES CANAUX CALCIQUES DE LA SOUS-UNITE ALPHA-2-DELTA

(30) Priority: 21.03.2003 US 456835 P; 10.07.2003 US 486148 P; 08.10.2003 US 509570 P; 08.01.2004 US 534871 P; 27.02.2004 US 548250 P
(43) Date of publication of application: 05.01.2005
(60) Divisional application: 06010942.8
(73) Proprietor: Dynogen Pharmaceuticals, Inc., Waltham MA 02451 (US)
(72) Inventor: FRASER, Matthew, Oliver, Apex, NC 27502 (US); THOR, Karl, Bruce, Morrisville, NC 27560 (US); BURGARD, Edward, C., Chapel Hill, NC 27516 (US); BRETTMAN, Lee, R., Sudbury, MA 01776 (US); LANDAU, Steven, B., Wellesley, MA 02481 (US); RICCA, Daniel, J., Rougemont, NC 27572 (US)
(74) Representative: Bentham, Andrew
(86) International application number: PCT/US2004/008605
(87) International publication number: WO 2004/084879

(56) References cited:
- WO-A-01/01983
- WO-A-01/24792
- WO-A-01/37832
- WO-A-96/37202
- WO-A-03/000642
- WO-A-03/070237
- WO-A-20/04054560
- FIELD MARK J ET AL: "Gabapentin and the neurokinin1 receptor antagonist Cl-1021 act synergistically in two rat models of neuropathic pain." JOURNAL OF PHARMACOLOGY AND EXPERIMENTAL THERAPEUTICS, vol. 303, no. 2, November 2002 (2002-11), pages 730-735, XP002294267 ISSN: 0022-3565
- DE SARRO G ET AL: "Gabapentin potentiates the antiseizure activity of certain anticonvulsants in DBA/2 mice." EUROPEAN JOURNAL OF PHARMACOLOGY. 22 MAY 1998, vol. 349, no. 2-3, 22 May 1998 (1998-05-22), pages 179-185, XP002291581 ISSN: 0014-2999
- OBERPENNING F ET AL: "Interstitial cystitis: An update" CURRENT OPINION IN UROLOGY 2002 UNITED KINGDOM, vol. 12, no. 4, 2002, pages 321-332, XP009034871 ISSN: 0963-0643
- KATZUNG BG: "Basic & Clinical Pharmacology, Eighth edition" 2001, LANGE MEDICAL BOOKS/MCGRAW-HILL , UNITED STATES OF AMERICA , XP002291582 page 457, column 2, last paragraph - page 458, column 2, paragraph 1 page 459, column 2, paragraph 3
- FELIX R: "Voltage-dependent Ca<2+> channel [alpha]2[delta] auxiliary subunit: Structure, function and regulation" RECEPTORS AND CHANNELS 1999 NETHERLANDS, vol. 6, no. 5, 1999, pages 351-362, XP009034843 ISSN: 1060-6823

## Description

### FIELD OF THE INVENTION

The invention relates to the use of the α₂δ subunit calcium channel modulators gabapentin and pregabalin in combination with an antimuscarinic in the manufacture of medicaments for treating and/or alleviating the symptoms associated with painful and non-painful lower urinary tract disorders in normal and spinal cord injured patients.

### BACKGROUND OF THE INVENTION

Lower urinary tract disorders affect the quality of life of millions of men and women in the United States every year. Disorders of the lower urinary tract include overactive bladder, prostatitis and prostadynia, interstitial cystitis, benign prostatic hyperplasia and associated irritative or obstructive symptoms, and, in spinal cord injured patients, spastic bladder.

Overactive bladder is a treatable medical condition that is estimated to affect 17 to 20 million people in the United States. Current treatments for overactive bladder include medication, diet modification, programs in bladder training, electrical stimulation, and surgery. Currently, antimuscarinics (which are subtypes of the general class of anticholinergics) are the primary medication used for the treatment of overactive bladder. This treatment suffers from limited efficacy and side effects such as dry mouth, dry eyes, dry vagina, palpitations, drowsiness, and constipation, which have proven difficult for some individuals to tolerate.

In recent years, it has been recognized among those of skill in the art that OAB can be divided into urgency without any demonstrable loss of urine as well as urgency with loss of urine. For example, a recent study examined the impact of all OAB symptoms on the quality of life of a community-based sample of the United States population. (Liberman *et al.* (2001) *Urology* 57: 1044-1050). This study demonstrated that the group of individuals suffering from OAB without any demonstrable loss of urine have an impaired quality of life when compared with controls. Additionally, individuals with urgency alone have an impaired quality of life compared with controls.

Prostatitis and prostadynia are other lower urinary tract disorders that have been suggested to affect approximately 2-9% of the adult male population (Collins M M, *et al.,* (1998) *J. Urology,* 159: 1224-1228). Currently, there are no established treatments for prostatitis and prostadynia. Antibiotics are often prescribed, but with little evidence of efficacy. COX-2 selective inhibitors and α-adrenergic blockers and have been suggested as treatments, but their efficacy has not been established. Hot sitz baths and anticholinergic drugs have also been employed to provide some symptomatic relief.

Interstitial cystitis is another lower urinary tract disorder of unknown etiology that predominantly affects young and middle-aged females, although men and children can also be affected. Past treatments for interstitial cystitis have included the administration of antihistamines, sodium pentosanpolysulfate, dimethylsulfoxide, steroids, tricyclic antidepressants and narcotic antagonists, although these methods have generally been unsuccessful (Sant, G. R. (1989) Interstitial cystitis: pathophysiology, clinical evaluation and treatment. *Urology Annal* 3: 171-196).

Benign prostatic hyperplasia (BPH) is a non-malignant enlargement of the prostate that is very common in men over 40 years of age. Irritative symptoms of benign prostatic hyperplasia include urinary urgency, urinary frequency, and nocturia. Obstructive symptoms associated with benign prostatic hyperplasia include reduced urinary force and speed of flow. Invasive treatments for BPH include transurethral resection of the prostate, transurethral incision of the prostate, balloon dilation of the prostate, prostatic stents, microwave therapy, laser prostatectomy, transrectal high-intensity focused ultrasound therapy and transurethral needle ablation of the prostate. However, complications may arise through the use of some of these treatments, including retrograde ejaculation, impotence, postoperative urinary tract infection and some urinary incontinence. Non-invasive treatments for BPH include androgen deprivation therapy and the use of 5α-reductase inhibitors and α-adrenergic blockers. However, these treatments have proven only minimally to moderately effective for some patients.

Lower urinary tract disorders are particularly problematic for individuals suffering from spinal cord injury. Following spinal cord injury, the bladder is usually affected in one of two ways: 1) "spastic" or "reflex" bladder, in which the bladder fills with urine and a reflex automatically triggers the bladder to empty; or 2) "flaccid" or "non-reflex" bladder, in which the reflexes of the bladder muscles are absent or slowed. Treatment options for these disorders usually include intermittent catheterization, indwelling catheterization, or condom catheterization, but these methods are invasive and frequently inconvenient. Urinary sphincter muscles may also be affected by spinal cord injuries, resulting in an inability of urinary sphincter muscles to relax when the bladder contracts ("dyssynergia"). Traditional treatments for dyssynergia include medications that have been somewhat inconsistent in their efficacy or surgery.

Because existing therapies and treatments for lower urinary tract disorders and associated irritative symptoms in normal and spinal cord injured patients have limited efficacy and are associated with side effects that result in reduced patient compliance, the present invention presents a significant advantage over these treatments via increased efficacy and decreased side effects. Because detrimental side effects are lessened, the present invention also has the benefit of improving patient compliance.

### SUMMARY OF THE INVENTION

Compositions for treating and/or alleviating the symptoms associated with painful and non-painful lower urinary tract disorders in normal and spinal cord injured patients are provided. Compositions of the invention comprise the α₂δ subunit calcium channel modulators gabapentin or pregabalin in combination with an antimuscarinic. Compositions of the invention include combinations of the aforementioned compounds as well as pharmaceutically acceptable, pharmacologically active acids, salts, esters and amides thereof.

The compositions are administered in therapeutically effective amounts to a patient in need thereof for treating and/or alleviating the symptoms associated with painful and non-painful lower urinary tract disorders in normal and spinal cord injured patients. It is recognized that the compositions may be administered by any means of administration as long as an effective amount for treating and/or alleviating the symptoms associated with of painful and non-painful symptoms associated with lower urinary tract disorders in normal and spinal cord injured patients is delivered. The compositions may be formulated, for example, for sustained, continuous, or as-needed administration. Accordingly, the present invention also provides the use of an α₂δ subunit calcium channel modulator which is gabapentin or pregabalin or a pharmaceutically acceptable, pharmacologically active acid, salt, ester or amide thereof in the manufacture of a medicament for treating a symptom of a lower urinary tract disorder in combination with an antimuscarinic. Also provided is the use of an antimuscarinic in the manufacture of a medicament for treating a symptom of a lower urinary tract disorder in combination with an α₂δ subunit calcium channel modulator, which is gabapentin or pregabalin or a pharmaceutically acceptable, pharmacologically active acid, salt, ester or amide thereof.

One advantage of the present invention is that at least one detrimental side effect associated with single administration of an α₂δ subunit calcium channel modulator or an antimuscarinic is lessened by concurrent administration of an α₂δ subunit calcium channel modulator with an antimuscarinic. When an α₂δ subunit calcium channel modulator is administered in combination with an antimuscarinic, less of each agent is needed to achieve therapeutic efficacy. Because current treatments for painful and non-painful lower urinary tract disorders have limited efficacy and are associated with side effects that result in reduced patient compliance, the present invention presents a significant advantage over these treatments via increased efficacy and decreased side effects. Because detrimental side effects are lessened, the present invention also has the benefit of improving patient compliance.

### BRIEF DESCRIPTION OF THE DRAWINGS

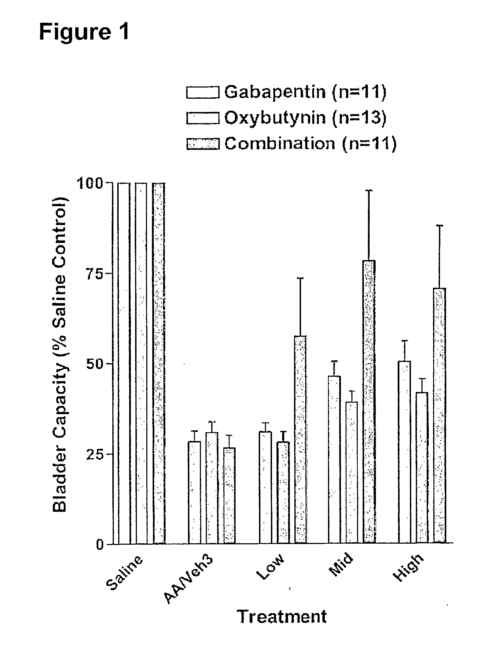

Figure 1. Figure 1 depicts the effect of cumulative increasing doses of oxybutynin (n=13), gabapentin (n=11) and their matched combinations (e.g. Dose 1 for the combination was 30 mg/kg gabapentin and 1 mg/kg oxybutynin; n=11) on bladder capacity. Data are normalized to saline controls and are presented as Mean ± SEM.

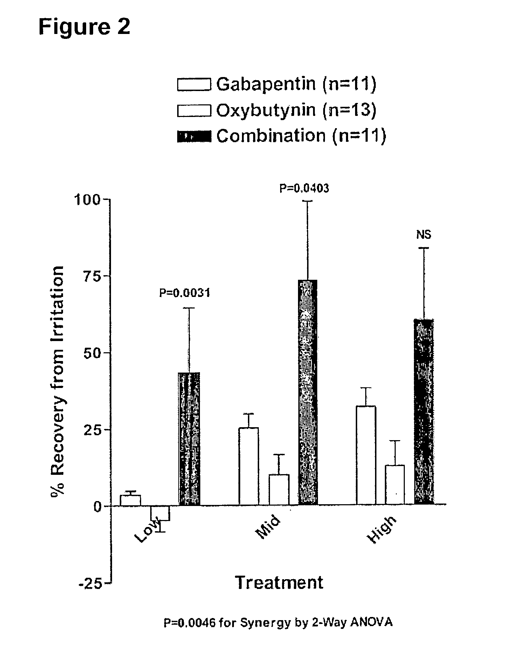

Figure 2. Figure 2 depicts the effect of cumulative increasing doses of oxybutynin (n=13), gabapentin (n=11) and their matched combinations (e.g. Dose 1 for the combination was 30 mg/kg gabapentin and 1 mg/kg oxybutynin; n=11) on bladder capacity (normalized to % Recovery from Irritation). Data are presented as Mean ± SEM.

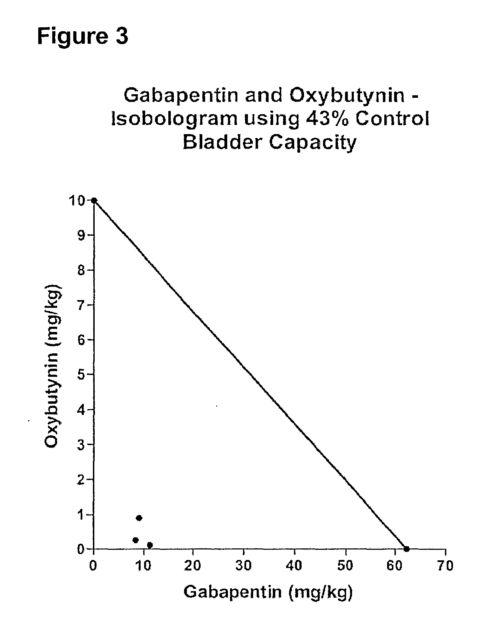

Figure 3. Figure 3 depicts the results of isobologram studies as determined by utilizing group means to determine effective doses. The common maximal effect for either drug alone was a return to 43% of saline control. The line connecting the two axes at the effective dose for each drug alone represents theoretical additivity.

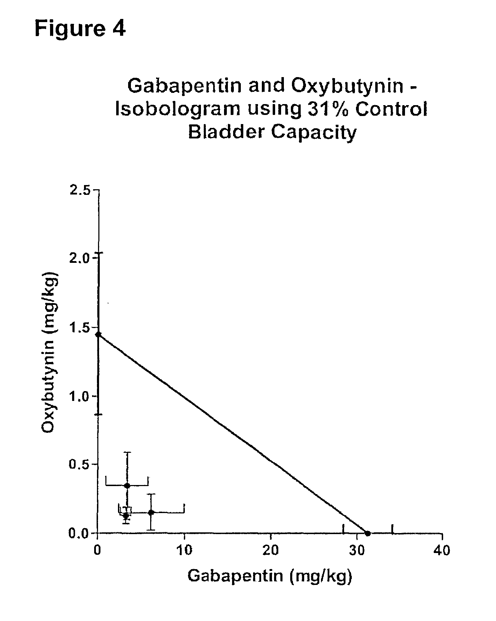

Figure 4. Figure 4 depicts the results of isobologram studies using a common maximal effect of individual animals using a return to 31% of saline control values. Data are presented as Mean ± SD.

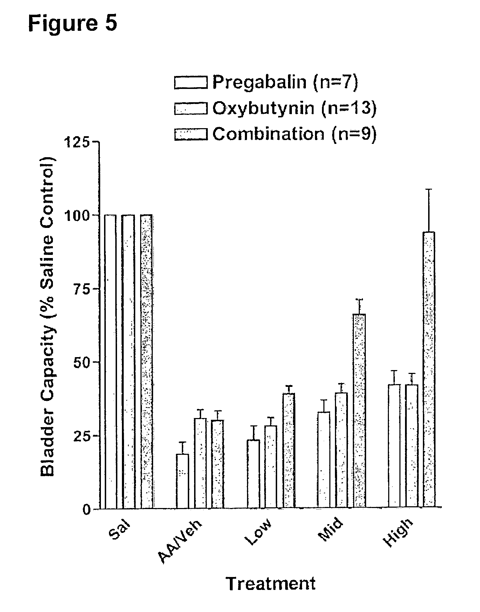

Figure 5. Figure 5 depicts the effect of cumulative increasing doses of oxybutynin (n=13), pregabalin (n=7) and matched combinations (e.g. Dose 1 for the combination was 10 mg/kg pregabalin and 1 mg/kg oxybutynin; n=9) on bladder capacity. Data are normalized to saline controls and are presented as Mean ± SEM.

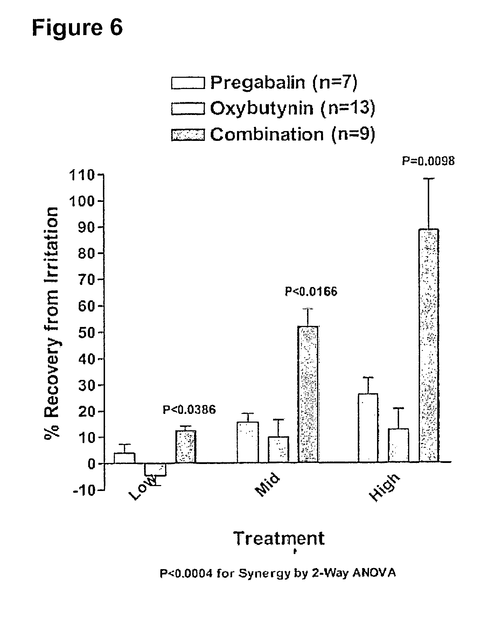

Figure 6. Figure 6 depicts the effect of cumulative increasing doses of oxybutynin (n=13), pregabalin (n=7) and matched combinations (e.g. Dose 1 for the combination was 10 mg/kg pregabalin and 1 mg/kg oxybutynin; n=9) on bladder capacity (normalized to % Recovery from Irritation).

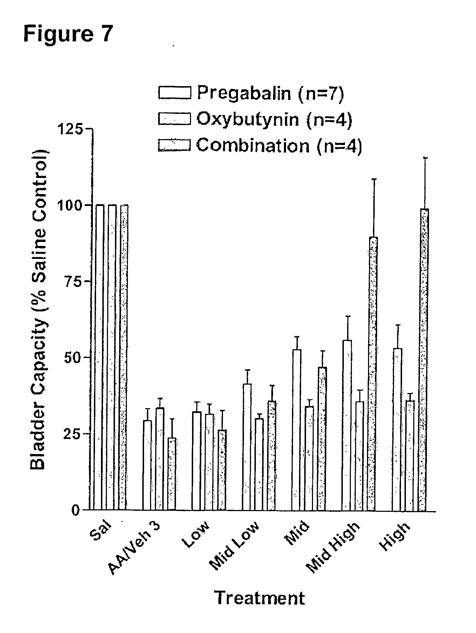

Figure 7. Figure 7 depicts the effect of cumulative increasing doses of oxybutynin (n=4), pregabalin (n=7) and matched combinations (e.g. Dose 1 for the combination was 3.75 mg/kg pregabalin and 0.625 mg/kg oxybutynin; n=4) on bladder capacity. Data are normalized to saline controls and are presented as Mean ± SEM.

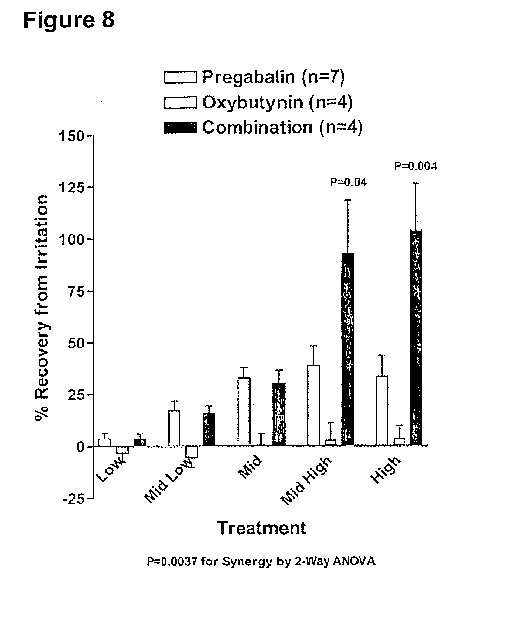

Figure 8. Figure 8 depicts the effect of cumulative increasing doses of oxybutynin (n=4), pregabalin (n=7) and matched combinations (e.g. Dose 1 for the combination was 3.75 mg/kg pregabalin and 0.625 mg/kg oxybutynin; n=4) on bladder capacity (normalized to % Recovery from Irritation). Data are presented as Mean ± SEM.

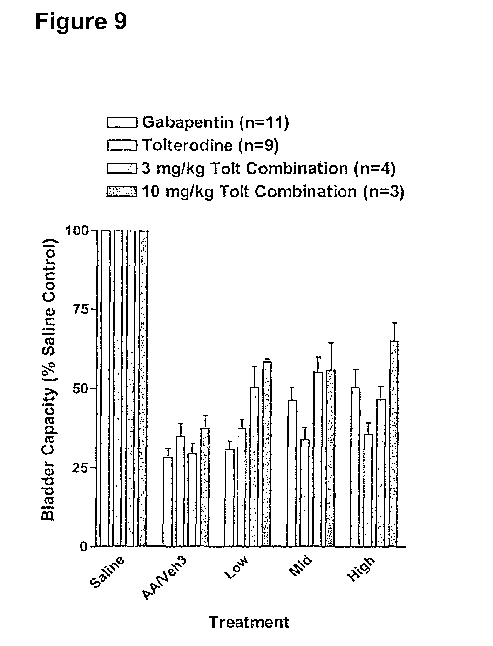

Figure 9. Figure 9 depicts the effect of cumulative increasing doses of tolterodine (n=9), gabapentin (n=11) and the 2 combinations tested (e.g. Dose 1 for the combination 1 was 30 mg/kg gabapentin and 3 mg/kg tolterodine; n=4 and 3 for 3 and 10 mg/kg tolterodine, respectively) on bladder capacity. Data are normalized to saline controls and are presented as Mean ± SEM.

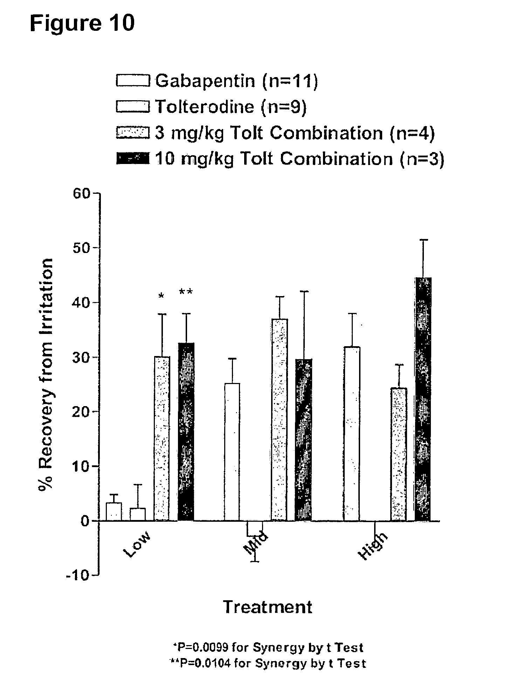

Figure 10. Figure 10 depicts the effect of cumulative increasing doses of tolterodine (n=9), gabapentin (n=11) and the 2 combinations (e.g. Dose 1 for the combination was 30 mg/kg gabapentin and 3 mg/kg tolterodine; n=4 and 3, for 3 mg/kg and 10 mg/kg tolterodine, respectively) on bladder capacity (normalized to % Recovery from Irritation).

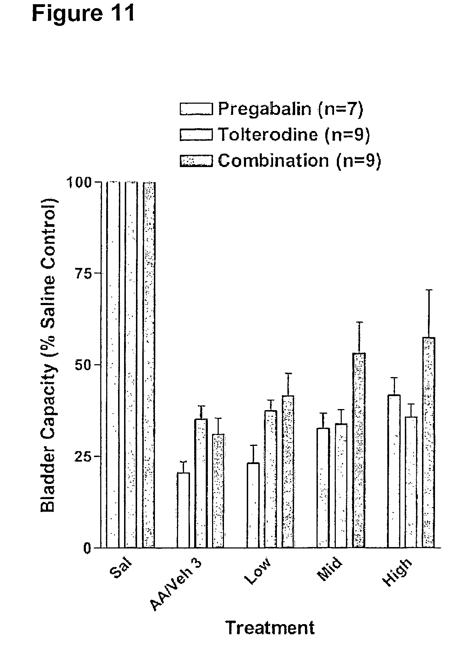

Figure 11. Figure 11 depicts the effect of cumulative increasing doses of tolterodine (n=9), pregabalin (n=7) and their matched combinations (e.g. Dose 1 for the combination was 10 mg/kg pregabalin and 1 mg/kg tolterodine; n=9) on bladder capacity. Data are normalized to saline controls and are presented as Mean ± SEM.

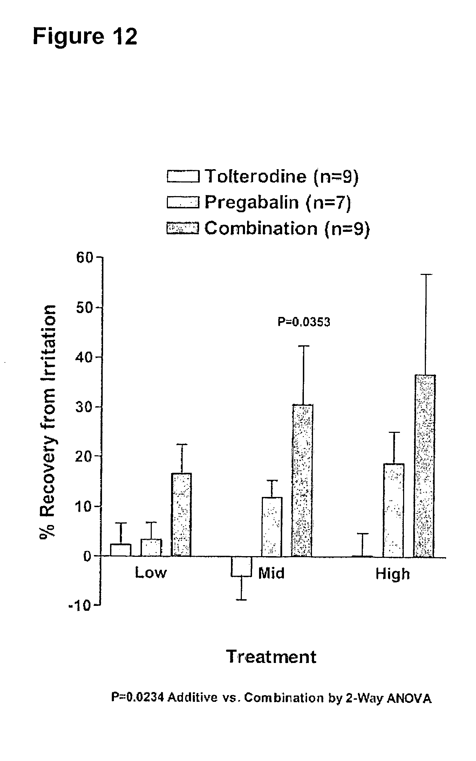

Figure 12. Figure 12 depicts the effect of cumulative increasing doses of tolterodine (n=9), pregabalin (n=7) and matched combinations (e.g. Dose 1 for the combination was 10 mg/kg pregabalin and 1 mg/kg tolterodine; n=9) on bladder capacity (normalized to % Recovery from Irritation).

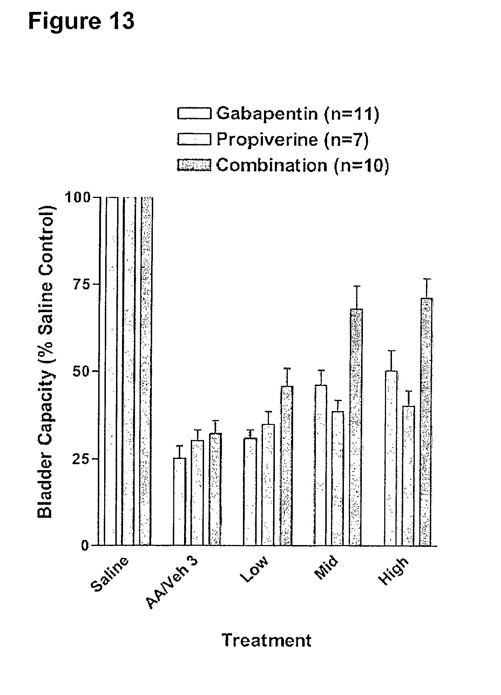

Figure 13. Figure 13 depicts the effect of cumulative increasing doses of propiverine (n=7), gabapentin (n=11) and matched combinations (e.g. Dose 1 for the combination was 10 mg/kg gabapentin and 3 mg/kg propiverine; n=10) on bladder capacity. Data are normalized to saline controls and are presented as Mean ± SEM.

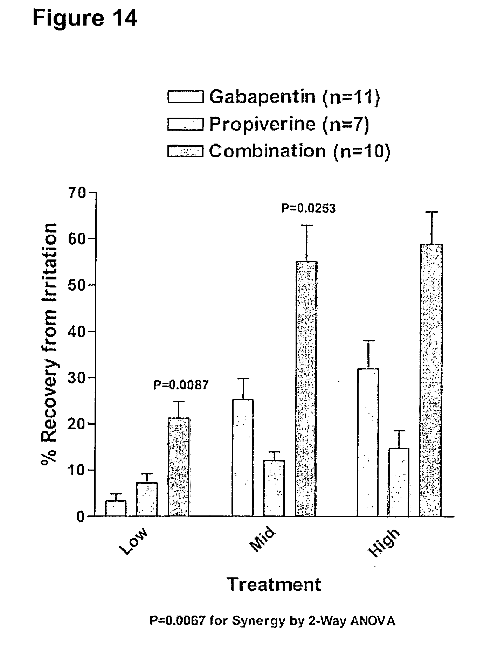

Figure 14. Figure 14 depicts the effect of cumulative increasing doses of propiverine (n=7), gabapentin (n=11) and their matched combinations (e.g. Dose 1 for the combination was 10 mg/kg gabapentin and 3 mg/kg propiverine; n=10) on bladder capacity (normalized to % Recovery from Irritation). Data are presented as Mean ± SEM.

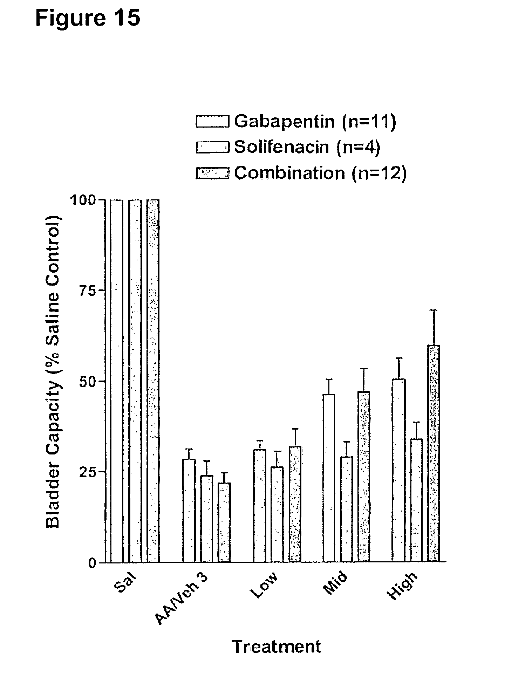

Figure 15. Figure 15 depicts the effect of cumulative increasing doses of solifenacin (n=4), gabapentin (n=11) and their matched combinations (e.g. Dose 1 for the combination was 10 mg/kg gabapentin and 3 mg/kg solifenacin; n=12) on bladder capacity. Data are normalized to saline controls and are presented as Mean ± SEM.

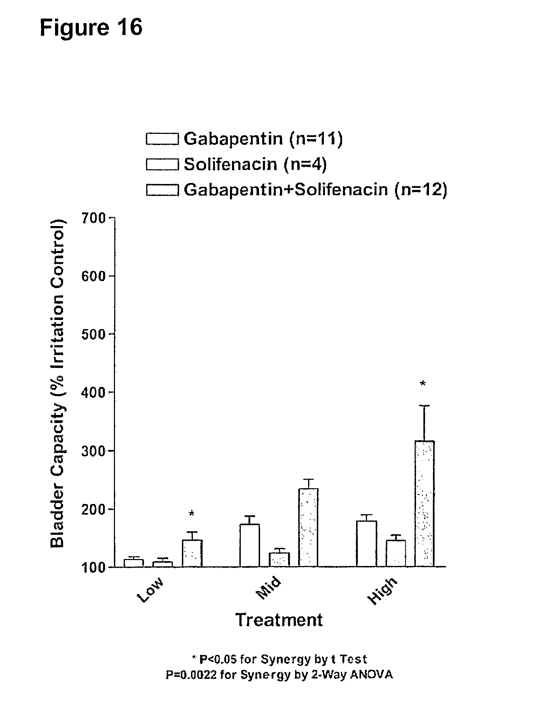

Figure 16. Figure 16 depicts the effect of cumulative increasing doses of solifenacin (n=4), gabapentin (n=11) and their matched combinations (e.g. Dose 1 for the combination was 10 mg/kg gabapentin and 3 mg/kg solifenacin; n=12) on bladder capacity (normalized to % Irritation Control). Data are presented as Mean ± SEM.

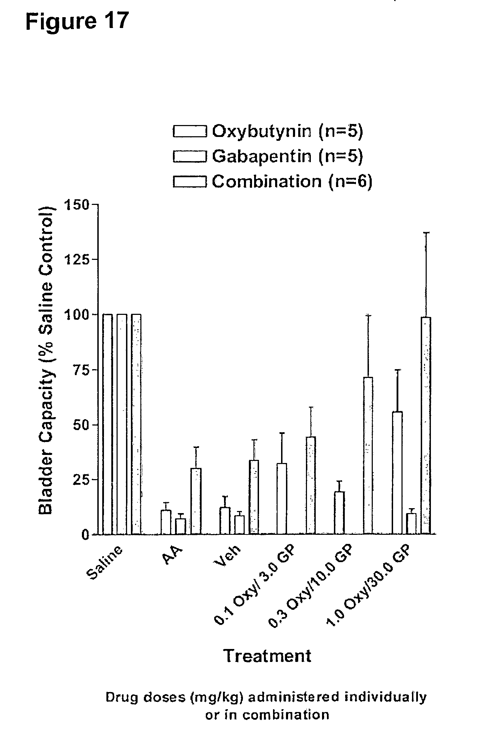

Figure 17. Figure 17 depicts the effect of cumulative increasing doses of oxybutynin (n=5), gabapentin (n=5) and their matched combinations (n=6) on bladder capacity. Data are normalized to saline controls and are presented as Mean ± SEM.

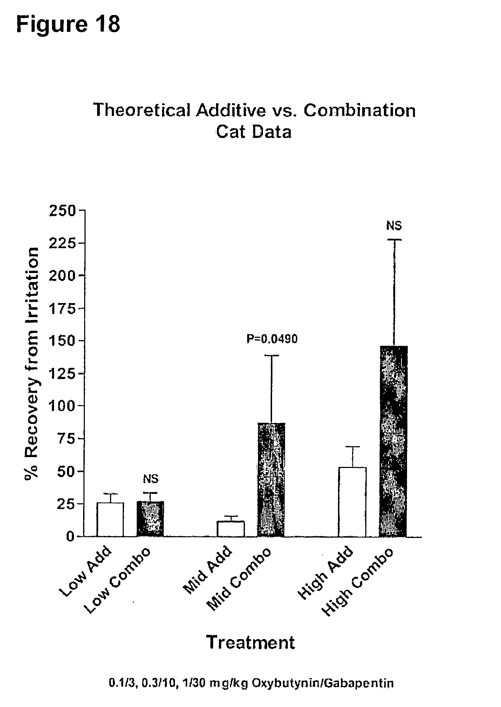

Figure 18. Figure 18 depicts the theoretical additive effect of cumulative increasing doses of oxybutynin (n=5) and gabapentin (n=5), and their matched combinations (e.g. Dose 1 for the combination was 3 mg/kg gabapentin and 0.1 mg/kg oxybutynin; n=6) on bladder capacity (normalized to % Recovery from Irritation). Data are presented as Mean ± SEM.

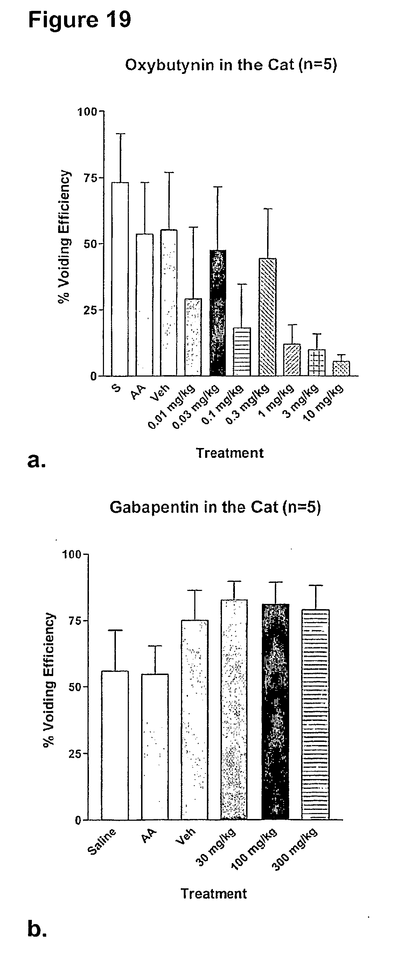

Figure 19. Figure 19 depicts the effect of cumulative increasing doses of oxybutynin (n=5; Figure 19A), gabapentin (n=5; Figure 19B) on voiding efficiency.

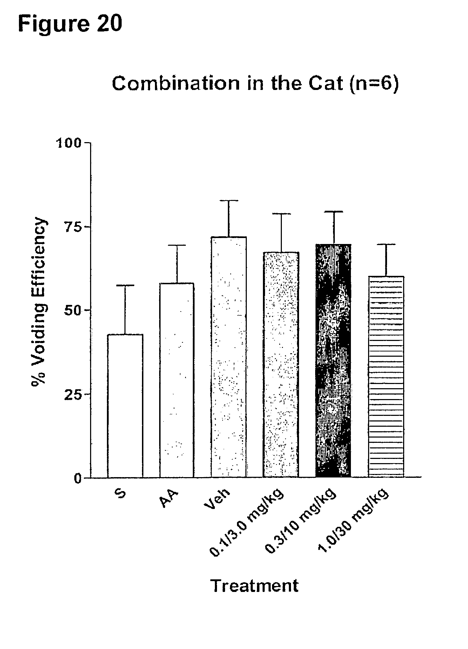

Figure 20. Figure 20 depicts the effect of cumulative increasing doses of oxybutynin and gabapentin in combination (n=6) on voiding efficiency.

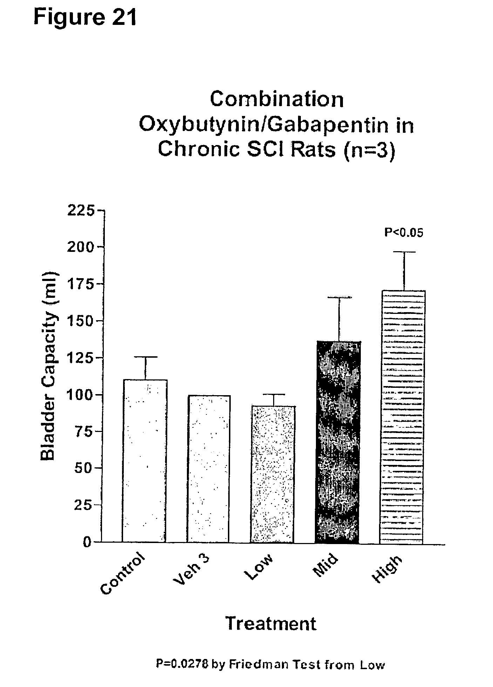

Figure 21. Figure 21 depicts the effect of cumulative increasing doses of the combination of oxybutynin and gabapentin (e.g. Dose 1 for the combination was 30 mg/kg gabapentin and 1 mg/kg oxybutynin; n=3) on bladder capacity in chronic SCI rats. Data are normalized to vehicle controls and are presented as Mean ± SEM.

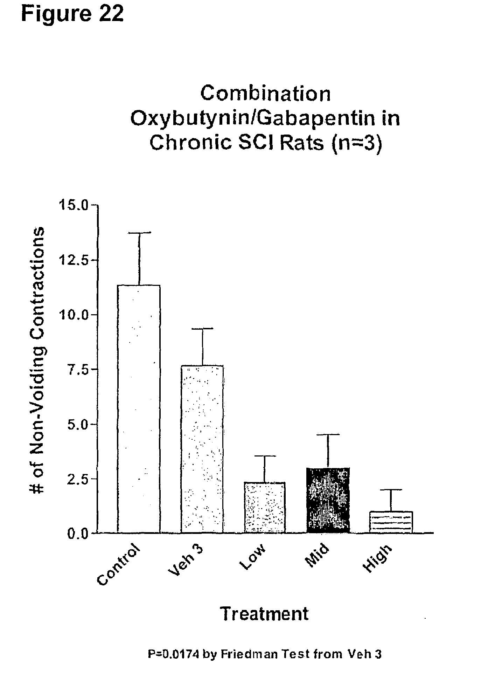

Figure 22. Figure 22 depicts a dose-dependent decrease in bladder instability, as measured by a decrease in the number of non-voiding contractions greater than 8 cm H₂O with increasing doses of the combination of oxybutynin and gabapentin (n=3). Data are presented as Mean ± SEM.

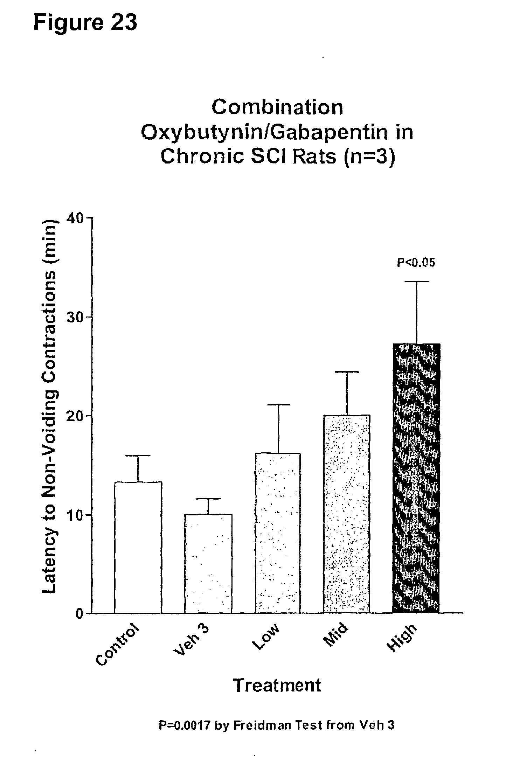

Figure 23. Figure 23 depicts a dose-dependent decrease in bladder instability, as measured the latency to the appearance of non-voiding contractions with increasing doses of the combination of oxybutynin and gabapentin (n=3). Data are presented as Mean ± SEM.

### DETAILED DESCRIPTION OF THE INVENTION

### Overview and Definitions

The present invention provides compositions and the use of compounds or compositions in the manufacture of medicaments for treating and/or alleviating the symptoms associated with painful and non-painful lower urinary tract disorders in normal and spinal cord injured patients. The lower urinary tract disorders of the present invention include, but are not limited to such disorders as painful and non-painful overactive bladder, prostatitis and prostadynia, interstitial cystitis, benign prostatic hyperplasia, and, in spinal cord injured patients, spastic bladder. Irritative symptoms of these disorders include at least one symptom selected from the group consisting of urinary urgency, urinary frequency, and nocturia. The compositions comprise a therapeutically effective dose of an α₂δ subunit calcium channel modulator which is gabapentin or pregabalin or a pharmaceutically acceptable, pharmacologically active acid, salt, ester or amide threof, in combination with one or more compounds with smooth muscle modulatory effects, including at least an antimuscarinic (particularly those that do not have an amine embedded in an 8-azabicyclo[3.2.1]octan-3-ol skeleton). Other compounds with smooth muscle modulatory effects include β3 adrenergic agonists, spasmolytics, neurokinin receptor antagonists, bradykinin receptor antagonists, and nitric oxide donors. The treatments involve administering, for example, various compositions and formulations that contain quantities of an α₂δ subunit calcium channel modulator and/or other compounds that interact with α₂δ subunit-containing calcium channels in combination with an antimuscarinic.

It is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

It must be noted that as used in this specification and the appended embodiments, the singular forms "a," "an," and "the" include plural referents unless the context clearly dictates otherwise. Thus, for example, reference to "an active agent" or "a pharmacologically active agent" includes a single active agent as well as two or more different active agents in combination, reference to "a carrier" includes mixtures of two or more carriers as well as a single carrier, and the like.

By "non-painful" is intended sensations or symptoms including mild or general discomfort that a patient subjectively describes as not producing or resulting in pain. Such symptoms may vary depending on the disorder being treated but generally include urinary urgency, incontinence, urge incontinence, stress incontinence, urinary frequency, nocturia, and the like. For benign prostatic hyperplasia, non-painful irritative symptoms include urinary frequency, urgency, and nocturia, while non-painful obstructive symptoms include reduced urinary force and speed of flow.

By "painful" is intended sensations or symptoms that a patient subjectively describes as producing or resulting in pain.

By "lower urinary tract" is intended all parts of the urinary system except the kidneys. By "lower urinary tract disorder" is intended any disorder involving the lower urinary tract, including but not limited to overactive bladder, prostatitis, interstitial cystitis, benign prostatic hyperplasia, and spastic and flaccid bladder. By "non-painful lower urinary tract disorder" is intended any lower urinary tract disorder involving sensations or symptoms, including mild or general discomfort, that a patient subjectively describes as not producing or resulting in pain. By "painful lower urinary tract disorder" is intended any lower urinary tract disorder involving sensations or symptoms that a patient subjectively describes as producing or resulting in pain.

By "bladder disorder" is intended any condition involving the urinary bladder. By "non-painful bladder disorder" is intended any bladder disorder involving sensations or symptoms, including mild or general discomfort, that a patient subjectively describes as not producing or resulting in pain. By "painful bladder disorder" is intended any bladder disorder involving sensations or symptoms that a patient subjectively describes as producing or resulting in pain.

By "overactive bladder"or "OAB" is intended any form of lower urinary tract disorder characterized by increased frequency of micturition or the desire to void, whether complete or episodic, and where loss of voluntary control ranges from partial to total and whether there is loss of urine (incontinence) or not. By "painful overactive bladder" is intended any form of overactive bladder, as defined above, involving sensations or symptoms that a patient subjectively describes as producing or resulting in pain. By "non-painful overactive bladder" is intended any form of overactive bladder, as defined above, involving sensations or symptoms, including mild or general discomfort, that a patient subjectively describes as not producing or resulting in pain. Non-painful symptoms can include, but are not limited to, urinary urgency, incontinence, urge incontinence, stress incontinence, urinary frequency, and nocturia.

"OAB wet" is used herein to describe overactive bladder in patients with incontinence, while "OAB dry" is used herein to describe overactive bladder in patients without incontinence.

By "urinary urgency" is intended sudden strong urges to urinate with little or no chance to postpone the urination. By "incontinence" is meant the inability to control excretory functions, including urination (urinary incontinence). By "urge incontinence" or "urinary urge incontinence" is intended the involuntary loss of urine associated with an abrupt and strong desire to void. By "stress incontinence" or "urinary stress incontinence" is intended a medical condition in which urine leaks when a person coughs, sneezes, laughs, exercises, lifts heavy objects, or does anything that puts pressure on the bladder. By "urinary frequency" is intended urinating more frequently than the patient desires. As there is considerable interpersonal variation in the number of times in a day that an individual would normally expect to urinate, "more frequently than the patient desires" is further defined as a greater number of times per day than that patient's historical baseline. "Historical baseline" is further defined as the median number of times the patient urinated per day during a normal or desirable time period. By "nocturia" is intended being awakened from sleep to urinate more frequently than the patient desires.

By "neurogenic bladder" or "neurogenic overactive bladder" is intended overactive bladder as described further herein that occurs as the result of neurological damage due to disorders including but not limited to stroke, Parkinson's disease, diabetes, multiple sclerosis, peripheral neuropathy, or spinal cord lesions.

By "detrusor hyperreflexia" is intended a condition characterized by uninhibited detrusor, wherein the patient has some sort of neurologic impairment. By "detrusor instability" or "unstable detrusor" is intended conditions where there is no neurologic abnormality.

By "prostatitis" is intended any type of disorder associated with an inflammation of the prostate, including chronic bacterial prostatitis and chronic non-bacterial prostatitis. By "non-painful prostatitis" is intended prostatitis involving sensations or symptoms, including mild or general discomfort, that a patient subjectively describes as not producing or resulting in pain. By "painful prostatitis" is intended prostatitis involving sensations or symptoms that a patient subjectively describes as producing or resulting in pain.

"Chronic bacterial prostatitis" is used in its conventional sense to refer to a disorder associated with symptoms that include inflammation of the prostate and positive bacterial cultures of urine and prostatic secretions. "Chronic non-bacterial prostatitis" is used in its conventional sense to refer to a disorder associated with symptoms that include inflammation of the prostate and negative bacterial cultures of urine and prostatic secretions. "Prostadynia" is used in its conventional sense to refer to a disorder generally associated with painful symptoms of chronic non-bacterial prostatitis as defined above, without inflammation of the prostate.

"Interstitial cystitis" is used in its conventional sense to refer to a disorder associated with symptoms that include irritative voiding symptoms, urinary frequency, urgency, nocturia, and suprapubic or pelvic pain related to and relieved by voiding.

"Benign prostatic hyperplasia" is used in its conventional sense to refer to a disorder associated with benign enlargement of the prostate gland. By "irritiative symptoms of benign prostatic hyperplasia" is intended urinary urgency, urinary frequency, and nocturia. By "obstructive symptoms of benign prostatic hyperplasia" is intended reduced urinary force and speed of flow.

"Spastic bladder" or "reflex bladder" is used in its conventional sense to refer to a condition following spinal cord injury in which bladder emptying has become unpredictable.

"Flaccid bladder" or "non-reflex bladder" is used in its conventional sense to refer to a condition following spinal cord injury in which the reflexes of the bladder muscles are absent or slowed.

"Dyssynergia" is used in its conventional sense to refer to a condition following spinal cord injury in which patients characterized by an inability of urinary sphincter muscles to relax when the bladder contracts.

By "irritative symptoms" generally is intended at least one symptom selected from the group consisting of urinary urgency, incontinence, urge incontinence, urinary frequency, nocturia. By "irritative symptoms of benign prostatic hyperplasia" is intended urinary urgency, urinary frequency, and nocturia.

The terms "active agent" and "pharmacologically active agent" are used interchangeably herein to refer to a chemical compound that induces a desired effect, i.e., in this case, treating and/or alleviating the symptoms associated with painful and non-painful lower urinary tract disorders and associated irritative symptoms in normal and spinal cord injured patients. The primary active agents herein are the α₂δ subunit calcium channel modulators gabapentin or pregabalin and/or smooth muscle relaxants which include an antimuscarinic. The present invention comprises a combination therapy wherein an α₂δ subunit calcium channel modulator is administered with one or more smooth muscle modulators including at least an antimuscarinic. Such combination therapy may be carried out by administration of the different active agents in a single composition, by concurrent administration of the different active agents in different compositions, or by sequential administration of the different active agents. The combination therapy may also include situations where the α₂δ subunit calcium channel modulator or the smooth muscle modulator is already being administered to the patient, and the additional component is to be added to the patient's drug regimen, as well as where different individuals (e.g., physicians or other medical professionals) are administering the separate components of the combination to the patient. Included are derivatives and analogs of those compounds or classes of compounds specifically mentioned that also induce the desired effect.

The term "α₂δ subunit calcium channel modulator" as used herein refers to an agent that is capable of interacting with the α₂δ subunit of a calcium channel, including a binding event, including subtypes of the α₂δ calcium channel subunit as disclosed in Klugbauer et al. (1999) *J. Neurosci.* 19: 684-691, to produce a physiological effect, such as opening, closing, blocking, up-regulating functional expression, down-regulating functional expression, or desensitization, of the channel. Unless otherwise indicated, the term "α₂δ subunit calcium channel modulator" is intended to indicate gabapentin and/or pregabalin, as well as acids, salts, esters and amides thereof. Further, it is understood that any salts, esters or amides are pharmaceutically acceptable as well as pharmacologically active.

The term "smooth muscle modulator" as used herein refers to any compound that inhibits or blocks the contraction of smooth muscles, including but not limited to antimuscarinics, β3 adrenergic agonists, spasmolytics, neurokinin receptor antagonists, bradykinin receptor antagonists, and nitric oxide donors. Smooth muscle modulators can be "direct" (also known as "musculotropic") or "indirect" (also known as "neurotropic"). "Direct smooth muscle modulators" are smooth muscle modulators that act by inhibiting or blocking contractile mechanisms within smooth muscle, including but not limited to modification of the interaction between actin and myosin. "Indirect smooth muscle modulators" are smooth muscle modulators that act by inhibiting or blocking neurotransmission that results in the contraction of smooth muscle, including but not limited to blockade of presynaptic facilitation of acetylcholine release at the axon terminal of motor neurons terminating in smooth muscle.

The term "anticholinergic agent" as used herein refers to any acetylcholine receptor antagonist, including antagonists of nicotinic and/or muscarinic acetylcholine receptors. The term "antinicotinic agent" as used herein is intended any nicotinic acytylcholine receptor antagonist. The term "antimuscarinic agent" as used herein is intended any muscarinic acetylcholine receptor antagonist. Unless otherwise indicated, the terms "anticholinergic agent," "antinicotinic agent," and "antimuscarinic agent" are intended to include anticholinergic, antinicotinic, and antimuscarinic agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

The term "β3 adrenergic agonist" is used in its conventional sense to refer to a compound that binds to and agonizes β3 adrenergic receptors. Unless otherwise indicated, the term "β3 adrenergic agonist" is intended to include β3 adrenergic agonist agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

The term "spasmolytic" (also known as "antispasmodic") is used in its conventional sense to refer to a compound that relieves or prevents muscle spasms, especially of smooth muscle. Unless otherwise indicated, the term "spasmolytic" is intended to include spasmolytic agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

The term "neurokinin receptor antagonist" is used in its conventional sense to refer to a compound that binds to and antagonizes neurokinin receptors. Unless otherwise indicated, the term "neurokinin receptor antagonist" is intended to include neurokinin receptor antagonist agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

The term "bradykinin receptor antagonist" is used in its conventional sense to refer to a compound that binds to and antagonizes bradykinin receptors. Unless otherwise indicated, the term "bradykinin receptor antagonist" is intended to include bradykinin receptor antagonist agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

The term "nitric oxide donor" is used in its conventional sense to refer to a compound that releases free nitric oxide when administered to a patient. Unless otherwise indicated, the term "nitric oxide donor" is intended to include nitric oxide donor agents as disclosed further herein, as well as acids, salts, esters, amides, prodrugs, active metabolites, and other derivatives thereof. Further, it is understood that any salts, esters, amides, prodrugs, active metabolites or other derivatives are pharmaceutically acceptable as well as pharmacologically active.

The terms "treating" and "treatment" as used herein refer to relieving the painful or non-painful (including irritative) symptoms or other clinically observed sequelae for clinically diagnosed disorders as described herein, including disorders associated with lower urinary tract in normal and spinal cord injured patients.

By an "effective" amount or a "therapeutically effective amount" of a drug or pharmacologically active agent is meant a nontoxic but sufficient amount of the drug or agent to provide the desired effect, i.e., relieving the painful or non-painful (including irritative) symptoms associated with lower urinary tract disorders in normal and spinal cord injured patients, as explained above. It is recognized that the effective amount of a drug or pharmacologically active agent will vary depending on the route of administration, the selected compound, and the species to which the drug or pharmacologically active agent is administered, as well as the age, weight, and sex of the individual to which the drug or pharmacologically active agent is administered. It is also recognized that one of skill in the art will determine appropriate effective amounts by taking into account such factors as metabolism, bioavailability, and other factors that affect plasma levels of a drug or pharmacologically active agent following administration within the unit dose ranges disclosed further herein for different routes of administration.

By "pharmaceutically acceptable," such as in the recitation of a "pharmaceutically acceptable carrier," or a "pharmaceutically acceptable acid addition salt," is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. "Pharmacologically active" (or simply "active") as in a "pharmacologically active" derivative or metabolite, refers to a derivative or metabolite having the same type of pharmacological activity as the parent compound. When the term "pharmaceutically acceptable" is used to refer to a derivative (e.g., a salt or an analog) of an active agent, it is to be understood that the compound is pharmacologically active as well, i.e., therapeutically effective for treating and/or alleviating the symptoms associated with painful and non-painful lower urinary tract disorders in normal and spinal cord injured patients.

By "continuous" dosing is meant the chronic administration of a selected active agent.

By "as-needed" dosing, also known as *"pro re nata"* "pm" dosing, and "on demand" dosing or administration is meant the administration of a single dose of the active agent at some time prior to commencement of an activity wherein suppression of the painful and non-painful (including irritative) symptoms of a lower urinary tract disorder in normal and spinal cord injured patients, would be desirable. Administration can be immediately prior to such an activity, including about 0 minutes, about 10 minutes, about 20 minutes, about 30 minutes, about 1 hour, about 2 hours, about 3 hours, about 4 hours, about 5 hours, about 6 hours, about 7 hours, about 8 hours, about 9 hours, or about 10 hours prior to such an activity, depending on the formulation.

By "short-term" is intended any period of time up to and including about 8 hours, about 7 hours, about 6 hours, about 5 hours, about 4 hours, about 3 hours, about 2 hours, about 1 hour, about 40 minutes, about 20 minutes, or about 10 minutes after drug administration.

By "rapid-offset" is intended any period of time up to and including about 8 hours, about 7 hours, about 6 hours, about 5 hours, about 4 hours, about 3 hours, about 2 hours, about 1 hour, about 40 minutes, about 20 minutes, or about 10 minutes after drug administration.

The term "controlled release" is intended to refer to any drug-containing formulation in which release of the drug is not immediate, i.e., with a "controlled release" formulation, oral administration does not result in immediate release of the drug into an absorption pool. The term is used interchangeably with "non-immediate release" as defined in Remington: The Science and Practice of Pharmacy, Twentieth Ed. (Philadelphia, Pa.: Lippincott Williams & Wilkins, 2000).

The "absorption pool" represents a solution of the drug administered at a particular absorption site, and kᵣ, kₐ, and kₑ are first-order rate constants for: 1) release of the drug from the formulation; 2) absorption; and 3) elimination, respectively. For immediate release dosage forms, the rate constant for drug release kᵣ is far greater than the absorption rate constant kₐ. For controlled release formulations, the opposite is true, i.e., kᵣ <<< kₐ, such that the rate of release of drug from the dosage form is the rate-limiting step in the delivery of the drug to the target area. The term "controlled release" as used herein includes any nonimmediate release formulation, including but not limited to sustained release, delayed release and pulsatile release formulations.

The term "sustained release" is used in its conventional sense to refer to a drug formulation that provides for gradual release of a drug over an extended period of time, and that preferably, although not necessarily, results in substantially constant blood levels of a drug over an extended time period such as up to about 72 hours, about 66 hours, about 60 hours, about 54 hours, about 48 hours, about 42 hours, about 36 hours, about 30 hours, about 24 hours, about 18 hours, about 12 hours, about 10 hours, about 8 hours, about 7 hours, about 6 hours, about 5 hours, about 4 hours, about 3 hours, about 2 hours, or about 1 hour after drug administration.

The term "delayed release" is used in its conventional sense to refer to a drug formulation that provides for an initial release of the drug after some delay following drug administration and that preferably, although not necessarily, includes a delay of up to about 10 minutes, about 20 minutes, about 30 minutes, about 1 hour, about 2 hours, about 3 hours, about 4 hours, about 5 hours, about 6 hours, about 7 hours, about 8 hours, about 9 hours, about 10 hours, about 11 hours, or about 12 hours.

The term "pulsatile release" is used in its conventional sense to refer to a drug formulation that provides release of the drug in such a way as to produce pulsed plasma profiles of the drug after drug administration. The term "immediate release" is used in its conventional sense to refer to a drug formulation that provides for release of the drug immediately after drug administration.

The term "immediate release" is used in its conventional sense to refer to a drug formulation that provides for release of the drug immediately after drug administration.

By the term "transdermal" drug delivery is meant delivery by passage of a drug through the skin or mucosal tissue and into the bloodstream.

The term "topical administration" is used in its conventional sense to mean delivery of a topical drug or pharmacologically active agent to the skin or mucosa.

The term "oral administration" is used in its conventional sense to mean delivery of a drug through the mouth and ingestion through the stomach and digestive tract.

The term "inhalation administration" is used in its conventional sense to mean delivery of an aerosolized form of the drug by passage through the nose or mouth during inhalation and passage of the drug through the walls of the lungs.

The term "intravesical administration" is used in its conventional sense to mean delivery of a drug directly into the bladder.

By the term "parenteral" drug delivery is meant delivery by passage of a drug into the blood stream without first having to pass through the alimentary canal, or digestive tract. Parenteral drug delivery may be "subcutaneous," referring to delivery of a drug by administration under the skin. Another form of parenteral drug delivery is "intramuscular," referring to delivery of a drug by administration into muscle tissue. Another form of parenteral drug delivery is "intradermal," referring to delivery of a drug by administration into the skin. An additional form of parenteral drug delivery is "intravenous," referring to delivery of a drug by administration into a vein. An additional form of parenteral drug delivery is "intra-arterial," referring to delivery of a drug by administration into an artery. Another form of parenteral drug delivery is "transdermal," referring to delivery of a drug by passage of the drug through the skin and into the bloodstream. Another form of parenteral drug delivery is "intrathecal," referring to delivery of a drug directly into the into the intrathecal space (where fluid flows around the spinal cord).

Still another form of parenteral drug delivery is "transmucosal," referring to administration of a drug to the mucosal surface of an individual so that the drug passes through the mucosal tissue and into the individual's blood stream. Transmucosal drug delivery may be "buccal" or "transbuccal," referring to delivery of a drug by passage through an individual's buccal mucosa and into the bloodstream. Another form of transmucosal drug delivery herein is "lingual" drug delivery, which refers to delivery of a drug by passage of a drug through an individual's lingual mucosa and into the bloodstream. Another form of transmucosal drug delivery herein is "sublingual" drug delivery, which refers to delivery of a drug by passage of a drug through an individual's sublingual mucosa and into the bloodstream. Another form of transmucosal drug delivery is "nasal" or "intranasal" drug delivery, referring to delivery of a drug through an individual's nasal mucosa and into the bloodstream. An additional form of transmucosal drug delivery herein is "rectal" or "transrectal" drug delivery, referring to delivery of a drug by passage of a drug through an individual's rectal mucosa and into the bloodstream. Another form of transmucosal drug delivery is "urethral" or "transurethral" delivery, referring to delivery of the drug into the urethra such that the drug contacts and passes through the wall of the urethra. An additional form of transmucosal drug delivery is "vaginal" or "transvaginal" delivery, referring to delivery of a drug by passage of a drug through an individual's vaginal mucosa and into the bloodstream. An additional form of transmucosal drug delivery is "perivaginal" delivery, referring to delivery of a drug through the vaginolabial tissue into the bloodstream.

In order to carry out the method described herein, a selected active agent is administered to a patient suffering from a painful or non-painful lower urinary tract disorder or associated irritative symptoms in normal and spinal cord injured patients. A therapeutically effective amount of the active agent may be administered orally, intravenously, subcutaneously, transmucosally (including buccally, sublingually, transurethrally, and rectally), topically, transdermally, by inhalation, intravesically, intrathecally or using any other route of administration

### Lower Urinary Tract Disorders

Lower urinary tract disorders affect the quality of life of millions of men and women in the United States every year. While the kidneys filter blood and produce urine, the lower urinary tract is concerned with storage and elimination of this waste liquid and includes all other parts of the urinary tract except the kidneys. Generally, the lower urinary tract includes the ureters, the urinary bladder, and the urethra. Disorders of the lower urinary tract include painful and non-painful overactive bladder, prostatitis and prostadynia, interstitial cystitis, benign prostatic hyperplasia, and, in spinal cord injured patients, spastic bladder and flaccid bladder.

Overactive bladder is a treatable medical condition that is estimated to affect 17 to 20 million people in the United States. Symptoms of overactive bladder include urinary frequency, urgency, nocturia (the disturbance of nighttime sleep because of the need to urinate) and urge incontinence (accidental loss of urine) due to a sudden and unstoppable need to urinate. As opposed to stress incontinence, in which loss of urine is associated with physical actions such as coughing, sneezing, exercising, or the like, urge incontinence is usually associated with an overactive detrusor muscle (the smooth muscle of the bladder which contracts and causes it to empty).

There is no single etiology for overactive bladder. Neurogenic overactive bladder (or neurogenic bladder) occurs as the result of neurological damage due to disorders such as stroke, Parkinson's disease, diabetes, multiple sclerosis, peripheral neuropathy, or spinal cord lesions. In these cases, the overactivity of the detrusor muscle is termed detrusor hyperreflexia. By contrast, non-neurogenic overactive bladder can result from non-neurological abnormalities including bladder stones, muscle disease, urinary tract infection or drug side effects.

Due to the enormous complexity of micturition (the act of urination) the exact mechanism causing overactive bladder is unknown. Overactive bladder may result from hypersensitivity of sensory neurons of the urinary bladder, arising from various factors including inflammatory conditions, hormonal imbalances, and prostate hypertrophy. Destruction of the sensory nerve fibers, either from a crushing injury to the sacral region of the spinal cord, or from a disease that causes damage to the dorsal root fibers as they enter the spinal cord may also lead to overactive bladder. In addition, damage to the spinal cord or brain stem causing interruption of transmitted signals may lead to abnormalities in micturition. Therefore, both peripheral and central mechanisms may be involved in mediating the altered activity in overactive bladder.

In spite of the uncertainty regarding whether central or peripheral mechanisms, or both, are involved in overactive bladder, many proposed mechanisms implicate neurons and pathways that mediate non-painful visceral sensation. Pain is the perception of an aversive or unpleasant sensation and may arise through a variety of proposed mechanisms. These mechanisms include activation of specialized sensory receptors that provide information about tissue damage (nociceptive pain), or through nerve damage from diseases such as diabetes, trauma or toxic doses of drugs (neuropathic pain) (See, e.g., A.I. Basbaum and T.M. Jessell (2000) The perception of pain. In *Principles of Neural Science,* 4th. ed.; Benevento *et al.* (2002) *Physical Therapy Journal* 82:601-12). Nociception may give rise to pain, but not all stimuli that activate nociceptors are experienced as pain (A.I. Basbaum and T.M. Jessell (2000) The perception of pain. In *Principles of Neural Science,* 4th. ed.). Somatosensory information from the bladder is relayed by nociceptive Aδ and C fibers that enter the spinal cord via the dorsal root ganglion (DRG) and project to the brainstem and thalamus via second or third order neurons (Andersson (2002) *Urology* 59:18-24; Andersson (2002) *Urology* 59:43-50; Morrison, J., Steers, W.D., Brading, A., Blok, B., Fry, C., de Groat, W.C., Kakizaki, H., Levin, R., and Thor, K.B., "Basic Urological Sciences" In: Incontinence (vol. 2) Abrams, P. Khoury, S., and Wein, A. (Eds.) Health Publications, Ltd., Plymbridge Ditributors, Ltd., Plymouth, UK., (2002). A number of different subtypes of sensory afferent neurons may be involved in neurotransmission from the lower urinary tract. These may be classified as, but not limited to, small diameter, medium diameter, large diameter, myelinated, unmyelinated, sacral, lumbar, peptidergic, non-peptidergic, IB4 positive, IB4 negative, C fiber, Aδ fiber, high threshold or low threshold neurons. Nociceptive input to the DRG is thought to be conveyed to the brain along several ascending pathways, including the spinothalamic, spinoreticular, spinomesencephalic, spinocervical, and in some cases dorsal column/medial lemniscal tracts (A.I. Basbaum and T.M. Jessell (2000) The perception of pain. In *Principles of Neural Science,* 4th. ed.). Central mechanisms, which are not fully understood, are thought to convert some, but not all, nociceptive information into painful sensory perception (A.I. Basbaum and T.M. Jessell (2000) The perception of pain. In *Principles of Neural Science,* 4th. ed.).

Although many compounds have been explored as treatments for disorders involving pain of the bladder or other pelvic visceral organs, relatively little work has been directed toward treatment of non-painful sensory symptoms associated with bladder disorders such as overactive bladder. Current treatments for overactive bladder include medication, diet modification, programs in bladder training, electrical stimulation, and surgery. Currently, antimuscarinics (which are subtypes of the general class of anticholinergics) are the primary medication used for the treatment of overactive bladder. This treatment suffers from limited efficacy and side effects such as dry mouth, dry eyes, dry vagina, palpitations, drowsiness, and constipation, which have proven difficult for some individuals to tolerate.

Overactive bladder (or OAB) can occur with or without incontinence. In recent years, it has been recognized among those of skill in the art that the cardinal symptom of OAB is urgency without regard to any demonstrable loss of urine. For example, a recent study examined the impact of all OAB symptoms on the quality of life of a community-based sample of the United States population. (Liberman *et al. (2001) Urology* 57: 1044-1050). This study demonstrated that individuals suffering from OAB without any demonstrable loss of urine have an impaired quality of life when compared with controls. Additionally, individuals with urgency alone have an impaired quality of life compared with controls.

Although urgency is now believed to be the primary symptom of OAB, to date it has not been evaluated in a quantified way in clinical studies. Corresponding to this new understanding of OAB, however, the terms OAB Wet (with incontinence) and OAB Dry (without incontinence) have been proposed to describe these different patient populations (see, e.g., WO03/051354). The prevalence of OAB Wet and OAB Dry is reported to be similar in men and women, with a prevalence rate in the United States of 16.6% (Stewart *et al.,* "Prevalence of Overactive Bladder in the United States: Results from the NOBLE Program," Abstract Presented at the *Second International Consultation on Incontinence,* July 2001, Paris, France).

Prostatitis and prostadynia are other lower urinary tract disorders that have been suggested to affect approximately 2-9% of the adult male population (Collins M M, et al., (1998) "How common is prostatitis? A national survey of physician visits," *Journal of Urology,* 159: 1224-1228). Prostatitis is associated with an inflammation of the prostate, and may be subdivided into chronic bacterial prostatitis and chronic non-bacterial prostatitis. Chronic bacterial prostatitis is thought to arise from bacterial infection and is generally associated with such symptoms as inflammation of the prostate, the presence of white blood cells in prostatic fluid, and/or pain. Chronic non-bacterial prostatitis is an inflammatory and painful condition of unknown etiology characterized by excessive inflammatory cells in prostatic secretions despite a lack of documented urinary tract infections, and negative bacterial cultures of urine and prostatic secretions. Prostadynia (chronic pelvic pain syndrome) is a condition associated with the painful symptoms of chronic non-bacterial prostatitis without an inflammation of the prostate.

Currently, there are no established treatments for prostatitis and prostadynia. Antibiotics are often prescribed, but with little evidence of efficacy. COX-2 selective inhibitors and α-adrenergic blockers and have been suggested as treatments, but their efficacy has not been established. Hot sitz baths and anticholinergic drugs have also been employed to provide some symptomatic relief.

Interstitial cystitis is another lower urinary tract disorder of unknown etiology that predominantly affects young and middle-aged females, although men and children can also be affected. Symptoms of interstitial cystitis may include irritative voiding symptoms, urinary frequency, urgency, nocturia and suprapubic or pelvic pain related to and relieved by voiding. Many interstitial cystitis patients also experience headaches as well as gastrointestinal and skin problems. In some extreme cases, interstitial cystitis may also be associated with ulcers or scars of the bladder.

Past treatments for interstitial cystitis have included the administration of antihistamines, sodium pentosanpolysulfate, dimethylsulfoxide, steroids, tricyclic antidepressants and narcotic antagonists, although these methods have generally been unsuccessful (Sant, G. R. (1989) Interstitial cystitis: pathophysiology, clinical evaluation and treatment. *Urology Annal* 3: 171-196).

Benign prostatic hyperplasia (BPH) is a non-malignant enlargement of the prostate that is very common in men over 40 years of age. BPH is thought to be due to excessive cellular growth of both glandular and stromal elements of the prostate. Irritative symptoms of benign prostatic hyperplasia include urinary urgency, urinary frequency, and nocturia. Obstructive symptoms associated with benign prostatic hyperplasia are characterized by reduced urinary force and speed of flow.

Invasive treatments for BPH include transurethral resection of the prostate, transurethral incision of the prostate, balloon dilation of the prostate, prostatic stents, microwave therapy, laser prostatectomy, transrectal high-intensity focused ultrasound therapy and transurethral needle ablation of the prostate. However, complications may arise through the use of some of these treatments, including retrograde ejaculation, impotence, postoperative urinary tract infection and some urinary incontinence. Non-invasive treatments for BPH include androgen deprivation therapy and the use of 5α-reductase inhibitors and α-adrenergic blockers. However, these treatments have proven only minimally to moderately effective for some patients.

Lower urinary tract disorders are particularly problematic for individuals suffering from spinal cord injury. After spinal cord injury, the kidneys continue to make urine, and urine can continue to flow through the ureters and urethra because they are the subject of involuntary neural and muscular control, with the exception of conditions where bladder to smooth muscle dyssenergia is present. By contrast, bladder and sphincter muscles are also subject to voluntary neural and muscular control, meaning that descending input from the brain through the spinal cord drives bladder and sphincter muscles to completely empty the bladder. Following spinal cord injury, such descending input may be disrupted such that individuals may no longer have voluntary control of their bladder and sphincter muscles. Spinal cord injuries can also disrupt sensory signals that ascend to the brain, preventing such individuals from being able to feel the urge to urinate when their bladder is full.

The compositions of the invention and medicaments manufactured in accordance with the invention find use in relieving or reducing the irritative symptoms and/or obstructive symptoms of benign prostatic hyperplasia and may reduce the need for other more invasive treatments.

Following spinal cord injury, the bladder is usually affected in one of two ways. The first is a condition called "spastic" or "reflex" bladder, in which the bladder fills with urine and a reflex automatically triggers the bladder to empty. This usually occurs when the injury is above the T12 level. Individuals with spastic bladder are unable to determine when, or if, the bladder will empty. The second is "flaccid" or "non-reflex" bladder, in which the reflexes of the bladder muscles are absent or slowed. This usually occurs when the injury is below the T12/L1 level. Individuals with flaccid bladder may experience over-distended or stretched bladders and "reflux" of urine through the ureters into the kidneys. Treatment options for these disorders usually include intermittent catheterization, indwelling catheterization, or condom catheterization, but these methods are invasive and frequently inconvenient.

Urinary sphincter muscles may also be affected by spinal cord injuries, resulting in a condition known as "dyssynergia." Dyssynergia involves an inability of urinary sphincter muscles to relax when the bladder contracts, including active contraction in response to bladder contraction, which prevents urine from flowing through the urethra and results in the incomplete emptying of the bladder and "reflux" of urine into the kidneys. Traditional treatments for dyssynergia include medications that have been somewhat inconsistent in their efficacy or surgery.

### Peripheral vs. Central Effects

The mammalian nervous system comprises a central nervous system (CNS, comprising the brain and spinal cord) and a peripheral nervous system (PNS, comprising sympathetic, parasympathetic, sensory, motor, and enteric neurons outside of the brain and spinal cord). Where an active agent according to the present invention is intended to act centrally (i.e., exert its effects via action on neurons in the CNS), the active agent must either be administered directly into the CNS or be capable of bypassing or crossing the blood-brain barrier. The blood-brain barrier is a capillary wall structure that effectively screens out all but selected categories of substances present in the blood, preventing their passage into the CNS. The unique morphologic characteristics of the brain capillaries that make up the blood-brain barrier are: 1) epithelial-like high resistance tight junctions which literally cement all endothelia of brain capillaries together within the blood-brain barrier regions of the CNS; and 2) scanty pinocytosis or transendothelial channels, which are abundant in endothelia of peripheral organs. Due to the unique characteristics of the blood-brain barrier, hydrophilic drugs and peptides that readily gain access to other tissues in the body are barred from entry into the brain or their rates of entry are very low.

The blood-brain barrier can be bypassed effectively by direct infusion of the active agent into the brain, or by intranasal administration or inhalation of formulations suitable for uptake and retrograde transport of the active agent by olfactory neurons.
The most common procedure for administration directly into the CNS is the implantation of a catheter into the ventricular system or intrathecal space. Alternatively, the active agent can be modified to enhance its transport across the blood-brain barrier. This generally requires some solubility of the drug in lipids, or other appropriate modification known to one of skill in the art. For example, the active agent may be truncated, derivatized, latentiated (converted from a hydrophilic drug into a lipid-soluble drug), conjugated to a lipophilic moiety or to a substance that is actively transported across the blood-brain barrier, or modified using standard means known to those skilled in the art. See, for example, Pardridge, Endocrine Reviews 7: 314-330 (1986) and U.S. Pat. No. 4,801,575.

Where an active agent according to the present invention is intended to act exclusively peripherally (i.e., exert its effects via action either on neurons in the PNS or directly on target tissues), it may be desirable to modify the compounds of the present invention such that they will not pass the blood-brain barrier. The principle of blood-brain barrier permeability can therefore be used to design active agents with selective potency for peripheral targets. Generally, a lipid-insoluble drug will not cross the blood-brain barrier, and will not produce effects on the CNS. A basic drug that acts on the nervous system may be altered to produce a selective peripheral effect by quaternization of the drug, which decreases its lipid solubility and makes it virtually unavailable for transfer to the CNS. For example, the charged antimuscarinic drug methscopalamine bromide has peripheral effects while the uncharged antimuscarinic drug scopolamine acts centrally. One of skill in the art can select and modify active agents of the present invention using well-known standard chemical synthetic techniques to add a lipid impermeable functional group such a quaternary amine, sulfate, carboxylate, phosphate, or sulfonium to prevent transport across the blood-brain barrier. Such modifications are by no means the only way in which active agents of the present invention may be modified to be impermeable to the blood-brain barrier; other well known pharmaceutical techniques exist and would be considered to fall within the scope of the present invention.

### Agents

Compounds useful in the present invention include any active agent as defined elsewhere herein. Such active agents include, for example, the α₂δ subunit calcium channel modulators gabapentin and pregabalin, as described elsewhere herein, as well as smooth muscle modulators, including antimuscarinics, β3 adrenergic agonists, spasmolytics, neurokinin receptor antagonists, bradykinin receptor antagonists, and nitric oxide donors, as described elsewhere herein.

Voltage gated calcium channels, also known as voltage dependent calcium channels, are multi-subunit membrane-spanning proteins which permit controlled calcium influx from an extracellular environment into the interior of a cell. Opening and closing (gating) of voltage gated calcium channels is controlled by a voltage sensitive region of the protein containing charged amino acids that move within an electric field. The movement of these charged groups leads to conformational changes in the structure of the channel resulting in conducting (open/activated) or non-conducting (closed/inactivated) states.

Voltage gated calcium channels are present in a variety of tissues and are implicated in several vital processes in animals. Changes in calcium influx into cells mediated through these calcium channels have been implicated in various human diseases such as epilepsy, stroke, brain trauma, Alzheimer's disease, multi-infarct dementia, other classes of dementia, Korsakoff's disease, neuropathy caused by a viral infection of the brain or spinal cord (e.g., human immunodeficiency viruses, etc.), amyotrophic lateral sclerosis, convulsions, seizures, Huntington's disease, amnesia, or damage to the nervous system resulting from reduced oxygen supply, poison, or other toxic substances (See, e.g., U.S. Pat. No. 5,312,928).

Voltage gated calcium channels have been classified by their electrophysiological and pharmacological properties as T; L, N, P and Q types (for reviews see McCleskey *et al.* (1991) *Curr. Topics Membr.* 39:295-326; and Dunlap *et al.* (1995) *Trends. Neurosci.* 18:89-98). Because there is some overlap in the biophysical properties of the high voltage-activated channels, pharmacological profiles are useful to further distinguish them. L-type channels are sensitive to dihydropyridine agonists and antagonists. N-type channels are blocked by the peptides ω-conotoxin GVIA and ω-conotoxin MVIIA, peptide toxins from the cone shell mollusks, *Conus geographus* and *Conus magus,* respectively. P-type channels are blocked by the peptide ω-agatoxin IVA from the venom of the funnel web spider, *Agelenopsis aperta,* although some studies have suggested that ω-agatoxin IVA also blocks N-type channels (Sidach at al. (2000) *J. Neurosci.* 20: 7174-82). A fourth type of high voltage-activated calcium channel (Q-type) has been described, although whether the Q- and P-type channels are distinct molecular entities is controversial *(Sather et al.(1995) Neuron* 11:291-303; *Stea et al.* (1994) *Proc. Natl. Acad. Sci. USA* 91:10576-10580; *Bourinet et al.* (1999) *Nature Neuroscience* 2:407-415).

Voltage gated calcium channels are primarily defined by the combination of different subunits: α₁, α₂, β, γ, and δ (see Caterall (2000) *Annu. Rev. Cell. Dev. Biol.* 16: 521-55). Ten types of α₁ subunits, four complexes, four β subunits, and two γ subunits are known (see Caterall, *Annu. Rev. Cell. Dev. Biol.,* supra; see also Klugbauer et al. (1999) *J. Neurosci.* 19: 684-691).

Based upon the combination of different subunits, calcium channels may be divided into three structurally and functionally related families: Caᵥ1, Caᵥ2, and Caᵥ3 (for reviews, see Caterall, *Annu. Rev. Cell. Dev. Biol.,* supra; Ertel *et al.* (2000) *Neuron* 25: 533-55). L-type currents are mediated by a Caᵥ1 family of α₁ subunits (see Caterall, *Annu. Rev. Cell. Dev. Biol.,* supra). Caᵥ2 channels form a distinct family with less than 40% amino acid sequence identity with Caᵥ1α₁ subunits (see Caterall, *Annu. Rev. Cell. Dev. Biol.,* supra). Cloned Caᵥ2.1 subunits conduct P- or Q-type currents that are inhibited by ω-agatoxin IVA (see Caterall, *Annu. Rev. Cell. Dev. Biol.,* supra; Sather *et al.* (1993) *Neuron* 11: 291-303; Stea *et al.* (1994) *Proc. Natl. Acad. Sci. USA* 91: 10576-80; Bourinet *et al.* (1999) *Nat. Neurosci.* 2: 407-15). Caᵥ2.2 subunits conduct N-type calcium currents and have a high affinity for ω-conotoxin GVIA, ω-conotoxin MVIIA, and synthetic versions of these peptides including Ziconotide (see Caterall, *Annu. Rev. Cell. Dev. Biol.,* supra; Dubel *et al.* (1992) *Proc. Natl. Acad. Sci. USA* 89:5058-62; Williams *et al.* (1992) *Science 257:* 389-95). Cloned Caᵥ2.3 subunits conduct a calcium current known as R-type and are resistant to organic antagonists specific for L-type calcium currents and peptide toxins specific for N-type or P/Q-type currents (see Caterall, *Annu. Rev. Cell. Dev. Biol.*, supra; Randall *et al.* (1995) *J. Neurosci.* 15: 2995-3012; Soong *et al.* (1994) *Science* 260: 1133-36; *Zhang et al.* (1993) *Neuropharmacology* 32: 1075-88).

Gabapentin (Neurontin, or 1-(aminomethyl) cyclohexaneacetic acid) is an anticonvulsant drug with a high binding affinity for some calcium channel subunits, and is represented by the following structure:

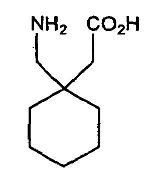

Gabapentin is one of a series of compounds of formula: 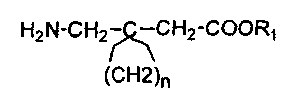 in which R₁ is hydrogen or a lower alkyl radical and n is 4, 5, or 6. Although gabapentin was originally developed as a GABA-mimetic compound to treat spasticity, gabapentin has no direct GABAergic action and does not block GABA uptake or metabolism. (For review, see *Rose et al.* (2002) *Analgesia* 57:451-462). Gabapentin has been found, however, to be an effective treatment for the prevention of partial seizures in patients who are refractory to other anticonvulsant agents (Chadwick (1991) *Gabapentin,* In Pedley T A, Meldrum B S (eds.), *Recent Advances in Epilepsy,* Churchill Livingstone, New York, pp. 211-222). Gabapentin and the related drug pregabalin may interact with the α₂δ subunit of calcium channels *(Gee et al.* (1996) *J. Biol. Chem.* 271: 5768-5776).

In addition to its known anticonvulsant effects, gabapentin has been shown to block the tonic phase of nociception induced by formalin and carrageenan, and exerts an inhibitory effect in neuropathic pain models of mechanical hyperalgesia and mechanical/thermal allodynia (Rose *et al.* (2002) *Analgesia* 57: 451-462). Double-blind, placebo-controlled trials have indicated that gabapentin is an effective treatment for painful symptoms associated with diabetic peripheral neuropathy, post-herpetic neuralgia, and neuropathic pain (see, e.g., Backonja *et al.* (1998) *JAMA* 280:1831-1836; Mellegers *et al.* (2001) *Clin. J. Pain* 17:284-95).

Pregabalin, (S)-(3-aminomethyl)-5-methylhexanoic acid or (S)-isobutyl GABA, is another GABA analog whose use as an anticonvulsant has been explored (Bryans *et al.* (1998) *J. Med. Chem.* 41:1838-1845). Pregabalin has been shown to possess even higher binding affinity for the α₂δ subunit of calcium channels than gabapentin (Bryans *et al.* (1999) *Med. Res. Rev.* 19:149-177).

Acetylcholine is a chemical neurotransmitter in the nervous systems of all animals. "Cholinergic neurotransmission" refers to neurotransmission that involves acetylcholine, and has been implicated in the control of functions as diverse as locomotion, digestion, cardiac rate, "fight or flight" responses, and learning and memory (Salvaterra (Feb. 2000) Acetylcholine. In *Encyclopedia of Life Sciences.* London: Nature Publishing Group, http:/www.els.net). Receptors for acetylcholine are classified into two general categories based on the plant alkaloids that preferentially interact with them: 1) nicotinic (nicotine binding); or 2) muscarinic (muscarine binding) (See, e.g., Salvaterra, Acetylcholine, supra).

The two general categories of acetylcholine receptors may be further divided into subclasses based upon differences in their pharmacological and electrophysiological properties. For example, nicotinic receptors are composed of a variety of subunits that are used to identify the following subclasses: 1) muscle nicotinic acetylcholine receptors; 2) neuronal nicotinic acetylcholine receptors that do not bind the snake venom α-bungarotoxin; and 3) neuronal nicotinic acetylcholine receptors that do bind the snake venom α-bimgarotoxin (Dani et al. (July 1999) Nicotinic Acetylcholine Receptors in Neurons. In *Encyclopedia of Life Sciences.* London: Nature Publishing Group, http:/www.els.net; Lindstrom (October 2001) Nicotinic Acetylcholine Receptors. In *Encyclopedia of Life Sciences.* London: Nature Publishing Group, http:/www.els.net). By contrast, muscarinic receptors may be divided into five subclasses, labeled M₁-M₅, and preferentially couple with specific G-proteins (M₁, M₃, and M₅ with G_{q}; M₂ and M₄ with Gᵢ/Gₒ) (Nathanson (July 1999) Muscarinic Acetylcholine Receptors. In *Encyclopedia of Life Sciences.* London: Nature Publishing Group, http:/www.els.net). In general, muscarinic receptors have been implicated in bladder function (See, e.g., Appell (2002) *Cleve. Clin. J. Med.* 69: 761-9; Diouf et al. (2002) *Bioorg. Med. Chem. Lett.* 12: 2535-9; Crandall (2001) *J. Womens Health Gend. Based Med.* 10: 735-43; Chapple (2000) *Urology* 55: 33-46).

Agents useful in the present invention include any antimuscarinic agent. Particularly useful in the methods of the present invention is oxybutynin, also known as 4-diethylaminio-2-butynyl phenylcyclohexyglycolate. It has the following structure:

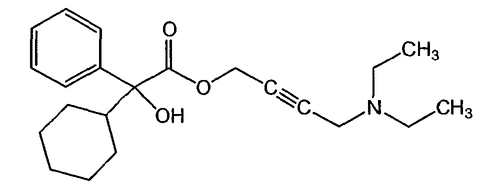

Ditropan^{®} (oxybutynin chloride) is the d,l racemic mixture of the above compound, which is known to exert antispasmodic effect on smooth muscle and inhibit the muscarinic action of acetylcholine on smooth muscle. Metabolites and isomers of oxybutynin have also been shown to have activity useful according to the present invention. Examples include, but are not limited to N-desethyl-oxybutynin and S-oxybutynin (see, e.g., US Patent Nos. 5,736,577 and 5,532,278).

Additional compounds that have been identified as antimuscarinic agents and are useful in the present invention include, but are not limited to:
a. Darifenacin (Daryon^{®}) or acids, salts, enantiomers, analogs, esters, amides, prodrugs, active metabolites, and derivatives thereof;
b. Solifenacin or acids, salts, enantiomers, analogs, esters, amides, prodrugs, active metabolites, and derivatives thereof;
c. YM-905 (solifenacin succinate) or acids, salts, enantiomers, analogs, esters, amides, prodrugs, active metabolites, and derivatives thereof;
d. Solifenacin monohydrochloride or acids, salts, enantiomers, analogs, esters, amides, prodrugs, active metabolites, and derivatives thereof;
e. Tolterodine (Detrol^{®}) or acids, salts, enantiomers, analogs, esters, amides, prodrugs, active metabolites, and derivatives thereof;
f. Propiverine (Detrunorm^{®}) or acids, salts, enantiomers, analogs, esters, amides, prodrugs, active metabolites, and derivatives thereof;
g. Propantheline bromide (Pro-Banthine^{®}) or acids, salts, enantiomers, analogs, esters, amides, prodrugs, active metabolites, and derivatives thereof;
h. Hyoscyamine sulfate (Levsin^{®}, Cystospaz^{®}) or acids, salts, enantiomers, analogs, esters, amides, prodrugs, active metabolites, and derivatives thereof;
i. Dicyclomine hydrochloride (Bentyl^{®}) or acids, salts, enantiomers, analogs, esters, amides, prodrugs, active metabolites, and derivatives thereof;
j. Flavoxate hydrochloride (Urispas^{®}) or acids, salts, enantiomers, analogs, esters, amides, prodrugs, active metabolites, and derivatives thereof;
k. d,l (racemic) 4- diethylamino-2-butynyl phenylcyclohexylglycolate or acids, salts, enantiomers, analogs, esters, amides, prodrugs, active metabolites, and derivatives thereof;
l. (R)-N,N-diisopropyl-3-(2-hydroxy-5-methylphenyl)-3-phenylpropanamine L-hydrogen tartrate or acids, salts, enantiomers, analogs, esters, amides, prodrugs, active metabolites, and derivatives thereof;
m. (+)-(1S,3'R)-quinuclidin-3'-yl 1-phenyl-1,2,3,4-tetrahydroisoquinoline-2-carboxylate monosuccinate or acids, salts, enantiomers, analogs, esters, amides, prodrugs, active metabolites, and derivatives thereof;
n. alpha(+)-4-(Dimethylamino)-3-methyl-1,2-diphenyl-2-butanol proprionate or acids, salts, enantiomers, analogs, esters, amides, prodrugs, active metabolites, and derivatives thereof;
o. 1-methyl-4-piperidyl diphenylpropoxyacetate or acids, salts, enantiomers, analogs, esters, amides, prodrugs, active metabolites, and derivatives thereof;
p. 3α-hydroxyspiro[1α H,5α H-nortropane-8,1'-pyrrolidinium benzilate or acids, salts, enantiomers, analogs, esters, amides, prodrugs, active metabolites, and derivatives thereof;
q. 4 amino-piperidine containing compounds as disclosed in Diouf *et al.* (2002) *Bioorg. Med. Chem. Lett.* 12: 2535-9;
r. pirenzipine or acids, salts, enantiomers, analogs, esters, amides, prodrugs, active metabolites, and derivatives thereof;
s. methoctramine or acids, salts, enantiomers, analogs, esters, amides, prodrugs, active metabolites, and derivatives thereof;
t. 4-diphenylacetoxy-N-methyl piperidine methiodide;
u. tropicamide or acids, salts, enantiomers, analogs, esters, amides, prodrugs, active metabolites, and derivatives thereof;
v. (2R)-N-[1-(6-aminopyridin-2-ylmethyl)piperidin-4-yl]-2-[(1R)-3,3-difluorocyclopentyl]-2-hydroxy-2-phenylacetamide or acids, salts, enantiomers, analogs, esters, amides, prodrugs, active metabolites, and derivatives thereof;
w. PNU-200577 ((R)-N, N-diisopropyl-3-(2-hydroxy-5-hydroxymethylphenyl)-3-phenylpropanamine) or acids, salts, enantiomers, analogs, esters, amides, prodrugs, active metabolites, and derivatives thereof;
x. KRP-197 (4-(2-methylimidazolyl)-2,2-diphenylbutyramide) or acids, salts, enantiomers, analogs, esters, amides, prodrugs, active metabolites, and derivatives thereof;
y. Fesoterodine or acids, salts, enantiomers, analogs, esters, amides, prodrugs, active metabolites, and derivatives thereof; and
z. SPM 7605 (the active metabolite of Fesoterodine), or acids, salts, enantiomers, analogs, esters, amides, prodrugs, active metabolites; and derivatives thereof.

The identification of further compounds that have antimuscarinic activity and would therefore be useful in the present invention can be determined by performing muscarinic receptor binding specificity studies as described by Nilvebrant (2002) *Pharmacol. Toxicol.* 90: 260-7 or cystometry studies as described by Modiri *et al.* (2002) *Urology* 59: 963-8.

Adrenergic receptors are cell-surface receptors for two major catecholamine hormones and neurotransmitters: noradrenaline and adrenaline. (Malbon *et al.* (Feb. 2000) Adrenergic Receptors. In *Encyclopedia of Life Sciences.* London: Nature Publishing Group, http:/www.els.net). Adrenergic receptors have been implicated in critical physiological processes, including blood pressure control, myocardial and smooth muscle contractility, pulmonary function, metabolism, and central nervous system activity (See, e.g., Malbon *et al.,* Adrenergic Receptors, supra). Two classes of adrenergic receptors have been identified, α and β, that may be further subdivided into three major families (α1, α2, and β), each with at least three subtypes (α1A, B, and, D; α₂A, B, and C; and, β1, β2, and β3) based upon their binding characteristics to different agonists and molecular cloning techniques. (See, e.g., Malbon *et al.,* Adrenergic Receptors, supra). It has been shown that β3 adrenergic receptors are expressed in the detrusor muscle, and that the detrusor muscle relaxes with a β3-agonist (Takeda, M. *et al.* (1999) *J.Pharmacol.Exp.Ther.* 258: 1367-1373), and in general, β3 adrenergic receptors have been implicated in bladder function (See, e.g., Takeda *et al.* (2002) *Neuourol. Urodyn.* 21: 558-65; Takeda *et al.* (2000) *J. Pharmacol. Exp. Ther.* 293: 939-45.

Other agents useful in the present invention include any β3 adrenergic agonist agent. Compounds that have been identified as β3 adrenergic agonist agents and are useful in the present invention include, but are not limited to:
a. TT-138 and phenylethanolamine compounds as disclosed in US Patent No. 6,069,176, PCT Publication No. WO 97/15549 and available from Mitsubishi Pharma Corp., or acids, salts, esters, amides, prodrugs, active metabolites, and other derivatives thereof;
b. FR-149174 and propanolamine derivatives as disclosed in US Patent Nos. 6,495,546 and 6,391,915 and available from Fujisawa Pharmaceutical Co., or acids, salts, esters, amides, prodrugs, active metabolites, and other derivatives thereof;
c. KUC-7483, available from Kissei Pharmaceutical Co., or acids, salts, esters, amides, prodrugs, active metabolites, and other derivatives thereof,
d. 4'-hydroxynorephedrine derivatives such as 2- 2-chloro-4-(2-((1S,2R)-2-hydroxy-2-(4-hydroxyphenyl)-1-methylethylamino)ethyl)phenoxy acetic acid as disclosed in Tanaka *et al.* (2003) *J. Med. Chem.* 46: 105-12 or acids, salts, esters, amides, prodrugs, active metabolites, and other derivatives thereof;
e. 2-amino-1-phenylethanol compounds, such as BRL35135 ((R*R*)-(.+-.)-[4-[2-[2-(3-chlorophenyl)-2-ydroxyethylamino]propyl]phenox y]acetic acid methyl ester hydrobromide salt as disclosed in Japanese Patent Publication No. 26744 of 1988 and European Patent Publication No. 23385), and SR58611A ((RS)-N-(7-ethoxycarbonylmethoxy-1,2,3,4-tetrahydronaphth-2-yl)-2-(3-chlor ophenyl)-2-hydroxyethanamine hydrochloride as disclosed in Japanese Laid-open Patent Publication No. 66152 of 1989 and European Laid-open Patent Publication No. 255415) or acids, salts, esters, amides, prodrugs, active metabolites, and other derivatives thereof;
f. GS 332 (Sodium (2R)-[3-[3-[2-(3 Chlorophenyl)-2-hydroxyethylamino] cyclohexyl]phenoxy] acetate) as disclosed in Iizuka *et al.* (1998) *J. Smooth Muscle Res. 34:* 139-49 or acids, salts, esters, amides, prodrugs, active metabolites, and other derivatives thereof;
g. BRL-37,344 (4-[-[(2-hydroxy-(3-chlorophenyl) ethyl)-amino]propyl]phenoxyacetate) as disclosed in Tsujii *et al.* (1998) *Physiol. Behav.* 63: 723-8 and available from GlaxoSmithKline or acids, salts, esters, amides, prodrugs, active metabolites, and other derivatives thereof;
h. BRL-26830A as disclosed in Takahashi *et al.* (1992) *Jpn Circ. J.* 56: 936-42 and available from GlaxoSmithKline or acids, salts, esters, amides, prodrugs, active metabolites, and other derivatives thereof;
i. CGP 12177 (4-[3-t-butylamino-2-hydroxypropoxy]benzimidazol-2- one) (a β1/β2 adrenergic antagonist reported to act as an agonist for the β3 adrenergic receptor) as described in Tavernier *et al.* (1992) J. Pharmacol. Exp. Ther. 263: 1083-90 and available from Ciba-Geigy or acids, salts, esters, amides, prodrugs, active metabolites, and other derivatives thereof;
j. CL 316243 (R,R-5-[2-[[2-(3-chlorophenyl)-2-hydroxyethyl]amino]propyl]-1,3- benzodioxole-2,2-dicarboxylate) as disclosed in Berlan *et al.* (1994) *J. Pharmacol. Exp. Ther.* 268: 1444-51 or acids, salts, esters, amides, prodrugs, active metabolites, and other derivatives thereof;
k. Compounds having β3 adrenergic agonist activity as disclosed in US Patent Application 20030018061 or acids, salts, esters, amides, prodrugs, active metabolites, and other derivatives thereof;
l. ICI 215,001 HCl ((*S*)-4-[2-Hydroxy-3-phenoxypropylaminoethoxy]phenoxyacetic acid hydrochloride) as disclosed in Howe (1993) *Drugs Future* 18: 529 and available from AstraZeneca/ICI Labs or acids, salts, enantiomers, analogs, esters, amides, prodrugs, active metabolites, and derivatives thereof;
m. ZD 7114 HCl (ICI D7114; (*S*)-4-[2-Hydroxy-3-phenoxypropylaminoethoxy]-*N*-(2-methoxyethyl)phenoxyacetamide HCl) as disclosed in Howe (1993) *Drugs Future* 18: 529 and available from AstraZeneca/ICI Labs or acids, salts, enantiomers, analogs, esters, amides, prodrugs, active metabolites, and derivatives thereof;
n. Pindolol (1-(1*H*-Indol-4-yloxy)-3-[(1-methylethyl)amino]-2-propanol) as disclosed in Blin *et al* (1994) *Mol.Pharmacol.* 44: 1094 or acids, salts, enantiomers, analogs, esters, amides, prodrugs, active metabolites, and derivatives thereof;
o. (*S*)-(-)-Pindolol ((*S*)-1-(1*H*-indol-4-yloxy)-3-[(1-methylethyl)amino]-2-propanol) as disclosed in Walter *et al* (1984) *Naunyn-Schmied.Arch.Pharmacol.* 327: 159 and Kalkman (1989) *Eur.J.Pharmacol.* 173: 121 or acids, salts, enantiomers, analogs, esters, amides, prodrugs, active metabolites, and derivatives thereof;
p. SR 59230A HCl (1-(2-Ethylphenoxy)-3-[[(1*S*)-1,2,3,4-tetrahydro-1-naphthalenylJamino]-(2S)-2-propanol hydrochloride) as disclosed in *Manara et al.* (1995) *Pharmacol. Comm.* 6: 253 and Manara *et al.* (1996) *Br. J. Pharmacol.* 117: 435 and available from Sanofi-Midy or acids, salts, enantiomers, analogs, esters, amides, prodrugs, active metabolites, and derivatives thereof;
q. SR 58611 (*N*[2s)7-carb-ethoxymethoxy-1,2,3,4-tetrahydronaphth]-(2r)-2-hydroxy-2(3-chlorophenyl) ethamine hydrochloride) as disclosed in Gauthier *et al.* (1999) *J*. *Pharmacol. Exp. Ther.* 290: 687-693 and available from Sanofi Research; and
r. YM178 available from Yamanouchi Pharmaceutical Co. or acids, salts, esters, amides, prodrugs, active metabolites, and other derivatives thereof.

The identification of further compounds that have β3 adrenergic agonist activity and would therefore be useful in the present invention can be determined by performing radioligand binding assays and/or contractility studies as described by Zilberfarb *et al:* (1997) *J. Cell Sci.* 110: 801-807; Takeda *et al.* (1999) *J. Pharmacol. Exp. Ther.* 288: 1367-1373; and Gauthier *et al.* (1999) *J. Pharmacol. Exp. Ther.* 290: 687-693.

Spasmolytics are compounds that relieve or prevent muscle spasms, especially of smooth muscle. In general, spasmolytics have been implicated as having efficacy in the treatment of bladder disorders (See. e.g., Takeda *et al.* (2000) *J*. *Pharmacol. Exp. Ther.* 293: 939-45).

Other agents useful in the present invention include any spasmolytic agent. Compounds that have been identified as spasmolytic agents and are useful in the present invention include, but are not limited to:
a. α-α-diphenylacetic acid-4-(N-methyl-piperidyl) esters as disclosed in US Patent No. 5,897,875 or acids, salts, enantiomers, analogs, esters, amides, prodrugs, active metabolites, and derivatives thereof;
b. Human and porcine spasmolytic polypeptides in glycosylated form and variants thereof as disclosed in US Patent No. 5,783,416 or acids, salts, enantiomers, analogs, esters, amides, prodrugs, active metabolites, and derivatives thereof;
c. Dioxazocine derivatives as disclosed in US Patent No. 4,965,259 or acids, salts, enantiomers, analogs, esters, amides, prodrugs, active metabolites, and derivatives thereof;
d. Quaternary 6,11-dihydro-dibenzo-[b,e]-thiepine-11-N-alkylnorscopine ethers as disclosed in US Patent No. 4,608,377 or acids, salts, enantiomers, analogs, esters, amides, prodrugs, active metabolites, and derivatives thereof;
e. Quaternary salts of dibenzo[1,4]diazepinones, pyrido-[1,4]benzodiazepinones, pyrido[1,5]benzodiazepinones as disclosed in US Patent No. 4,594,190 or acids, salts, enantiomers, analogs, esters, amides, prodrugs, active metabolites, and derivatives thereof;
f. Endo-8,8-dialkyl-8-azoniabicyclo (3.2.1) octane-6,7-exo-epoxy-3-alkyl-carboxylate salts as disclosed in US Patent No. 4,558,054 or acids, salts, enantiomers, analogs, esters, amides, prodrugs, active metabolites, and derivatives thereof;
g. Pancreatic spasmolytic polypeptides as disclosed in US Patent No. 4,370,317 or acids, salts, enantiomers, analogs, esters, amides, prodrugs, active metabolites, and derivatives thereof;
h. Triazinones as disclosed in US Patent No. 4,203,983 or acids, salts, enantiomers, analogs, esters, amides, prodrugs, active metabolites, and derivatives thereof;
i. 2-(4-Biphenylyl)-N-(2-diethylamino alkyl)propionamide as disclosed in US Patent No. 4,185,124 or acids, salts, enantiomers, analogs, esters, amides, prodrugs, active metabolites, and derivatives thereof;
j. Piperazino-pyrimidines as disclosed in US Patent No. 4,166,852 or acids, salts, enantiomers, analogs, esters, amides, prodrugs, active metabolites, and derivatives thereof;
k. Aralkylamino carboxylic acids as disclosed in US Patent No. 4,163,060 or acids, salts, enantiomers, analogs, esters, amides, prodrugs, active metabolites, and derivatives thereof;
l. Aralkylamino sulfones as disclosed in US Patent No. 4,034,103 or acids, salts, enantiomers, analogs, esters, amides, prodrugs, active metabolites, and derivatives thereof;
m. Smooth muscle spasmolytic agents as disclosed in US Patent No. 6,207,852 or acids, salts, enantiomers, analogs, esters, amides, prodrugs, active metabolites, and derivatives thereof; and
n. Papaverine or acids, salts, enantiomers, analogs, esters, amides, prodrugs, active metabolites, and derivatives thereof.
The identification of further compounds that have spasmolytic activity and would therefore be useful in the present invention can be determined by performing bladder strip contractility studies as described in US Patent No. 6,207,852; Noronha-Blob *et al.* (1991) *J. Pharmacol. Exp. Ther.*256: 562-567; and/or Kachur *et al.* (1988) *J. Pharmacol. Exp. Then.*247: 867-872.

Tachykinins (TKs) are a family of structurally related peptides that include substance P, neurokinin A (NKA) and neurokinin B (NKB). Neurons are the major source of TKs in the periphery. An important general effect of TKs is neuronal stimulation, but other effects include endothelium-dependent vasodilation, plasma protein extravasation, mast cell recruitment and degranulation and stimulation of inflammatory cells (See Maggi, C. A. (1991) *Gen. Pharmacol.,* 22: 1-24). In general, tachykinin receptors have been implicated in bladder function (See, e.g., Kamo *et al.* (2000) *Eur. J. Pharmacol.* 401: 235-40 and Omhura *et al.* (1997) *Urol. Int.* 59: 221-5).

Substance P activates the neurokinin receptor subtype referred to as NK₁. Substance P is an undecapeptide that is present in sensory nerve terminals. Substance P is known to have multiple actions that produce inflammation and pain in the periphery after C-fiber activation, including vasodilation, plasma extravasation and degranulation of mast cells (Levine, J. D. *et. al.* (1993) *J*. *Neurosci.* 13: 2273).

Neurokinin A is a peptide which is colocalized in sensory neurons with substance P and which also promotes inflammation and pain. Neurokinin A activates the *specific neurokinin* receptor referred to as NK₂ (Edmonds-Alt, S., *et. al.* (1992) *Life Sci.* 50: PL101). In the urinary tract, TKs are powerful spasmogens acting through only the NK₂ receptor in the human bladder, as well as the human urethra and ureter (Maggi, C. A. (1991) *Gen. Pharmacol.,* 22: 1-24).

Other agents useful in the present invention include any neurokinin receptor antagonist agent. Suitable neurokinin receptor antagonists for use in the present invention that act on the NK₁ receptor include, but are not limited to: 1-imino-2-(2-methoxy-phenyl)-ethyl)-7,7-diphenyl-4-perhydroisoindolone(3aR ,7aR) ("RP 67580"); 2S,3S-cis-3-(2-methoxybenzylamino)-2-benzhydrylquinuclidine ("CP 96,345"); and (aR,9R)-7-[3,5-bis(trifluoromethyl)benzyl]-8,9,10, 11-tetrahydro-9-methyl-5-(4-methylphenyl)-7H-[1,4]diazocino[2,1-g] [1,7]naphthyridine-6,13-dione)("TAh-637"). Suitable neurokinin receptor antagonists for use in the present invention that act on the NK₂ receptor include but are not limited to: ((S)-N-methyl-N-4-(4-acetylamino-4-phenylpiperidino)-2-(3,4-dichloropheny 1)butylbenzamide ("SR 48968"); Met-Asp-Trp-Phe-Dap-Leu ("MEN 10,627"); and cyc(Gln-Trp-Phe-Gly-Leu-Met) ("L 659,877"). Suitable neurokinin receptor antagonists for use in the present invention also include acids, salts, esters, amides, prodrugs, active metabolites, and other derivatives of any of the agents mentioned above. The identification of further compounds that have neurokinin receptor antagonist activity and would therefore be useful in the present invention can be determined by performing binding assay studies as described in Hopkins *et al.* (1991) *Biochem. Biophys. Res. Comm.* 180: 1110-1117; and Aharony *et al.* (1994) Mol. Pharmacol. 45: 9-19.

Bradykinin receptors generally are divided into bradykinin₁ (B₁) and bradykinin₂ (B₂) subtypes. Studies have shown that acute peripheral pain and inflammation produced by bradykinin are mediated by the B₂ subtype whereas bradykinin-induced pain in the setting of chronic inflammation is mediated via the B₁ subtype (Perkins, M. N., *et. al.* (1993) *Pain* 53: 191-97); Dray, A., *et. al.* (1993) *Trends Neurosci.* 16: 99-104). In general, bradykinin receptors have been implicated in bladder function (See, e.g., Meini *et al.* (2000) *Eur. J. Pharmacol.* 388: 177-82 and Belichard *et al.* (1999) *Br. J. Pharmacol.* 128: 213-9).

Other agents useful in the present invention include any bradykinin receptor antagonist agent. Suitable bradykinin receptor antagonists for use in the present invention that act on the B₁ receptor include but are not limited to: des-arg¹⁰HOE 140 (available from Hoechst Pharmaceuticals) and des-Arg⁹bradykinin (DABK). Suitable bradykinin receptor antagonists for use in the present invention that act on the B₂ receptor include but are not limited to: D-Phe⁷-BK; D-Arg-(Hyp³ -Thi^{5,8} -D-Phe⁷)-BK ("NPC 349"); D-Arg-(Hyp³-D-Phe⁷)-BK ("NPC 567"); D-Arg-(Hyp³ -Thi⁵ -D-Tic⁷⁻Oic⁸)-BK ("HOE 140"); H-DArg-Arg-Pro-Hyp-Gly-Thi-c(Dab-DTic-Oic-Arg)c(7gamma-10alpha)("MEN11270"); H-DArg-Arg-Pro-Hyp-Gly-Thi-Ser-DTic-Oic-Arg-OH("Icatibant"); (E)-3-(6-acetamido-3-pyridyl)-N-[N-[2,4-dichloro-3-[(2-methyl-8- quinolinyl) oxymethyl] phenyl]-N-methylaminocarbonylmethyl]acrylamide ("FR173567"); and WIN 64338. These compounds are more fully described in Perkins, M. N., *et. al., Pain, supra;* Dray, A., *et. al., Trends Neurosci., supra;* and Meini *et al.* (2000) *Eur. J. Pharmacol.* 388: 177-82. Suitable neurokinin receptor antagonists for use in the present invention also include acids, salts, esters, amides, prodrugs, active metabolites, and other derivatives of any of the agents mentioned above. The identification of further compounds that have bradykinin receptor antagonist activity and would therefore be useful in the present invention can be determined by performing binding assay studies as described in Manning *et al.* (1986) *J Pharmacol. Exp. Ther.* 237: 504 and US Patent No. 5,686,565.

Nitric oxide donors may be included in the present invention particularly for their anti-spasm activity. Nitric oxide (NO) plays a critical role as a molecular mediator of many physiological processes, including vasodilation and regulation of normal vascular tone. The action of NO is implicated in intrinsic local vasodilation mechanisms. NO is the smallest biologically active molecule known and is the mediator of an extraordinary range of physiological processes (Nathan (1994) *Cell* 78: 915-918; Thomas (1997) *Neurosurg. Focus* 3: Article 3). NO is also a known physiologic antagonist of endothelin-1, which is the most potent known mammalian vasoconstrictor, having at least ten times the vasoconstrictor potency of angiotensin II (Yanagisawa *et al.* (1988) *Nature* 332: 411-415; Kasuya *et al.* (1993) *J. Neurosurg.* 79: 892-898; Kobayashi *et al.,* (1991) *Neurosurgery* 28: 673-679). The biological half-life of NO is extremely short (Morris *et al.* (1994) *Am. J. Physiol.* 266: E829-E839; Nathan (1994) *Cell* 78: 915-918). NO accounts entirely for the biological effects of endothelium-derived relaxing factor (EDRF) and is an extremely potent vasodilator that is believed to work through the action of cGMP-dependent protein kinases to effect vasodilation (Henry *et al.* (1993) *FASEB J.* 7: 1124-1134; Nathan (1992) *FASEB J.* 6: 3051-3064; Palmer *et al.,* (1987) *Nature* 327: 524-526; Snyder *et al.* (1992) *Scientific American* 266: 68-77).

Within endothelial cells, an enzyme known as NO synthase (NOS) catalyzes the conversion of L-arginine to NO which acts as a diffusible second messenger and mediates responses in adjacent smooth muscle cells. NO is continuously formed and released by the vascular endothelium under basal conditions which inhibits contractions and controls basal coronary tone and is produced in the endothelium in response to various agonists (such as acetylcholine) and other endothelium dependent vasodilators. Thus, regulation of NOS activity and the resultant levels of NO are key molecular targets controlling vascular tone (Muramatsu *et. al.* (1994) *Coron. Artery Dis.* 5: 815-820).

Other agents useful in the present invention include any nitric oxide donor agent. Suitable nitric oxide donors for the practice of the present invention include but are not limited to:
a. Nitroglycerin or acids, salts, enantiomers, analogs, esters, amides, prodrugs, active metabolites, and derivatives thereof;
b. Sodium nitroprusside or acids, salts, enantiomers, analogs, esters, amides, prodrugs, active metabolites, and derivatives thereof;
c. FK 409 (NOR-3) or acids, salts, enantiomers, analogs, esters, amides, prodrugs, active metabolites, and derivatives thereof;
d. FR 144420 (NOR-4) or acids, salts, enantiomers, analogs, esters, amides, prodrugs, active metabolites, and derivatives thereof;
e. 3-morpholinosydnonimine or acids, salts, enantiomers, analogs, esters, amides, prodrugs, active metabolites, and derivatives thereof;
f. Linsidomine chlorohydrate ("SIN-1") or acids, salts, enantiomers, analogs, esters, amides, prodrugs, active metabolites, and derivatives thereof;
g. S-nitroso-N-acetylpenicillamine ("SNAP") or acids, salts, enantiomers, analogs, esters, amides, prodrugs, active metabolites, and derivatives thereof;
h. AZD3582 (CINOD lead compound, available from NicOx S.A.) or acids, salts, enantiomers, analogs, esters, amides, prodrugs, active metabolites, and derivatives thereof;
i. NCX 4016 (available from NicOx S.A.) or acids, salts, enantiomers, analogs, esters, amides, prodrugs, active metabolites, and derivatives thereof;
j. NCX 701 (available from NicOx S.A.) or acids, salts, enantiomers, analogs, esters, amides, prodrugs, active metabolites, and derivatives thereof;
k. NCX 1022 (available from NicOx S.A.) or acids, salts, enantiomers, analogs, esters, amides, prodrugs, active metabolites, and derivatives thereof;
l. HCT 1026 (available from NicOx S.A.) or acids, salts, enantiomers, analogs, esters, amides, prodrugs, active metabolites, and derivatives thereof;
m. NCX 1015 (available from NicOx S.A.) or acids, salts, enantiomers, analogs, esters, amides, prodrugs, active metabolites, and derivatives thereof;
n. NCX 950 (available from NicOx S.A.) or acids, salts, enantiomers, analogs, esters, amides, prodrugs, active metabolites, and derivatives thereof;
o. NCX 1000 (available from NicOx S.A.) or acids, salts, enantiomers, analogs, esters, amides, prodrugs, active metabolites, and derivatives thereof;
p. NCX 1020 (available from NicOx S.A.) or acids, salts, enantiomers, analogs, esters, amides, prodrugs, active metabolites, and derivatives thereof;
q. AZD 4717 (available from NicOx S.A.) or acids, salts, enantiomers, analogs, esters, amides, prodrugs, active metabolites, and derivatives thereof;
r. NCX 1510/NCX 1512 (available from NicOx S.A.) or acids, salts, enantiomers, analogs, esters, amides, prodrugs, active metabolites, and derivatives thereof;
s. NCX 2216 (available from NicOx S.A.) or acids, salts, enantiomers, analogs, esters, amides, prodrugs, active metabolites, and derivatives thereof;
t. NCX 4040 (available from NicOx S.A.) or acids, salts, enantiomers, analogs, esters, amides, prodrugs, active metabolites, and derivatives thereof;
u. Nitric oxide donors as disclosed in U.S. Patent No. 5,155,137 or acids, salts, enantiomers, analogs, esters, amides, prodrugs, active metabolites, and derivatives thereof;
v. Nitric oxide donors as disclosed in U.S. Patent No. 5,366,997 or acids, salts, enantiomers, analogs, esters, amides, prodrugs, active metabolites, and derivatives thereof;
w. Nitric oxide donors as disclosed in U.S. Patent No. 5,405,919 or acids, salts, enantiomers, analogs, esters, amides, prodrugs, active metabolites, and derivatives thereof;
x. Nitric oxide donors as disclosed in U.S. Patent No. 5,650,442 or acids, salts, enantiomers, analogs, esters, amides, prodrugs, active metabolites, and derivatives thereof;
y. Nitric oxide donors as disclosed in U.S. Patent No. 5,700,830 or acids, salts, enantiomers, analogs, esters, amides, prodrugs, active metabolites, and derivatives thereof;
z. Nitric oxide donors as disclosed in U.S. Patent No. 5,632,981 or acids, salts, enantiomers, analogs, esters, amides, prodrugs, active metabolites, and derivatives thereof;
aa. Nitric oxide donors as disclosed in U.S. Patent No. 6,290,981 or acids, salts, enantiomers, analogs, esters, amides, prodrugs, active metabolites, and derivatives thereof;
bb. Nitric oxide donors as disclosed in U.S. Patent No. 5,691,423 or acids, salts, enantiomers, analogs, esters, amides, prodrugs, active metabolites, and derivatives thereof;
cc. Nitric oxide donors as disclosed in U.S. Patent No. 5,721,365 or acids, salts, enantiomers, analogs, esters, amides, prodrugs, active metabolites, and derivatives thereof;
dd. Nitric oxide donors as disclosed in U.S. Patent No.5,714,511 or acids, salts, enantiomers, analogs, esters, amides, prodrugs, active metabolites, and derivatives thereof;
ee. Nitric oxide donors as disclosed in U.S. Patent No. 6,511,911 or acids, salts, enantiomers, analogs, esters, amides, prodrugs, active metabolites, and derivatives thereof; and
ff. Nitric oxide donors as disclosed in U.S. Patent No. 5,814,666.
The identification of further compounds that have nitric oxide donor activity and would therefore be useful in the present invention can be determined by release profile and/or induced vasospasm studies as described in US Patent Nos. 6,451,337 and 6,358,536, as well as Moon (2002) *IBJU Int.* 89: 942-9 and Fathian-Sabet *et al.* (2001) *J. Urol.* 165: 1724-9.

### Enantiomers and Diasteromers

Many organic compounds exist in optically active forms, i.e., they have the ability to rotate the plane of plane-polarized light. In describing an optically active compound the prefixes R and S are used to denote the absolute configuration of the molecule about its chiral center(s). The prefixes D and L, or (+) or (-), designate the sign of rotation of plane-polarized light by the compound, with L or (-) meaning that the compound is levorotatory. In contrast, a compound prefixed with D or (+) is dextrorotatory. There is no correlation between nomenclature for the absolute stereochemistry and for the rotation of an enantiomer. Thus, D-lactic acid is the same as (-)-lactic acid, and L-lactic acid is the same as (+)-lactic acid. For a given chemical structure, each of a pair of enantiomers are identical except that they are non-superimposable mirror images of one another. A specific stereoisomer may also be referred to as an enantiomer, and a mixture of such isomers is often called an enantiomeric, or racemic, mixture.

Stereochemical purity is important in the pharmaceutical field, where many of the most often prescribed drugs exhibit chirality. For example, the L-enantiomer of the beta-adrenergic blocking agent, propranolol, is known to be 100 times more potent than its D-enantiomer. Additionally, optical purity is important in the pharmaceutical drug field because certain isomers have been found to impart a deleterious effect, rather than an advantageous or inert effect. For example, it is believed that the D-enantiomer of thalidomide is a safe and effective sedative when prescribed for the control of morning sickness during pregnancy, whereas its corresponding L-enantiomer is believed to be a potent teratogen.

When two chiral centers exist in one molecule, there are four possible stereoisomers: (R,R), (S,S), (R,S), and (S,R). Of these, (R,R) and (S,S) are an example of a pair of enantiomers (mirror images of each other), which typically share chemical properties and melting points just like any other enantiomeric pair. The mirror images of (R,R) and (S,S) are not, however, superimposable on (R,S) and (S,R). This relationship is called diastereoisomeric, and the (S,S) molecule is a diastereoisomer of the (R,S) molecule, whereas the (R,R) molecule is a diastereoisomer of the (S,R) molecule.

An example of a compound with two chiral centers is the antimuscarinic solifenacin. Solifenacin is described in U.S. Patent No. 6,174,896 and is represented by the following chemical formula: 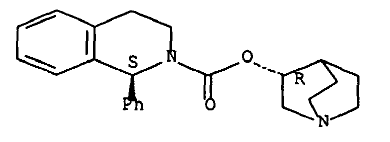 Because solifenacin has two chiral centers, diastereomers as well as enantiomers exist for this molecule (see U.S. Patent No. 6,174,896). Solifenacin succinate (development number YM-905) is a salt form of solifenacin that is co-promoted as Vesicare^{®} by Yamanouchi Pharmaceutical Co., Ltd. (through Yamanouchi Pharma America) and GlaxoSmithKline as an investigational muscarinic antagonist that is thought to act on receptors in the smooth muscle of the bladder. Solifenacin was discovered and developed by Yamanouchi, and a New Drug Application was submitted to the U.S. Food and Drug Administration by YPA in December 2002 for solifenacin succinate. A market authorization application for Vesicare^{®} was submitted in Europe in January 2003, and Yamanouchi has initiated Phase III clinical trials for Vesicare^{®} in Japan. Other salt forms of solifenacin have also been specifically described by Yamanouchi, including solifenacin monohydrochloride (development number YM-53705).

For use in the present invention, any diastereomer or enantiomer of an active agent as disclosed herein, can be administered to treat painful and non-painful lower urinary tract disorders and associated irritative symptoms in normal and spinal cord injured patients.

### Formulations

Formulations of the present invention may include, but are not limited to, continuous, as needed, short-term, rapid-offset, controlled release, sustained release, delayed release, and pulsatile release formulations.

Compositions of the invention comprise the α₂δ subunit calcium channel modulator gabapentin or pregabalin in combination with one or more compounds with smooth muscle modulatory effects including at least an antimuscarinic (particularly those that do not have an amine embedded in an 8-azabicyclo[3.2.1]octan-3-ol skeleton). β3 adrenergic agonists, spasmolytics, neurokinin receptor antagonists, bradykinin receptor antagonists, and nitric oxide donors are further examples of compounds with smooth muscle modulatory effects which may be included. The compositions are administered in therapeutically effective amounts to a patient in need thereof for treating and/or alleviating the symptoms associated with painful and non-painful lower urinary tract disorders in normal and spinal cord injured patients. It is recognized that the compositions may be administered by any means of administration as long as an effective amount for treating and/or alleviating the symptoms associated with painful and non-painful symptoms associated with lower urinary tract disorders in normal and spinal cord injured patients is delivered.

Salts, esters, amides, prodrugs and other derivatives of the active agents may be prepared using standard procedures known to those skilled in the art of synthetic organic chemistry and described, for example, by J. March, Advanced Organic Chemistry: Reactions, Mechanisms and Structure, 4th Ed. (New York: Wiley-Interscience, 1992). For example, acid addition salts are prepared from the free base using conventional methodology, and involves reaction with a suitable acid. Suitable acids for preparing acid addition salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, e.g., hydrochloric acid, hydrobromic acid, sulfuric acid, nitric acid, phosphoric acid, and the like. An acid addition salt may be reconverted to the free base by treatment with a suitable base. Particularly preferred acid addition salts of the active agents herein are salts prepared with organic acids. Conversely, preparation of basic salts of acid moieties which may be present on an active agent are prepared in a similar manner using a pharmaceutically acceptable base such as sodium hydroxide, potassium hydroxide, ammonium hydroxide, calcium hydroxide, trimethylamine, or the like.

Preparation of esters involves functionalization of hydroxyl and/or carboxyl groups that may be present within the molecular structure of the drug. The esters are typically acyl-substituted derivatives of free alcohol groups, i.e., moieties that are derived from carboxylic acids of the formula RCOOH where R is alkyl, and preferably is lower alkyl. Esters can be reconverted to the free acids, if desired, by using conventional hydrogenolysis or hydrolysis procedures. Amides and prodrugs may also be prepared using techniques known to those skilled in the art or described in the pertinent literature. For example, amides may be prepared from esters, using suitable amine reactants, or they may be prepared from an anhydride or an acid chloride by reaction with ammonia or a lower alkyl amine. Prodrugs are typically prepared by covalent attachment of a moiety, which results in a compound that is therapeutically inactive until modified by an individual's metabolic system.

One set of formulations for gabapentin are those marketed by Pfizer Inc. under the brand name Neurontin^{®}. Neurontin^{®} Capsules, Neurontin^{®} Tablets, and Neurontin^{®} Oral Solution are supplied either as imprinted hard shell capsules containing 100 mg, 300 mg, and 400 mg of gabapentin, elliptical film-coated tablets containing 600 mg and 800 mg of gabapentin or an oral solution containing 250 mg/5 mL of gabapentin. The inactive ingredients for the capsules are lactose, cornstarch, and talc. The 100 mg capsule shell contains gelatin and titanium dioxide. The 300 mg capsule shell contains gelatin, titanium dioxide, and yellow iron oxide. The 400 mg capsule shell contains gelatin, red iron oxide, titanium dioxide, and yellow iron oxide. The inactive ingredients for the tablets are poloxamer 407, copolyvidonum, cornstarch, magnesium stearate, hydroxypropyl cellulose, talc, candelilla wax and purified water. The inactive ingredients for the oral solution are glycerin, xylitol, purified water and artificial cool strawberry anise flavor. In addition to these formulations, gabapentin and formulations are generally described in the following patents: US 6,683,112; US 6,645,528; US 6,627,211; US 6,569,463; US 6,544,998; US 6,531,509; 6,495,669; US 6,465,012; US 6,346,270; US 6,294,198; US 6,294,192; US 6,207,685; US 6,127,418; US 6,024,977; US 6,020,370; US 5,906,832; US 5,876,750; and US 4,960,931.

One set of formulations for oxybutynin are those marketed by Ortho-McNeil Pharmaceuticals, Inc. under the brand name Ditropan^{®}. Ditropan^{®} tablets are supplied containing 5 mg/tablets of the active ingredient, oxybutynin chloride, and the inactive ingredients anhydrous lactose, microcrystalline cellulose, calcium stearate, and FD&C blue #1 lake. Ditropan^{®} syrup is supplied as 5mg/5mL of the active ingredient, oxybutynin chloride, and the inactive ingredients citric acid, FD&C green #3, flavor, glycerin, methylparaben, sodium citrate, sorbitol, sucrose, and water. Ditropan XL^{®} is an extended release tablet form of Ditropan^{®} supplied containing either 5 mg (pale yellow color) of oxybutynin chloride, 10 mg (pink color) of oxybutynin chloride, or 15 mg (gray color) of oxybutynin chloride. Inactive ingredients are cellulose acetate, hydroxypropyl methylcellulose, lactose, magnesium stearate, polyethylene glycol, polyethylene oxide, synthetic iron oxides, titanium dioxide, polysorbate 80, sodium chloride, and butylated hydroxytoluene.

Oxybutynin is also supplied by Watson Pharmaceuticals under the brand name Oxytrol^{®} (oxybutynin transdermal system). Oxytrol^{®} is a transdermal patch designed to deliver oxybutynin continuously and consistently over a 3 to 4 day interval. It is supplied as a 39 cm² patch containing 36 mg of oxybutynin, which is designed to deliver 3.9 mg/day. The patch is worn continuously, and a new patch is applied every 3 to 4 days.

A formulation useful in the present invention comprises a combination of gabapentin and oxybutynin chloride. The combination can be supplied in various pharmaceutical composition and dosage forms as described herein. One formulation for supplying the combination is in a tablet formulation. Additional formulations for the combination of the present invention, such as capsules, syrups, etc. are also envisioned for delivery of the combination, and any description of tablet formulations is in no way meant to be limiting of possible delivery modes for the combination of the present invention.

Tablet formulations useful for supplying the gabapentin/oxybutynin combination useful in the present invention can comprise, in addition to the active ingredients in combination, functional excipients. Such excipients as are useful for preparing pharmaceutical compositions in a tablet formulation are known in the art and include compounds known to be useful as fillers, binders, lubricants, disintegrants, diluents, coatings, plastizers, glidants, compression aids, stabilizers, sweeteners, solubilizers, and other excipients that would be known to one of skill in the pharmaceutical arts.

The active ingredients of the combination useful in the present invention (gabapentin and oxybutynin) can be combined, particularly in tablet form, according to ratios provided herein. The relative ratio of the active ingredients of the combination for use in the present invention is about 1:1 to about 1:800, oxybutynin and gabapentin respectively, more preferably about 2.5:200 to 2.5:800, oxybutynin and gabapentin respectively. Generally, the ratio of oxybutynin to gabapentin in the combination is about 2.5:50, about 2.5:100, about 2.5:150, about 2.5:200, about 2.5:250, about 2.5:300, about 2.5:350, about 2.5:400, about 2.5:450, about 2.5:500, about 2.5:550, about 2.5:600, about 2.5:650, about 2.5:700, about 2.5:750, or about 2.5:800. Alternately, the ratio of oxybutynin to gabapentin in the combination is about about 1.25:50, about 1.25:100, about 1.25:150, about 1.25:200, about 1.25:250, about 1.25:300, about 1.25:350, about 1.25:400, about 1.25:450, about 1.25:500, about 1.25:550, about 1.25:600, about 1.25:650, about 1.25:700, about 1.25:750, or about 1.25:800. Alternately, the ratio of oxybutynin to gabapentin in the combination is about about 5:50, about 5:100, about 5:150, about 5:200, about 5:250, about 5:300, about 5:350, about 5:400, about 5:450, about 5:500, about 5:550, about 5:600, about 5:650, about 5:700, about 5:750, or about 5:800. Examples of formulations for preparing tablets comprising gabapentin and oxybutynin in combination suitable for use in the present invention are provided below in Tables 1 and 2.

| **Table 1** | |
|---|---|
| **Ingredient** | **Weight per Unit** |
| Gabapentin | 200.0 |
| Oxybutynin chloride | 2.50 |
| Lactose, monohydrate | 85.50 |
| Purified water | 130.0 |
| Providone | 24.00 |
| Microcrystalline cellulose | 80.00 |
| Crospovidone | 4.00 |
| Magnesium stearate | 4.00 |
| **Total** | **400.0** |

| **Table 2** | |
|---|---|
| **Ingredient** | **Weight per Unit** |
| Gabapentin | 200.0 |
| Oxybutynin chloride | 2.50 |
| Lactose, monohydrate | 89.50 |
| Purified water | 235.0⁵ |
| Hydroxypropylmethylcellulose | 20.00 |
| Microcrystalline cellulose | 80.00 |
| Crospovidone | 4.00 |
| Magnesium stearate | 4.00 |
| **Total** | **400.0** |

Tablets according to the above formulations can be prepared according to a number of possible methods. One method used in preparing a tablet comprising a formulation as provided above includes the following steps:
(1) sift ingredients through 20-mesh screen, transfer to granulator with impeller and chopper, and mix for five minutes;
(2) wet granulate mixed ingredients with a binder solution (such as povidone or methocel);
(3) transfer wet granules to fluid bed dryer and dry until %LOD values are within a 1-2.5% range;
(4) mill dried granules;
(5) lubricate milled granules (such as with magnesium stearate) in blender;
(6) compress into tablets.

Other derivatives and analogs of the active agents may be prepared using standard techniques known to those skilled in the art of synthetic organic chemistry, or may be deduced by reference to the pertinent literature. In addition, chiral active agents may be in isomerically pure form, or they may be administered as a racemic mixture of isomers.

### Pharmaceutical Compositions and Dosage Forms

Suitable compositions and dosage forms include tablets, capsules, caplets, pills, gel caps, troches, dispersions, suspensions, solutions, syrups, transdermal patches, gels, powders, magmas, lozenges, creams, pastes, plasters, lotions, discs, suppositories, liquid sprays for nasal or oral administration, dry powder or aerosolized formulations for inhalation, compositions and formulations for intravesical administration and the like. Further, those of ordinary skill in the art can readily deduce that suitable formulations involving these compositions and dosage forms, including those formulations as described elsewhere herein.

### Oral Dosage Forms

Oral dosage forms include tablets, capsules, caplets, solutions, suspensions and/or syrups, and may also comprise a plurality of granules, beads, powders or pellets that may or may not be encapsulated. Such dosage forms are prepared using conventional methods known to those in the field of pharmaceutical formulation and described in the pertinent texts, e.g., in Remington: The Science and Practice of Pharmacy, supra). Tablets and capsules represent the most convenient oral dosage forms, in which case solid pharmaceutical carriers are employed.

Tablets may be manufactured using standard tablet processing procedures and equipment. One method for forming tablets is by direct compression of a powdered, crystalline or granular composition containing the active agent(s), alone or in combination with one or more carriers, additives, or the like. As an alternative to direct compression, tablets can be prepared using wet-granulation or dry-granulation processes. Tablets may also be molded rather than compressed, starting with a moist or otherwise tractable material; however, compression and granulation techniques are preferred.

In addition to the active agent(s), then, tablets prepared for oral administration using the method of the invention will generally contain other materials such as binders, diluents, lubricants, disintegrants, fillers, stabilizers, surfactants, preservatives, coloring agents, flavoring agents and the like. Binders are used to impart cohesive qualities to a tablet, and thus ensure that the tablet remains intact after compression. Suitable binder materials include, but are not limited to, starch (including corn starch and pregelatinized starch), gelatin, sugars (including sucrose, glucose, dextrose and lactose), polyethylene glycol, propylene glycol, waxes, and natural and synthetic gums, e.g., acacia sodium alginate, polyvinylpyrrolidone, cellulosic polymers (including hydroxypropyl cellulose, hydroxypropyl methylcellulose, methyl cellulose, ethyl cellulose, hydroxyethyl cellulose, and the like), and Veegum. Diluents are typically necessary to increase bulk so that a practical size tablet is ultimately provided. Suitable diluents include dicalcium phosphate, calcium sulfate, lactose, cellulose, kaolin, mannitol, sodium chloride, dry starch and powdered sugar. Lubricants are used to facilitate tablet manufacture; examples of suitable lubricants include, for example, vegetable oils such as peanut oil, cottonseed oil, sesame oil, olive oil, corn oil, and oil of theobroma, glycerin, magnesium stearate, calcium stearate, and stearic acid. Stearates, if present, preferably represent at no more than approximately 2 wt. % of the drug-containing core. Disintegrants are used to facilitate disintegration of the tablet, and are generally starches, clays, celluloses, algins, gums or crosslinked polymers. Fillers include, for example, materials such as silicon dioxide, titanium dioxide, alumina, talc, kaolin, powdered cellulose and microcrystalline cellulose, as well as soluble materials such as mannitol, urea, sucrose, lactose, dextrose, sodium chloride and sorbitol. Stabilizers are used to inhibit or retard drug decomposition reactions that include, by way of example, oxidative reactions. Surfactants may be anionic, cationic, amphoteric or nonionic surface active agents.

The dosage form may also be a capsule, in which case the active agent-containing composition may be encapsulated in the form of a liquid or solid (including particulates such as granules, beads, powders or pellets). Suitable capsules may be either hard or soft, and are generally made of gelatin, starch, or a cellulosic material, with gelatin capsules preferred. Two-piece hard gelatin capsules are preferably sealed, such as with gelatin bands or the like. (See, for e.g., Remington: The Science and Practice of Pharmacy, supra), which describes materials and methods for preparing encapsulated pharmaceuticals. If the active agent-containing composition is present within the capsule in liquid form, a liquid carrier is necessary to dissolve the active agent(s). The carrier must be compatible with the capsule material and all components of the pharmaceutical composition, and must be suitable for ingestion.

Solid dosage forms, whether tablets, capsules, caplets, or particulates, may, if desired, be coated so as to provide for delayed release. Dosage forms with delayed release coatings may be manufactured using standard coating procedures and equipment. Such procedures are known to those skilled in the art and described in the pertinent texts (See, for e.g., Remington: The Science and Practice of Pharmacy, supra). Generally, after preparation of the solid dosage form, a delayed release coating composition is applied using a coating pan, an airless spray technique, fluidized bed coating equipment, or the like. Delayed release coating compositions comprise a polymeric material, e.g., cellulose butyrate phthalate, cellulose hydrogen phthalate, cellulose proprionate phthalate, polyvinyl acetate phthalate, cellulose acetate phthalate, cellulose acetate trimellitate, hydroxypropyl methylcellulose phthalate, hydroxypropyl methylcellulose acetate, dioxypropyl methylcellulose succinate, carboxymethyl ethylcellulose, hydroxypropyl methylcellulose acetate succinate, polymers and copolymers formed from acrylic acid, methacrylic acid, and/or esters thereof.

Sustained release dosage forms provide for drug release over an extended time period, and may or may not be delayed release. Generally, as will be appreciated by those of ordinary skill in the art, sustained release dosage forms are formulated by dispersing a drug within a matrix of a gradually bioerodible (hydrolyzable) material such as an insoluble plastic, a hydrophilic polymer, or a fatty compound, or by coating a solid, drug-containing dosage form with such a material. Insoluble plastic matrices may be comprised of, for example, polyvinyl chloride or polyethylene. Hydrophilic polymers useful for providing a sustained release coating or matrix cellulosic polymers include, without limitation: cellulosic polymers such as hydroxypropyl cellulose, hydroxyethyl cellulose, hydroxypropyl methyl cellulose, methyl cellulose, ethyl cellulose, cellulose acetate, cellulose acetate phthalate, cellulose acetate trimellitate, hydroxypropylmethyl cellulose phthalate, hydroxypropylcellulose phthalate, cellulose hexahydrophthalate, cellulose acetate hexahydrophthalate, and carboxymethylcellulose sodium; acrylic acid polymers and copolymers, preferably formed from acrylic acid, methacrylic acid, acrylic acid alkyl esters, methacrylic acid alkyl esters, and the like, e.g. copolymers of acrylic acid, methacrylic acid, methyl acrylate, ethyl acrylate, methyl methacrylate and/or ethyl methacrylate, with a terpolymer of ethyl acrylate, methyl methacrylate and trimethylammonioethyl methacrylate chloride (sold under the tradename Eudragit RS) preferred; vinyl polymers and copolymers such as polyvinyl pyrrolidone, polyvinyl acetate, polyvinylacetate phthalate, vinylacetate crotonic acid copolymer, and ethylene-vinyl acetate copolymers; zein; and shellac, ammoniated shellac, shellac-acetyl alcohol; and shellac n-butyl stearate. Fatty compounds for use as a sustained release matrix material include, but are not limited to, waxes generally (e.g., camauba wax) and glyceryl tristearate.

### Transmucosal Compositions and Dosage Forms

Although the present compositions may be administered orally, other modes of administration are suitable as well. For example, transmucosal administration may be advantageously employed. Transmucosal administration is carried out using any type of formulation or dosage unit suitable for application to mucosal tissue. For example, the selected active agent may be administered to the buccal mucosa in an adhesive tablet or patch, sublingually administered by placing a solid dosage form under the tongue, lingually administered by placing a solid dosage form on the tongue, administered nasally as droplets or a nasal spray, administered by inhalation of an aerosol formulation, a non-aerosol liquid formulation, or a dry powder, placed within or near the rectum ("transrectal" formulations), or administered to the urethra as a suppository, ointment, or the like.

Preferred buccal dosage forms will typically comprise a therapeutically effective amount of an active agent and a bioerodible (hydrolyzable) polymeric carrier that may also serve to adhere the dosage form to the buccal mucosa. The buccal dosage unit is fabricated so as to erode over a predetermined time period, wherein drug delivery is provided essentially throughout. The time period is typically in the range of from about 1 hour to about 72 hours. Preferred buccal delivery preferably occurs over a time period of from about 2 hours to about 24 hours. Buccal drug delivery for short term use should preferably occur over a time period of from about 2 hours to about 8 hours, more preferably over a time period of from about 3 hours to about 4 hours. As needed buccal drug delivery preferably will occur over a time period of from about 1 hour to about 12 hours, more preferably from about 2 hours to about 8 hours, most preferably from about 3 hours to about 6 hours. Sustained buccal drug delivery will preferably occur over a time period of from about 6 hours to about 72 hours, more preferably from about 12 hours to about 48 hours, most preferably from about 24 hours to about 48 hours. Buccal drug delivery, as will be appreciated by those skilled in the art, avoids the disadvantages encountered with oral drug administration, e.g., slow absorption, degradation of the active agent by fluids present in the gastrointestinal tract and/or first-pass inactivation in the liver.

The "therapeutically effective amount" of the active agent in the buccal dosage unit will of course depend on the potency of the agent and the intended dosage, which, in turn, is dependent on the particular individual undergoing treatment, the specific indication, and the like. The buccal dosage unit will generally contain from about 1.0 wt. % to about 60 wt. % active agent, preferably on the order of from about 1 wt. % to about 30 wt. % active agent. With regard to the bioerodible (hydrolyzable) polymeric carrier, it will be appreciated that virtually any such carrier can be used, so long as the desired drug release profile is not compromised, and the carrier is compatible with the active agents to be administered and any other components of the buccal dosage unit. Generally, the polymeric carrier comprises a hydrophilic (water-soluble and water-swellable) polymer that adheres to the wet surface of the buccal mucosa. Examples of polymeric carriers useful herein include acrylic acid polymers and co, e.g., those known as "carbomers" (Carbopol®, which may be obtained from B. F. Goodrich, is one such polymer). Other suitable polymers include, but are not limited to: hydrolyzed polyvinylalcohol; polyethylene oxides (e.g., Sentry Polyox® water soluble resins, available from Union Carbide); polyacrylates (e.g., Gantrez®, which may be obtained from GAF); vinyl polymers and copolymers; polyvinylpyrrolidone; dextran; guar gum; pectins; starches; and cellulosic polymers such as hydroxypropyl methylcellulose, (e.g., Methocel®, which may be obtained from the Dow Chemical Company), hydroxypropyl cellulose (e.g., Klucel®, which may also be obtained from Dow), hydroxypropyl cellulose ethers (see, e.g., U.S. Pat. No. 4,704,285 to Alderman), hydroxyethyl cellulose, carboxymethyl cellulose, sodium carboxymethyl cellulose, methyl cellulose, ethyl cellulose, cellulose acetate phthalate, cellulose acetate butyrate, and the like.

Other components may also be incorporated into the buccal dosage forms described herein. The additional components include, but are not limited to, disintegrants, diluents, binders, lubricants, flavoring, colorants, preservatives, and the like. Examples of disintegrants that may be used include, but are not limited to, cross-linked polyvinylpyrrolidones, such as crospovidone (e.g., Polyplasdone® XL, which may be obtained from GAF), cross-linked carboxylic methylcelluloses, such as croscarmelose (e.g., Ac-di-sol®, which may be obtained from FMC), alginic acid, and sodium carboxymethyl starches (e.g., Explotab®, which may be obtained from Edward Medell Co., Inc.), methylcellulose, agar bentonite and alginic acid. Suitable diluents are those which are generally useful in pharmaceutical formulations prepared using compression techniques, e.g., dicalcium phosphate dihydrate (e.g., Di-Tab®, which may be obtained from Stauffer), sugars that have been processed by cocrystallization with dextrin (e.g., co-crystallized sucrose and dextrin such as Di-Pak®, which may be obtained from Amstar), calcium phosphate, cellulose, kaolin, mannitol, sodium chloride, dry starch, powdered sugar and the like. Binders, if used, are those that enhance adhesion. Examples of such binders include, but are not limited to, starch, gelatin and sugars such as sucrose, dextrose, molasses, and lactose. Particularly preferred lubricants are stearates and stearic acid, and an optimal lubricant is magnesium stearate.

Sublingual and lingual dosage forms include tablets, creams, ointments, lozenges, pastes, and any other solid dosage form where the active ingredient is admixed into a disintegrable matrix. The tablet, cream, ointment or paste for sublingual or lingual delivery comprises a therapeutically effective amount of the selected active agent and one or more conventional nontoxic carriers suitable for sublingual or lingual drug administration. The sublingual and lingual dosage forms of the present invention can be manufactured using conventional processes. The sublingual and lingual dosage units are fabricated to disintegrate rapidly. The time period for complete disintegration of the dosage unit is typically in the range of from about 10 seconds to about 30 minutes, and optimally is less than 5 minutes.

Other components may also be incorporated into the sublingual and lingual dosage forms described herein. The additional components include, but are not limited to binders, disintegrants, wetting agents, lubricants, and the like. Examples of binders that may be used include water, ethanol, polyvinylpyrrolidone; starch solution gelatin solution, and the like. Suitable disintegrants include dry starch, calcium carbonate, polyoxyethylene sorbitan fatty acid esters, sodium lauryl sulfate, stearic monoglyceride, lactose, and the like. Wetting agents, if used, include glycerin, starches, and the like. Particularly preferred lubricants are stearates and polyethylene glycol. Additional components that may be incorporated into sublingual and lingual dosage forms are known, or will be apparent, to those skilled in this art (See, e.g., Remington: The Science and Practice of Pharmacy, supra).

For transurethral administration, the formulation comprises a urethral dosage form containing the active agent and one or more selected carriers or excipients, such as water, silicone, waxes, petroleum jelly, polyethylene glycol ("PEG"), propylene glycol ("PG"), liposomes, sugars such as mannitol and lactose, and/or a variety of other materials, with polyethylene glycol and derivatives thereof particularly preferred.

Depending on the particular active agent administered, it may be desirable to incorporate a transurethral permeation enhancer in the urethral dosage form. Examples of suitable transurethral permeation enhancers include dimethylsulfoxide ("DMSO"), dimethyl formamide ("DMF"), N, N-dimethylacetamide ("DMA"), decylmethylsulfoxide ("C₁₀ MSO"), polyethylene glycol monolaurate ("PEGML"), glycerol monolaurate, lecithin, the 1-substituted azacycloheptan-2-ones, particularly 1-n-dodecylcyclazacycloheptan-2-one (available under the trademark Azone® from Nelson Research & Development Co., Irvine, Calif.), SEPA® (available from Macrochem Co., Lexington, Mass.), surfactants as discussed above, including, for example, Tergitol®, Nonoxyliol-9® and TWEEN-80®, and lower alkanols such as ethanol.

Transurethral drug administration, as explained in U.S. Pat. Nos. 5,242,391, 5,474,535, 5,686,093 and 5,773,020, can be carried out in a number of different ways using a variety of urethral dosage forms. For example, the drug can be introduced into the urethra from a flexible tube, squeeze bottle, pump or aerosol spray. The drug may also be contained in coatings, pellets or suppositories that are absorbed, melted or bioeroded in the urethra. In certain embodiments, the drug is included in a coating on the exterior surface of a penile insert. It is preferred, although not essential, that the drug be delivered from at least about 3 cm into the urethra, and preferably from at least about 7 cm into the urethra. Generally, delivery from at least about 3 cm to about 8 cm into the urethra will provide effective results in conjunction with the present method.

Urethral suppository formulations containing PEG or a PEG derivative may be conveniently formulated using conventional techniques, e.g., compression molding, heat molding or the like, as will be appreciated by those skilled in the art and as described in the pertinent literature and pharmaceutical texts. (See, e.g., Remington: The Science and Practice of Pharmacy, supra), which discloses typical methods of preparing pharmaceutical compositions in the form of urethral suppositories. The PEG or PEG derivative preferably has a molecular weight in the range of from about 200 to about 2,500 g/mol, more preferably in the range of from about 1,000 to about 2,000 g/mol. Suitable polyethylene glycol derivatives include polyethylene glycol fatty acid esters, for example, polyethylene glycol monostearate, polyethylene glycol sorbitan esters, e.g., polysorbates, and the like. Depending on the particular active agent, it may also be preferred that urethral suppositories contain one or more solubilizing agents effective to increase the solubility of the active agent in the PEG or other transurethral vehicle.

It may be desirable to deliver the active agent in a urethral dosage form that provides for controlled or sustained release of the agent. In such a case, the dosage form comprises a biocompatible, biodegradable material, typically a biodegradable polymer. Examples of such polymers include polyesters, polyalkylcyanoacrylates, polyorthoesters, polyanhydrides, albumin, gelatin and starch. As explained, for example, in PCT Publication No. WO 96/40054, these and other polymers can be used to provide biodegradable microparticles that enable controlled and sustained drug release, in turn minimizing the required dosing frequency.

The urethral dosage form will preferably comprise a suppository that is on the order of from about 2 to about 20 mm in length, preferably from about 5 to about 10 mm in length, and less than about 5 mm in width, preferably less than about 2 mm in width. The weight of the suppository will typically be in the range of from about 1 mg to about 100 mg, preferably in the range of from about 1 mg to about 50 mg. However, it will be appreciated by those skilled in the art that the size of the suppository can and will vary, depending on the potency of the drug, the nature of the formulation, and other factors.

Transurethral drug delivery may involve an "active" delivery mechanism such as iontophoresis, electroporation or phonophoresis. Devices and methods for delivering drugs in this way are well known in the art. Iontophoretically assisted drug delivery is, for example, described in PCT Publication No. WO 96/40054, cited above. Briefly, the active agent is driven through the urethral wall by means of an electric current passed from an external electrode to a second electrode contained within or affixed to a urethral probe.

Preferred transrectal dosage forms include rectal suppositories, creams, ointments, and liquid formulations (enemas). The suppository, cream, ointment or liquid formulation for transrectal delivery comprises a therapeutically effective amount of the selected phosphodiesterase inhibitor and one or more conventional nontoxic carriers suitable for transrectal drug administration. The transrectal dosage forms of the present invention can be manufactured using conventional processes. The transrectal dosage unit can be fabricated to disintegrate rapidly or over a period of several hours. The time period for complete disintegration is preferably in the range of from about 10 minutes to about 6 hours, and optimally is less than about 3 hours.

Other components may also be incorporated into the transrectal dosage forms described herein. The additional components include, but are not limited to, stiffening agents, antioxidants, preservatives, and the like. Examples of stiffening agents that may be used include, for example, paraffin, white wax and yellow wax. Preferred antioxidants, if used, include sodium bisulfite and sodium metabisulfite.

Preferred vaginal or perivaginal dosage forms include vaginal suppositories, creams, ointments, liquid formulations, pessaries, tampons, gels, pastes, foams or sprays. The suppository, cream, ointment, liquid formulation, pessary, tampon, gel, paste, foam or spray for vaginal or perivaginal delivery comprises a therapeutically effective amount of the selected active agent and one or more conventional nontoxic carriers suitable for vaginal or perivaginal drug administration. The vaginal or perivaginal forms of the present invention can be manufactured using conventional processes as disclosed in Remington: The Science and Practice of Pharmacy, supra (see also drug formulations as adapted in U.S. Patent Nos. 6,515,198; 6,500,822; 6,417,186; 6,416,779; 6,376,500; 6,355,641; 6,258,819; 6,172,062; and 6,086,909). The vaginal or perivaginal dosage unit can be fabricated to disintegrate rapidly or over a period of several hours. The time period for complete disintegration is preferably in the range of from about 10 minutes to about 6 hours, and optimally is less than about 3 hours.

Other components may also be incorporated into the vaginal or perivaginal dosage forms described herein. The additional components include, but are not limited to, stiffening agents, antioxidants, preservatives, and the like. Examples of stiffening agents that may be used include, for example, paraffin, white wax and yellow wax. Preferred antioxidants, if used, include sodium bisulfite and sodium metabisulfite.

The active agents may also be administered intranasally or by inhalation. Compositions for intranasal administration are generally liquid formulations for administration as a spray or in the form of drops, although powder formulations for intranasal administration, e.g., insufflations, are also known, as are nasal gels, creams, pastes or ointments. For liquid formulations, the active agent can be formulated into a solution, e.g., water or isotonic saline, buffered or unbuffered, or as a suspension. Preferably, such solutions or suspensions are isotonic relative to nasal secretions and of about the same pH, ranging e.g., from about pH 4.0 to about pH 7.4 or, from about pH 6.0 to about pH 7.0. Buffers should be physiologically compatible and include, simply by way of example, phosphate buffers. Furthermore, various devices are available in the art for the generation of drops, droplets and sprays, including droppers, squeeze bottles, and manually and electrically powered intranasal pump dispensers. Active agent containing intranasal carriers may also include nasal gels, creams, pastes or ointments with a viscosity of, e.g., from about 10 to about 6500 cps, or greater, depending on the desired sustained contact with the nasal mucosal surfaces. Such carrier viscous formulations may be based upon, simply by way of example, alkylcelluloses and/or other biocompatible carriers of high viscosity well known to the art (see e.g., Remington: The Science and Practice of Pharmacy, supra). Other ingredients, such as art known preservatives, colorants, lubricating or viscous mineral or vegetable oils, perfumes, natural or synthetic plant extracts such as aromatic oils, and humectants and viscosity enhancers such as, e.g., glycerol, can also be included to provide additional viscosity, moisture retention and a pleasant texture and odor for the formulation. Formulations for inhalation may be prepared as an aerosol, either a solution aerosol in which the active agent is solubilized in a carrier (e.g., propellant) or a dispersion aerosol in which the active agent is suspended or dispersed throughout a carrier and an optional solvent. Non-aerosol formulations for inhalation may take the form of a liquid, typically an aqueous suspension, although aqueous solutions may be used as well. In such a case, the carrier is typically a sodium chloride solution having a concentration such that the formulation is isotonic relative to normal body fluid. In addition to the carrier, the liquid formulations may contain water and/or excipients including an antimicrobial preservative (e.g., benzalkonium chloride, benzethonium chloride, chlorobutanol, phenylethyl alcohol, thimerosal and combinations thereof), a buffering agent (e.g., citric acid, potassium metaphosphate, potassium phosphate, sodium acetate, sodium citrate, and combinations thereof), a surfactant (e.g., polysorbate 80, sodium lauryl sulfate, sorbitan monopalmitate and combinations thereof), and/or a suspending agent (e.g., agar, bentonite, microcrystalline cellulose, sodium carboxymethylcellulose, hydroxypropyl methylcellulose, tragacanth, veegum and combinations thereof). Non-aerosol formulations for inhalation may also comprise dry powder formulations, particularly insufflations in which the powder has an average particle size of from about 0.1 *µ*m to about 50 *µ*m, preferably from about 1 *µ*m to about 25 *µ*m.

### Topical Formulations

Topical formulations may be in any form suitable for application to the body surface, and may comprise, for example, an ointment, cream, gel, lotion, solution, paste or the like, and/or may be prepared so as to contain liposomes, micelles, and/or microspheres. Preferred topical formulations herein are ointments, creams and gels.

Ointments, as is well known in the art of pharmaceutical formulation, are semisolid preparations that are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington: The Science and Practice of Pharmacy, supra, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Preferred water-soluble ointment bases are prepared from polyethylene glycols of varying molecular weight (See, e.g., Remington: The Science and Practice of Pharmacy, supra).

Creams, as also well known in the art, are viscous liquids or semisolid emulsions, either oil-in-water or water-in-oil. Cream bases are water-washable, and contain an oil phase, an emulsifier and an aqueous phase. The oil phase, also called the "internal" phase, is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic, cationic or amphoteric surfactant.

As will be appreciated by those working in the field of pharmaceutical formulation, gels-are semisolid, suspension-type systems. Single-phase gels contain organic macromolecules distributed substantially uniformly throughout the carrier liquid, which is typically aqueous, but also, preferably, contain an alcohol and, optionally, an oil. Preferred "organic macromolecules," i.e., gelling agents, are crosslinked acrylic acid polymers such as the "carbomer" family of polymers, e.g., carboxypolyalkylenes that may be obtained commercially under the Carbopol® trademark. Also preferred are hydrophilic polymers such as polyethylene oxides, polyoxyethylene-polyoxypropylene copolymers and polyvinylalcohol; cellulosic polymers such as hydroxypropyl cellulose, hydroxyethyl cellulose, hydroxypropyl methylcellulose, hydroxypropyl methylcellulose phthalate, and methylcellulose; gums such as tragacanth and xanthan gum; sodium alginate; and gelatin. In order to prepare a uniform gel, dispersing agents such as alcohol or glycerin can be added, or the gelling agent can be dispersed by trituration, mechanical mixing, and/or stirring.

Various additives, known to those skilled in the art, may be included in the topical formulations. For example, solubilizers may be used to solubilize certain active agents. For those drugs having an unusually low rate of permeation through the skin or mucosal tissue, it may be desirable to include a permeation enhancer in the formulation; suitable enhancers are as described elsewhere herein.

### Transdermal Administration

The compounds of the invention may also be administered through the skin or mucosal tissue using conventional transdermal drug delivery systems, wherein the agent is contained within a laminated structure (typically referred to as a transdermal "patch") that serves as a drug delivery device to be affixed to the skin. Transdermal drug delivery may involve passive diffusion or it may be facilitated using electrotransport, e.g., iontophoresis. In a typical transdermal "patch," the drug composition is contained in a layer, or "reservoir," underlying an upper backing layer. The laminated structure may contain a single reservoir, or it may contain multiple reservoirs. In one type of patch, referred to as a "monolithic" system, the reservoir is comprised of a polymeric matrix of a pharmaceutically acceptable contact adhesive material that serves to affix the system to the skin during drug delivery. Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are separate and distinct layers, with the adhesive underlying the reservoir which, in this case, may be either a polymeric matrix as described above, or it may be a liquid or hydrogel reservoir, or may take some other form.

The backing layer in these laminates, which serves as the upper surface of the device, functions as the primary structural element of the laminated structure and provides the device with much of its flexibility. The material selected for the backing material should be selected so that it is substantially impermeable to the active agent and any other materials that are present, the backing is preferably made of a sheet or film of a flexible elastomeric material. Examples of polymers that are suitable for the backing layer include polyethylene, polypropylene, polyesters, and the like.

During storage and prior to use, the laminated structure includes a release liner. Immediately prior to use, this layer is removed from the device to expose the basal surface thereof, either the drug reservoir or a separate contact adhesive layer, so that the system may be affixed to the skin. The release liner should be made from a drug/vehicle impermeable material.

Transdermal drug delivery systems may in addition contain a skin permeation enhancer. That is, because the inherent permeability of the skin to some drugs may be too low to allow therapeutic levels of the drug to pass through a reasonably sized area of unbroken skin, it is necessary to coadminister a skin permeation enhancer with such drugs. Suitable enhancers are well known in the art and include, for example, those enhancers listed above in transmucosal compositions.

### Parenteral Administration

Parenteral administration, if used, is generally characterized by injection, including intramuscular, intraperitoneal, intravenous (IV) and subcutaneous injection. Injectable formulations can be prepared in conventional forms, either as liquid solutions or suspensions; solid forms suitable for solution or suspension in liquid prior to injection, or as emulsions. Preferably, sterile injectable suspensions are formulated according to techniques known in the art using suitable dispersing or wetting agents and suspending agents. The sterile injectable formulation may also be a sterile injectable solution or a suspension in a nontoxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. A more recently revised approach for parenteral administration involves use of a slow release or sustained release system (See, e.g., U.S. Pat. No. 3,710,795).

### Intravesical Administration

Intravesical administration, if used, is generally characterized by administration directly into the bladder and may include methods as described elsewhere herein. Other methods of intravesical administration may include those described in U.S. Patent Nos. 6,207,180 and 6,039,967, as well as other methods that are known to one of skill in the art.

### Intrathecal Administration

Intrathecal administration, if used, is generally characterized by administration directly into the intrathecal space (where fluid flows around the spinal cord).

One common system utilized for intrathecal administration is the APT Intrathecal treatment system available from Medtronic, Inc. APT Intrathecal uses a small pump that is surgically placed under the skin of the abdomen to deliver medication directly into the intrathecal space. The medication is delivered through a small tube called a catheter that is also surgically placed. The medication can then be administered directly to cells in the spinal cord involved in conveying sensory and motor signals associated with lower urinary tract disorders.

Another system available from Medtronic that is commonly utilized for intrathecal administration is the is the fully implantable, programmable SynchroMed^{®} Infusion System. The SynchroMed^{®} Infusion System has two parts that are both placed in the body during a surgical procedure: the catheter and the pump. The catheter is a small, soft tube. One end is connected to the catheter port of the pump, and the other end is placed in the intrathecal space. The pump is a round metal device about one inch (2.5 cm) thick, three inches (8.5 cm) in diameter, and weighs about six ounces (205 g) that stores and releases prescribed amounts of medication directly into the intrathecal space. It is made of titanium, a lightweight, medical-grade metal. The reservoir is the space inside the pump that holds the medication. The fill port is a raised center portion of the pump through which the pump is refilled. The doctor or a nurse inserts a needle through the patient's skin and through the fill port to fill the pump. Some pumps have a side catheter access port that allows the doctor to inject other medications or sterile solutions directly into the catheter, bypassing the pump.

The SynchroMed^{®} pump automatically delivers a controlled amount of medication through the catheter to the intrathecal space around the spinal cord, where it is most effective. The exact dosage, rate and timing prescribed by the doctor are entered in the pump using a programmer, an external computer-like device that controls the pump's memory. Information about the patient's prescription is stored in the pump's memory. The doctor can easily review this information by using the programmer. The programmer communicates with the pump by radio signals that allow the doctor to tell how the pump is operating at any given time. The doctor also can use the programmer to change your medication dosage.

Methods of intrathecal administration may include those described above available from Medtronic, as well as other methods that are known to one of skill in the art.

### Additional Dosage Formulations and Drug Delivery Systems

As compared with traditional drug delivery approaches, some controlled release technologies rely upon the modification of both macromolecules and synthetic small molecules to allow them to be actively instead of passively absorbed into the body. For example, XenoPort Inc. utilizes technology that takes existing molecules and re-engineers them to create new chemical entities (unique molecules) that have improved pharmacologic properties to either: 1) lengthen the short half-life of a drug; 2) overcome poor absorption; and/or 3) deal with poor drug distribution to target tissues. Techniques to lengthen the short half-life of a drug include the use of prodrugs with slow cleavage rates to release drugs over time or that engage transporters in small and large intestines to allow the use of oral sustained delivery systems, as well as drugs that engage active transport systems. Examples of such controlled release formulations, tablets, dosage forms, and drug delivery systems, and that are suitable for use with the present invention, are described in the following published US and PCT patent applications assigned to Xenoport Inc.: US20030158254; US20030158089; US20030017964; US2003130246; WO02100172; WO02100392; WO02100347; WO02100344; WO0242414; WO0228881; WO0228882; WO0244324; WO0232376; WO0228883; and W00228411. In particular, Xenoport's XP13512 is a transported Prodrug of gabapentin that has been engineered to utilize high capacity transport mechanisms located in both the small and large intestine and to rapidly convert to gabapentin once in the body. In contrast to gabapentin itself, XP13512 was shown in preclinical and clinical studies to produce dose proportional blood levels of gabapentin across a broad range of oral doses, and to be absorbed efficiently from the large intestine.

Some other controlled release technologies rely upon methods that promote or enhance gastric retention, such as those developed by Depomed Inc. Because many drugs are best absorbed in the stomach and upper portions of the small intestine, Depomed has developed tablets that swell in the stomach during the postprandial or fed mode so that they are treated like undigested food. These tablets therefore sit safely and neutrally in the stomach for 6, 8, or more hours and deliver drug at a desired rate and time to upper gastrointestinal sites. Specific technologies in this area include: 1) tablets that slowly erode in gastric fluids to deliver drugs at almost a constant rate (particularly useful for highly insoluble drugs); 2) bi-layer tablets that combine drugs with different characteristics into a single table (such as a highly insoluble drug in an erosion layer and a soluble drug in a diffusion layer for sustained release of both); and 3) combination tablets that can either deliver drugs simultaneously or in sequence over a desired period of time (including an initial burst of a fast acting drug followed by slow and sustained delivery of another drug). Examples of such controlled release formulations that are suitable for use with the present invention and that rely upon gastric retention during the postprandial or fed mode, include tablets, dosage forms, and drug delivery systems in the following US patents assigned to Depomed Inc.: US 6,488,962; US 6,451,808; US 6,340,475; US 5,972,389; US 5,582,837; and US 5,007,790. Examples of such controlled release formulations that are suitable for use with the present invention and that rely upon gastric retention during the postprandial or fed mode, include tablets, dosage forms, and drug delivery systems in the following published US and PCT patent applications assigned to Depomed Inc.: US20030147952; US20030104062; US20030104053; US20030104052; US20030091630; US20030044466; US20030039688; US20020051820; WO0335040; WO0335039; WO0156544; WO0132217 WO9855107; WO9747285; and WO9318755.

Other controlled release systems include those developed by ALZA Corporation based upon: 1) osmotic technology for oral delivery; 2) transdermal delivery via patches; 3) liposomal delivery via intravenous injection; 4) osmotic technology for long-term delivery via implants; and 5) depot technology designed to deliver agents for periods of days to a month. ALZA oral delivery systems include those that employ osmosis to provide precise, controlled drug delivery for up to 24 hours for both poorly soluble and highly soluble drugs, as well as those that deliver high drug doses meeting high drug loading requirements. ALZA controlled transdermal delivery systems provide drug delivery through intact skin for as long as one week with a single application to improve drug absorption and deliver constant amounts of drug into the bloodstream over time. ALZA liposomal delivery systems involve lipid nanoparticles that evade recognition by the immune system because of their unique polyethylene glycol (PEG) coating, allowing the precise delivery of drugs to disease-specific areas of the body. ALZA also has developed osmotically driven systems to enable the continuous delivery of small drugs, peptides, proteins, DNA and other bioactive macromolecules for up to one year for systemic or tissue-specific therapy. Finally, ALZA depot injection therapy is designed to deliver biopharmaceutical agents and small molecules for periods of days to a month using a nonaqueous polymer solution for the stabilization of macromolecules and a unique delivery profile.

Examples of controlled release formulations, tablets, dosage forms, and drug delivery systems that are suitable for use with the present invention are described in the following US patents assigned to ALZA Corporation: US 4,367,741; US 4,402,695; US 4,418,038; US 4,434,153; US 4,439,199; US 4,450,198; US 4,455,142; US 4,455,144; US 4,484,923; US 4,486,193; US 4,489,197; US 4,511,353; US 4,519,801; US 4,526,578; US 4,526,933; US 4,534,757; US 4,553,973; US 4,559,222; US 4,564,364; US 4,578,075; US 4,588,580; US 4,610,686; US 4,612,008; US 4,615,487; US 4,627,851; US 4,629,449; US 4,642,233; US 4,649,043; US 4,650,484; US 4,659,558; US 4,661,105; US 4,662,880; US 4,675,174; US 4,681,583; US 4,684,524; US 4,692,336; US 4,693,895; US 4,704,119; US 4,705,515; US 4,717,566; US 4,721,613; US 4,723,957; US 4,725,272; US 4,728,498; US 4,743,248; US 4,747,847; US 4,751,071; US 4,753,802; US 4,755,180; US 4,756,314; US 4,764,380; US 4,773,907; US 4,777,049; US 4,781,924; US 4,783,337; US 4,786,503; US 4,788,062; US 4,810,502; US 4,512,313; US 4,816,258; US 4,824,675; US 4,834,979; US 4,837,027; US 4,842,867; US 4,846,826; US 4,847,093; US 4,849,226; US 4,851,229; US 4,851,231; US 4,851,232; US 4,853,229; US 4,857,330; US 4,859,470; US 4,863,456; US 4,863,744; US 4,865,598; US 4,867,969; US 4,871,548; US 4,872,873; US 4,874,388; US 4,876,093; US 4,892,778; US 4,902,514; US 4,904,474; US 4,913,903; US 4,915,949; US 4,915,952; US 4,917,895; US 4,931,285; US 4,946,685; US 4,948,592; US 4,954,344; US 4,957,494; US 4,960,416; US 4,961,931; US 4,961,932; US 4,963,141; US 4,966,769; US 4,971,790; US 4,976,966; US 4,986,987; US 5,006,346; US 5,017,381; US 5,019,397; US 5,023,076; US 5,023,088; US 5,024,842; US 5,028,434; US 5,030,454; US 5,071,656; US 5,077,054; US 5,082,668; US 5,104,390; US 5,110,597; US 5,122,128; US 5,125,894; US 5,141,750; US 5,141,752; US 5,156,850; US 5,160,743; US 5,160,744; US 5,169,382; US 5,171,576; US 5,176,665; US 5,185,158; US 5,190,765; US 5,198,223; US 5,198,229; US 5,200,195; US 5,200,196; US 5,204,116; US 5,208,037; US 5,209,746; US 5,221,254; US 5,221,278; US 5,229,133; US 5,232,438; US 5,232,705; US 5,236,689; US 5,236,714; US 5,240,713; US 5,246,710; US 5,246,711; US 5,252,338; US 5,254,349; US 5,266,332; US 5,273,752; US 5,284,660; US 5,286,491; US 5,308,348; US 5,318,558; US 5,320,850; US 5,322,502; US 5,326,571; US 5,330,762; US 5,338,550; US 5,340,590; US 5,342,623; US 5,344,656; US 5,348,746; US 5,358,721; US 5,364,630; US 5,376,377; US 5,391,381; US 5,402,777; US 5,403,275; US 5,411,740; US 5,417,675; US 5,417,676; US 5,417,682; US 5,423,739; US 5,424,289; US 5,431,919; US 5,443,442; US 5,443,459; US 5,443,461; US 5,456,679; US 5,460,826; US 5,462,741; US 5,462,745; US 5,489,281; US 5,499,979; US 5,500,222; US 5,512,293; US 5,512,299; US 5,529,787; US 5,531,736; US 5,532,003; US 5,533,971; US 5,534,263; US 5,540,912; US 5,543,156; US 5,571,525; US 5,573,503; US 5,591,124; US 5,593,695; US 5,595,759; US 5,603,954; US 5,607,696; US 5,609,885; US 5,614,211; US 5,614,578; US 5,620,705; US 5,620,708; US 5,622,530; US 5,622,944; US 5,633,011; US 5,639,477; US 5,660,861; US 5,667,804; US 5,667,805; US 5,674,895; US 5,688,518; US 5,698,224; US 5,702,725; US 5,702,727; US 5,707,663; US 5,713,852; US 5,718,700; US 5,736,580; US 5,770,227; US 5,780,058; US 5,783,213; US 5,785,994; US 5,795,591; US 5,811,465; US 5,817,624; US 5,824,340; US 5,830,501; US 5,830,502; US 5,840,754; US 5,858,407; US 5,861,439; US 5,863,558; US 5,876,750; US 5,883,135; US 5,840,754; US 5,897,878; US 5,904,934; US 5,904,935; US 5,906,832; US 5,912,268; US 5,914,131; US 5,916,582; US 5,932,547; US 5,938,654; US 5,941,844; US 5,955,103; US 5,972,369; US 5,972,370; US 5,972,379; US 5,980,943; US 5,981,489; US 5,983,130; US 5,989,590; US 5,995,869; US 5,997,902; US 6,001,390; US 6,004,309; US 6,004,578; US 6,008,187; US 6,020,000; US 6,034,101; US 6,036,973; US 6,039,977; US 6,057,374; US 6,066,619; US 6,068,850; US 6,077,538; US 6,083,190; US 6,096,339; US 6,106,845; US 6,110,499; US 6,120,798; US 6,120,803; US 6,124,261; US 6,124,355; US 6,130,200; US 6,146,662; US 6,153,678; US 6,174,547; US 6,183,466; US 6,203,817; US 6,210,712; US 6,210,713; US 6,224,907; US 6,235,712; US 6,245,357; US 6,262,115; US 6,264,990; US 6,267,984; US 6,287,598; US 6,289,241; US 6,331,311; US 6,333,050; US 6,342,249; US 6,346,270; US 6365183; US 6,368,626; US 6,387,403; US 6,419,952; US 6,440,457; US 6,468,961; US 6,491,683; US 6,512,010; US 6,514,530; US 6534089; US 6,544,252; US 6,548,083; US 6,551,613; US 6,572,879; and US 6,596,314.

Other examples of controlled release formulations, tablets, dosage forms, and drug delivery systems that are suitable for use with the present invention are described in the following published US patent application and PCT applications assigned to ALZA Corporation: US20010051183; WO0004886; WO0013663; WO0013674; WO0025753; WO0025790; WO0035419; WO0038650; WO0040218; WO0045790; WO006612; WO0074650; WO01193 WO0119352; WO0121211; W00137815; WO0141742; WO0143721; WO0156543; WO3041684; WO03041685; WO03041757; WO03045352; WO03051341; WO03053400; WO03053401; WO9000416; WO9004965; WO9113613; W09116884; WO9204011; W09211843; WO9212692; WO9213521; WO9217239; W09218102; WO9300071; WO9305843; WO9306819; WO9314813; WO9319739; WO9320127; WO9320134; WO9407562; WO9408572; WO9416699; WO9421262; WO9427587; WO9427589; WO9503823; WO9519174; WO9529665; WO9600065; WO9613248; WO9625922; WO9637202; WO9640049; WO9640050; WO9640139; WO9640364; WO9640365; WO9703634; WO9800158; WO9802169; WO9814168; WO9816250; WO9817315; WO9827962; WO9827963; WO9843611; WO9907342; WO9912526; WO9912527; WO9918159; WO9929297; WO9929348; WO9932096; WO9932153; WO9948494; WO9956730; WO9958115; and WO9962496.

Another drug delivery technology suitable for use in the present invention is that disclosed by DepoMed, Inc. in U.S. Patent No. 6,682,759, which discloses a method for manufacturing a pharmaceutical tablet for oral administration combining both immediate-release and prolonged-release modes of drug delivery. The tablet according to the method comprises a prolonged-release drug core and an immediate-release drug coating or layer, which can be insoluble or sparingly soluble in water. The method limits the drug particle diameter in the immediate-release coating or layer to 10 microns or less. The coating or layer is either the particles themselves, applied as an aqueous suspension, or a solid composition that contains the drug particles incorporated in a solid material that disintegrates rapidly in gastric fluid.

Andrx Corporation has also developed drug delivery technology suitable for use in the present invention that includes: 1) a pelletized pulsatile delivery system ("PPDS"); 2) a single composition osmotic tablet system ("SCOT"); 3) a solubility modulating hydrogel system ("SMHS"); 4) a delayed pulsatile hydrogel system ("DPHS"); 5) a stabilized pellet delivery system ("SPDS"); 6) a granulated modulating hydrogel system ("GMHS"); 7) a pelletized tablet system ("PELTAB"); 8) a porous tablet system ("PORTAB"); and 9) a stabilized tablet delivery system ("STDS"). PPDS uses pellets that are coated with specific polymers and agents to control the release rate of the microencapsulated drug and is designed for use with drugs that require a pulsed release. SCOT utilizes various osmotic modulating agents as well as polymer coatings to provide a zero-order drug release. SMHS utilizes a hydrogel-based dosage system that avoids the "initial burst effect" commonly observed with other sustained-release hydrogel formulations and that provides for sustained release without the need to use special coatings or structures that add to the cost of manufacturing. DPHS is designed for use with hydrogel matrix products characterized by an initial zero-order drug release followed by a rapid release that is achieved by the blending of selected hydrogel polymers to achieve a delayed pulse. SPDS incorporates a pellet core of drug and protective polymer outer layer, and is designed specifically for unstable drugs, while GMHS incorporates hydrogel and binding polymers with the drug and forms granules that are pressed into tablet form. PELTAB provides controlled release by using a water insoluble polymer to coat discrete drug crystals or pellets to enable them to resist the action of fluids in the gastrointestinal tract, and these coated pellets are then compressed into tablets. PORTAB provides controlled release by incorporating an osmotic core with a continuous polymer coating and a water soluble component that expands the core and creates microporous channels through which drug is released. Finally, STDS includes a dual layer coating technique that avoids the need to use a coating layer to separate the enteric coating layer from the omeprazole core.

Examples of controlled release formulations, tablets, dosage forms, and drug delivery systems that are suitable for use with the present invention are described in the following US patents assigned to Andrx Corporation: US 5,397,574; US 5,419,917; US 5,458,887; US 5,458,888; US 5,472,708; US 5,508,040; US 5,558,879; US 5,567,441; US 5,654,005; US 5,728,402; US 5,736,159; US 5,830,503; US 5,834,023; US 5,837,379; US 5,916,595; US 5,922,352; US 6,099,859; US 6,099,862; US 6,103,263; US 6,106,862; US 6,156,342; US 6,177,102; US 6,197,347; US 6,210,716; US 6,238,703; US 6,270,805; US 6,284,275; US 6,485,748; US 6,495,162; US 6,524,620; US 6,544,556; US 6,589,553; US 6,602,522; and US 6,610,326.

Examples of controlled release formulations, tablets, dosage forms, and drug delivery systems that are suitable for use with the present invention are described in the following published US and PCT patent applications assigned to Andrx Corporation: US20010024659; US20020115718; US20020156066; WO0004883; WO0009091; WO0012097; WO0027370; WO0050010; WO0132161; WO0134123; WO0236077; WO0236100; WO02062299; WO02062824; WO02065991; WO02069888; WO02074285; WO03000177; WO9521607; WO9629992; WO9633700; WO9640080; WO9748386; WO9833488; WO9833489; WO9930692; WO9947125; and WO9961005.

Some other examples of drug delivery approaches focus on non-oral drug delivery, providing parenteral, transmucosal, and topical delivery of proteins, peptides, and small molecules. For example, the Atrigel^{®} drug delivery system marketed by Atrix Laboratories Inc. comprises biodegradable polymers, similar to those used in biodegradable sutures, dissolved in biocompatible carriers. These pharmaceuticals may be blended into a liquid delivery system at the time of manufacturing or, depending upon the product, may be added later by a physician at the time of use. Injection of the liquid product subcutaneously or intramuscularly through a small gauge needle, or placement into accessible tissue sites through a cannula, causes displacement of the carrier with water in the tissue fluids, and a subsequent precipitate to form from the polymer into a solid film or implant. The drug encapsulated within the implant is then released in a controlled manner as the polymer matrix biodegrades over a period ranging from days to months. Examples of such drug delivery systems include Atrix's Eligard^{®}, Atridox^{®}/ Doxirobe^{®}, Atrisorb^{®} FreeFlow^{™}/ Atrisorb^{®}-D FreeFlow, bone growth products, and others as described in the following published US and PCT patent applications assigned to Atrix Laboratories Inc.: US RE37950; US 6,630,155; US 6,566,144; US 6,610,252; US 6,565,874; US 6,528,080; US 6,461,631; US 6,395,293; US 6,261,583; US 6,143,314; US 6,120,789; US 6,071,530; US 5,990,194; US 5,945,115; US 5,888,533; US 5,792,469; US 5,780,044; US 5,759,563; US 5,744,153; US 5,739,176; US 5,736,152; US 5,733,950; US 5,702,716; US 5,681,873; US 5,660,849; US 5,599,552; US 5,487,897; US 5,368,859; US 5,340,849; US 5,324,519; US 5,278,202; US 5,278,201; US20020114737, US20030195489; US20030133964;US 20010042317; US20020090398; US20020001608; and US2001042317.

Atrix Laboratories Inc. also markets technology for the non-oral transmucosal delivery of drugs over a time period from minutes to hours. For example, Atrix's BEMA^{™} (Bioerodible Muco-Adhesive Disc) drug delivery system comprises preformed bioerodible discs for local or systemic delivery. Examples of such drug delivery systems include those as described in US Patent No. 6,245,345.

Other drug delivery systems marketed by Atrix Laboratories Inc. focus on topical drug delivery. For example, SMP^{™} (Solvent Particle System) allows the topical delivery of highly water-insoluble drugs. This product allows for a controlled amount of a dissolved drug to permeate the epidermal layer of the skin by combining the dissolved drug with a microparticle suspension of the drug. The SMP^{™} system works in stages whereby: 1) the product is applied to the skin surface; 2) the product near follicles concentrates at the skin pore; 3) the drug readily partitions into skin oils; and 4) the drug diffuses throughout the area. By contrast, MCA^{®} (Mucocutaneous Absorption System) is a water-resistant topical gel providing sustained drug delivery. MCA® forms a tenacious film for either wet or dry surfaces where: 1) the product is applied to the skin or mucosal surface; 2) the product forms a tenacious moisture-resistant film; and 3) the adhered film provides sustained release of drug for a period from hours to days. Yet another product, BCP^{™} (Biocompatible Polymer System) provides a non-cytotoxic gel or liquid that is applied as a protective film for wound healing. Examples of these systems include Orajel^{®}-Ultra Mouth Sore Medicine as well as those as described in the following published US patents and applications assigned to Atrix Laboratories Inc.: US 6,537,565; US 6,432,415; US 6,355,657; US 5,962,006; US 5,725,491; US 5,722,950; US 5,717,030; US 5,707,647; US 5,632,727; and US20010033853.

Additional formulations and compositions available from Teva Pharmaceutical Industries Ltd., Warner Lambert & Co., and Godecke Aktiengesellshaft that include gabapentin and are useful in the present invention include those as described in the following US patents and published US and PCT patent applications: US 6,531,509; US 6,255,526; US 6,054,482; US2003055109; US2002045662; US2002009115; WO 01/97782; WO 01/97612; EP 2001946364; WO 99/59573; and WO 99/59572.

Additional formulations and compositions that include oxybutynin and are useful in the present invention include those as described in the following US patents and published US and PCT patent applications: US 5,834,010; US 5,601,839; and US 5,164,190.

### Dosage and Administration

The concentration of the active agent in any of the aforementioned dosage forms and compositions can vary a great deal, and will depend on a variety of factors, including the type of composition or dosage form, the corresponding mode of administration, the nature and activity of the specific active agent, and the intended drug release profile. Preferred dosage forms contain a unit dose of active agent, i.e., a single therapeutically effective dose. For creams, ointments, etc., a "unit dose" requires an active agent concentration that provides a unit dose in a specified quantity of the formulation to be applied. The unit dose of any particular active agent will depend, of course, on the active agent and on the mode of administration.

For the active agents of the present invention (including the α₂δ subunit calcium channel modulator gabapentin or pregabalin in combination with an antimuscarinic), the unit dose for oral, transmucosal, topical, transdermal, and parenteral administration will be in the range of from about 1 ng to about 10,000 mg, about 5 ng to about 9,500 mg, about 10 ng to about 9,000 mg, about 20 ng to about 8,500 mg, about 30 ng to about 7,500 mg, about 40 ng to about 7,000 mg, about 50 ng to about 6,500 mg, about 100 ng to about 6,000 mg, about 200 ng to about 5,500 mg, about 300 ng to about 5,000 mg, about 400 ng to about 4,500 mg, about 500 ng to about 4,000 mg, about 1 µg to about 3,500 mg, about 5 µg to about 3,000 mg, about 10 µg to about 2,600 mg, about 20 µg to about 2,575 mg, about 30 µg to about 2,550 mg, about 40 µg to about 2,500 mg, about 50 µg to about 2,475 mg, about 100 µg to about 2,450 mg, about 200 µg to about 2,425 mg, about 300 µg to about 2,000, about 400 µg to about 1,175 mg, about 500 µg to about 1,150 mg, about .5 mg to about 1,125 mg, about 1 mg to about 1,100 mg, about 1.25 mg to about 1,075 mg, about 1.5 mg to about 1,050 mg, about 2.0 mg to about 1,025 mg, about 2.5 mg to 1,000 mg, about 3.0 mg to about 975 mg, about 3.5 mg to about 950 mg, about 4.0 mg to about 925 mg, about 4.5 mg to about 900 mg, about 5 mg to about 875 mg, about 10 mg to about 850 mg, about 20 mg to about 825 mg, about 30 mg to about 800 mg, about 40 mg to about 775 mg, about 50 mg to about 750 mg, about 100 mg to about 725 mg, about 200 mg to about 700 mg, about 300 mg to about 675 mg, about 400 mg to about 650 mg, about 500 mg, or about 525 mg to about 625 mg.

Alternatively, for active agents of the present invention (including the α₂δ subunit calcium channel modulator gabapentin or pregabalin in combination with an antimuscarinic), the unit dose for oral, transmucosal, topical, transdermal, and parenteral administration will be equal to or greater than about 1 ng, about 5 ng, about 10 ng, about 20 ng, about 30 ng, about 40 ng, about 50 ng, about 100 ng, about 200 ng, about 300 ng, about 400 ng, about 500 ng, about 1 µg, about 5 µg, about 10 µg, about 20 µg, about 30 µg, about 40 µg, about 50 µg, about 100 µg, about 200 µg, about 300 µg, about 400 µg, about 500 µg, about .5 mg, about 1 mg, about 1.25 mg, about 1.5 mg, about 2.0 mg, about 2.5 mg, about 3.0 mg, about 3.5 mg, about 4.0 mg, about 4.5 mg, about 5 mg, about 10 mg, about 20 mg, about 30 mg, about 40 mg, about 50 mg, about 100 mg, about 200 mg, about 300 mg, about 400 mg, about 500 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125 mg, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg, about 2500 mg, about 2525 mg, about 2550 mg, about 2575 mg, about 2600 mg, about 3,000 mg, about 3,500 mg, about 4,000 mg, about 4,500 mg, about 5,000 mg, about 5,500 mg, about 6,000 mg, about 6,500 mg, about 7,000 mg, about 7,500 mg, about 8,000 mg, about 8,500 mg, about 9,000 mg, or about 9,500 mg.

For the active agents of the present invention (including the α₂δ subunit calcium channel modulator gabapentin or pregabalin in combination with an antimuscarinic), the unit dose for intrathecal administration will be in the range of from about 1 fg to about 1 mg, about 5 fg to about 500 µg, about 10 fg to about 400 µg, about 20 fg to about 300 µg, about 30 fg to about 200 µg, about 40 fg to about 100 µg, about 50 fg to about 50 µg, about 100 fg to about 40 µg, about 200 fg to about 30 µg, about 300 fg to about 20 µg, about 400 fg to about 10 µg, about 500 fg to about 5 µg, about 1 pg to about 1 µg, about 5 pg to about 500 ng, about 10 pg to about 400 ng, about 20 pg to about 300 ng, about 30 pg to about 200 ng, about 40 pg to about 100 ng, about 50 pg to about 50 ng, about 100 pg to about 40 ng, about 200 pg to about 30 ng, about 300 pg to about 20 ng, about 400 pg to about 10 ng, about 500 pg to about 5 ng,

Alternatively, for the active agents of the present invention (including the α₂δ subunit calcium channel modulator gabapentin or pregabalin in combination with an antimuscarinic), the unit dose for intrathecal administration will be equal to or greater than about 1 fg, about 5 fg, about 10 fg, about 20 fg, about 30 fg, about 40 fg, about 50 fg, about 100 fg, about 200 fg, about 300 fg, about 400 fg, about 500 fg, about 1 pg, about 5 pg, about 10 pg, about 20 pg, about 30 pg, about 40 pg, about 50 pg, about 100 pg, about 200 pg, about 300 pg, about 400 pg, about 500 pg, about 1 ng, about 5 ng, about 10 ng, about 20 ng, about 30 ng, about 40 ng, about 50 ng, about 100 ng, about 200 ng, about 300 ng, about 400 ng, about 500 ng, about 1 µg, about 5 µg, about 10 µg, about 20 µg, about 30 µg, about 40 µg, about 50 µg, about 100 µg, about 200 µg, about 300 µg, about 400 µg, or about 500 µg.

In the pharmaceutical formulations of the present invention encompassing oxybutynin, the unit dose for oral, transmucosal, topical, transdermal, and parenteral administration of said oxybutynin will be in an amount equal to or less than about 5 mg, about 4.5 mg, about 4 mg, about 3.5 mg, about 3 mg, about 2.5 mg, about 2 mg, about 1.5 mg, about 1.25 mg, about 1.0 mg, or about .5 mg. Because of the synergistic action of the α₂δ subunit calcium channel modulators gabapentin, pregabalin or the pharmaceutically acceptable, pharmacologically active acid, salt, ester or amide thereof when combined with antimuscarinics, dosages of α₂δ subunit calcium channel modulators and antimuscarinics that have been known in the art or predicted not to be effective for treating and/or alleviating the symptoms associated with painful and non-painful lower urinary tract disorders in normal and spinal cord injured patients are effective when administered as described herein.

A therapeutically effective amount of a particular active agent administered to a given individual will, of course, be dependent on a number of factors, including the concentration of the specific active agent, composition or dosage form, the selected mode of administration, the age and general condition of the individual being treated, the sex of the individual, the severity of the individual's condition, and other factors known to the prescribing physician.

In a preferred embodiment, drug administration is on an as-needed basis, and does not involve chronic drug administration. With an immediate release dosage form, as-needed administration may involve drug administration immediately prior to commencement of an activity wherein suppression of the symptoms of overactive bladder would be desirable, but will generally be in the range of from about 0 minutes to about 10 hours prior to such an activity, preferably in the range of from about 0 minutes to about 5 hours prior to such an activity, most preferably in the range of from about 0 minutes to about 3 hours prior to such an activity. With a sustained release dosage form, a single dose can provide therapeutic efficacy over an extended time period in the range of from about 1 hour to about 72 hours, typically in the range of from about 8 hours to about 48 hours, depending on the formulation. That is, the release period may be varied by the selection and relative quantity of particular sustained release polymers. If necessary, however, drug administration may be carried out within the context of an ongoing dosage regimen, i.e., on a weekly basis, twice weekly, daily, etc.

In another preferred embodiment, at least one detrimental side effect associated with single administration of the α₂δ subunit calcium channel modulator gabapentin, pregabalin or the pharmaceutically acceptable, pharmacologically active acid, salt, ester or amide theoreof or an antimuscarinic is lessened by concurrent administration of the α₂δ subunit calcium channel modulator gabapentin or pregabalin with an antimuscarinic. For example, side effects for oxybutynin, an antimuscarinic smooth muscle modulator, include dry mouth, sensitivity to bright light, blurred vision, dry eyes, decreased sweating, flushing, upset stomach, constipation, and drowsiness. However, when administered in combination with an α₂δ subunit calcium channel modulator such as gabapentin, significantly less of each agent is needed to achieve therapeutic efficacy (e.g., less than the 5 mg dose of oxybutynin currently marketed in the United States and also less than the 2.5 mg dose of oxybutynin currently marketed in Europe). Because detrimental side effects are lessened, the present invention also has the benefit of improving patient compliance.

### Packaged Kits

In another embodiment, a packaged kit is provided that contains the pharmaceutical formulation to be administered, i.e., a pharmaceutical formulation containing a therapeutically effective amount of the α₂δ subunit calcium channel modulator gabapentin or pregabalin in combination with an antimuscarinic for treating and/or alleviating the symptoms associated with painful and non-painful lower urinary tract disorders, including associated irritative symptoms in normal and spinal cord injured patients, a container, preferably sealed, for housing the formulation during storage and prior to use, and instructions for carrying out drug administration in a manner effective for treating and/or alleviating the symptoms associated with painful and non-painful lower urinary tract disorders, including associated irritative symptoms in normal and spinal cord injured patients. The instructions will typically be written instructions on a package insert and/or on a label. Depending on the type of formulation and the intended mode of administration, the kit may also include a device for administering the formulation. Formulations may be any suitable formulations as described herein. For example, formulations may be an oral dosage form containing a unit dosage of a selected active agent.

The kit may contain multiple formulations of different dosages of the same agent. The kit may also contain multiple formulations of different active agents. The kit may contain formulations suitable for sequential, separate and/or simultaneous use in treating and/or alleviating the symptoms associated with lower urinary tract disorders, and instructions for carrying out drug administration where the formulations are administered sequentially, separately and/or simultaneously in treating and/or alleviating the symptoms associated with lower urinary tract disorders.

The kit may also contain at least one component selected from the α₂δ subunit calcium channel modulators gabapentin and pregabalin and an antimuscarinic; a container housing said component or components during storage and prior to administration; and instructions for carrying out drug administration of gabapentin or pregabalin with an antimuscarinic in a manner effective to treat said lower urinary tract disorder. Such a kit may be useful, for example, where the gabapentin or pregabalin or the antimuscarinic is already being administered to the patient, and the additional component is to be added to the patient's drug regimen. Such a kit may also be useful where different individuals (e.g., physicians or other medical professionals) are administering the separate components of the combination of the present invention,

The parts of the kit may be independently held in one or more containerssuch as bottles, syringes, plates, wells, blister packs, or any other type of pharmaceutical packaging.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended embodiments. Although specific terms are employed herein, they are used in a generic and descriptive sense only and not for purposes of limitation.

### EXAMPLES

### Methods For Treating and/or Alleviating the Symptoms Associated With Lower Urinary Tract Disorders Using the α₂δ Subunit Calcium Channel Modulators gabapentin, pregabalin or the pharmaceutically acceptable, pharmacologically active acid, salt, ester or amide thereof. With Smooth Muscle Modulators

The invention will be further described in the following examples, which do not limit the scope of the invention described in the claims. The following examples illustrate the effects of administration of the combination of the α₂δ subunit calcium channel modulators gabapentin or pregabalin and an antimuscarinic on bladder capacity in an irritated bladder model. It is expected that these results will demonstrate the efficacy of the combination of the α₂δ subunit calcium channel modulators gabapentin or pregabalin and an antimuscarinic for treating and/or alleviating the symptoms associated with painful and non-painful lower urinary tract disorders in normal and spinal cord injured patients as described herein.

These methods include the use of a well accepted model of for urinary tract disorders involving the bladder using intravesically administered acetic acid as described in Sasaki *et al.* (2002) *J. Urol.* 168: 1259-64 and Thor and Katofiasc (1995) *J. Pharmacol. Exptl. Ther.* 274: 1014-24. Efficacy for treating spinal cord injured patients can be tested using methods as described in Yoshiyama *et al.* (1999) *Exp. Neurol.* 159: 250-7.

The present invention encompasses the use of antimuscarinics except for atropine, scopolomine, and trospium chloride. It is noted that each of these compounds all contain an amine embedded in an 8-azabicyclo[3.2.1]octan-3-ol skeleton.

### Example 1 - Dilute Acetic Acid Model: Gabapentin and Oxybutynin

### Objective and Rationale

The objective of this study was to determine the ability of an α₂δ subunit calcium channel modulator in combination with an antimuscarinic to reverse the reduction in bladder capacity seen following continuous infusion of dilute acetic acid, a commonly used model of overactive bladder. In particular, the current study utilized gabapentin as an exemplary α₂δ subunit calcium channel modulator, and oxybutynin as an exemplary antimuscarinic.

### Materials and Methods

Urethane anesthetized (1.2 g/kg) normal female rats were utilized in this study. Groups of rats were treated with oxybutynin alone (n=13), gabapentin alone (n=11), and respective dose-matched combinations of oxybutynin and gabapentin (n=11). Subsequently, three series at markedly lower doses and at different dose ratios were performed for the purposes of isobologram construction (n=4/group). Cumulative dose-response protocols were utilized with half log increments for all studies.

### Drugs and Preparation

Drugs were dissolved in normal saline at 1, 3 and 10 mg/ml for oxybutynin and 30,100 and 300 mg/ml for gabapentin. In these studies, individual doses and combinations may be subsequently referred to as Low, Mid and High.

Subsequent studies aimed at isobologram construction combined the drugs in dose combinations as shown in the table below (low, middle and high doses for each drug paired). Animals were dosed by volume of injection = body weight in kg.

**Table 1: Isobologram Dose Combinations (mg/kg)**

| **Isobologram Dose Combinations** | Combination 1 (n=4) | Combination 2 (n=4) | Combination 3 (n=4) |
|---|---|---|---|
| Oxybutynin | 0.1, 0.3, 1.0 | 0.1, 0.3, 1.0 | 0.03, 0.1, 0.3 |
| Gabapentin | 1.0, 3.0, 10.0 | 3.0, 10.0, 30.0 | 3.0, 10.0, 30.0 |

### Acute Anesthetized In Vivo Model

***Animal Preparation:*** Female rats (250-300 g body weight) were anesthetized with urethane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording. The abdominal cavity was moistened with saline and closed by covering with a thin plastic sheet in order to maintain access to the bladder for emptying purposes. Fine silver or stainless steel wire electrodes were inserted into the external urethral sphincter (EUS) percutaneously for electromyography (EMG).

***Experimental Design:*** Saline was continuously infused at a rate of 0.055 ml/min via the bladder-filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control saline cystometry period, the third vehicle, and 20 minutes following each subsequent treatment, the infusion pump was stopped, the bladder was emptied by fluid withdrawal via the infusion catheter and a single filling cystometrogram was performed at the same flow rate in order to determine changes in bladder capacity caused by the irritation protocol and subsequent intravenous drug administration.

### Data Analysis

Bladder capacity data for each animal were normalized to "% Recovery from Irritation," and this index was used as the measure of efficacy. Data from experiments in which each of the drugs were administered alone were utilized to create theoretical populations of additive effects for each dose (low, mid and high), and these were compared by one-tailed t-test (individual dose comparisons) and by 2-Way ANOVA (across doses) to the actual combination drug data. The means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N_{α2δ subunit modulator}) - 1. P<0.050 was considered significant. Only rats that showed between a 50-90% reduction in bladder capacity at the third vehicle measurement when compared to pre-irritation saline control values were utilized for numerical analyses.

Isobologram construction consisted of two methods, both utilizing the same data, but plotting the results either as group means or by individual responses. When utilizing group mean data, the common maximal effect reached by both drugs alone and the combinations listed in the above table was a return to 43% of saline control bladder capacity values. When utilizing individual responses for both drugs alone and the combinations listed in the above table, the target value was 31 % of saline control. These low values reflect the modest effectiveness of oxybutynin and gabapentin alone. For statistical purposes, the data were analyzed making comparisons for each drug, regardless of whether alone or in combination.

### Results and Conclusions

The effect of cumulative increasing doses of oxybutynin (n=13), gabapentin (n=11) and their matched combinations (e.g. Dose 1 for the combination was 30 mg/kg gabapentin and 1mg/kg oxybutynin; n=11) on bladder capacity is depicted in Figure 1. Data are normalized to saline controls and are presented as Mean ± SEM.

The effect of cumulative increasing doses of oxybutynin (n=13), gabapentin (n=11) and their matched combinations (e.g. Dose 1 for the combination was 30 mg/kg gabapentin and 1 mg/kg oxybutynin; n=11) on bladder capacity (normalized to % Recovery from Irritation) is depicted in Figure 2. Note that the combination of drugs produced a greater than additive effect at the Low (P=0.0031) and Mid doses (P=0.0403), on reduction in bladder capacity caused by continuous intravesical exposure to dilute acetic acid. Synergy is also suggested by significant differences between Additive and Combination effects by 2-Way ANOVA (P=0.0046). Data are presented as Mean ± SEM.

Results of the isobologram studies as determined by utilizing group means to determine effective doses is depicted in Figure 3. Using this technique, the common maximal effect for either drug alone was return to 43% of saline control. The line connecting the two axes at the effective dose for each drug alone represents theoretical additivity. The three isolated points clustered in the lower left field of the graph below the line of additivity represent the dose ranges from three sets of experiments utilizing low-dose ratios of drug combinations. As can be readily visualized by this isobologram, dramatically lower doses of both drugs were required in combination to achieve the same endpoint as either drug alone.

A common maximal effect of individual animals was determined (a return to 31% of saline control values; Figure 4). Using this approach, it was possible to show that no overlap existed between the doses of oxybutynin alone and those used in the isobologram combination studies in terms of standard deviation, and that all effective combination ranges of oxybutynin were significantly lower than the range of oxybutynin alone. Similarly, the effective ranges of gabapentin used in the combinations were significantly lower than when gabapentin was used alone. Data are presented as Mean ± SD.

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful lower urinary tract disorders and associated irritative symptoms in normal and spinal cord injured patients. Furthermore, the combination of an α₂δ subunit calcium channel modulator and an antimuscarinic produced a synergistic effect that was greater than what would be expected if the effects were simply additive, and also demonstrated efficacy using amounts of the individual agents that are much lower than would be expected to produce an effect if the agents were administered singly.

### Example 2 - Pharmacokinetic Analysis: Gabapentin and Oxybutynin

### Objective and Rationale

The purpose of this study was to determine concentrations of gabapentin, oxybutynin and desethyl oxybutynin in rat plasma samples over a 2 hour period following either 3 mg/kg oxybutynin, 100 mg/kg gabapentin, or the combination of those 2 drugs at those doses using a liquid chromatography with tandem mass spectrometric detection (LC/MS/MS) method.

### Materials and Methods

Urethane anesthetized (1.2 g/kg) normal female rats were utilized in this study. Groups of rats were treated with oxybutynin alone (n=6), gabapentin alone (n=8), and respective dose-matched combinations of oxybutynin and gabapentin (n=8).

### Drugs and Preparation

Drugs were dissolved in normal saline at 3 mg/ml for oxybutynin and 100 mg/ml for gabapentin. Animals were dosed by volume of injection = body weight in kg.

### Pharmacokinetic In Vivo Preparation

***Animal Preparation:*** Female rats (250-300 g body weight) were anesthetized with urethane (1.2 g/kg) and a saline-filled catheter (PE-50) was inserted into the jugular vein for intravenous drug administration.

***Experimental Design:*** Plasma samples (200 µl; K3 EDTA) were taken on ice at 4 time points (15, 30 60 and 120 minutes) following intravenous drug administration. Samples were spun at 1600 RPM for 7 minutes, plasma was drawn off and stored at -80 C until chromatographic analysis.

### Pharmacokinetic Chromatographic Analysis

***Internal Standards:*** Oxybutynin-D₁₁ chloride and baclofen were used as internal standards.

### LC/MS/MS Sample Analysis

**Method Summary**

| | |
|---|---|
| Analyte | Gabapentin, Oxybutynin and Desethyloxybutynin |
| Internal Standard (ISTD) | Baclofen and Oxybutynin-D₁₁ |
| Matrix | Rat plasma (K₃ EDTA) |
| Extraction | Protein precipitation |
| LC/MS/MS Instrumentation | Sciex API-3000 |
| Ionization Mode | Electrospray positive |

**Stock Solution Preparation**

| Solution ID | Stock Concentration | Solvent |
|---|---|---|
| Gabapentin | 200 µg/mL | MeOH |
| Oxybutynin | 200 µg/mL | ACN |
| Desethyloxybutynin | 200 µg/mL | ACN |
| Baclofen stock | 100 µg/mL | MeOH |
| Oxybutynin-D₁₁ stock | 100 µg/mL | ACN |

**Preparation of Intermediate Standard and Internal Standard Working Solutions**

| Working Solution ID | Source Solution ID | Source Solution Concentration (µg/mL) | Source Solution Volume (mL) | Final Total Volume (mL) | Final Solution Concentration (ng/mL) | Solvent |
|---|---|---|---|---|---|---|
| Initial STD | Gabapentin stock | 200 | 0.400 | | | |
| | Oxybutynin stock | 200 | 0.400 | 5.00 | 16000 | Rat plasma |
| | Desethyloxybutynin stock | 200 | 0.400 | | | |
| Working-IS | Baclofen stock | 100 | 0.010 | 100 | 10.0 | ACN |
| | Oxybutynin-D₁₁ stock | 100 | 0.010 | | | |

**Preparation of Calibration Standards**

| Working Solution ID | Source Solution ID | Source Solution Concentration (µg/mL) | Source Solution Volume (mL) | Final Total Volume (mL) | Final Solution Concentration (ng/mL) | Matrix |
|---|---|---|---|---|---|---|
| STD-1 | Initial STD | 16.0 | 0.050 | 0.200 | 4000 | Rat plasma |
| STD-2 | STD-1 | 4.00 | 0.050 | 0.200 | 1000 | Rat plasma |
| STD-3 | STD-1 | 1.00 | 0.050 | 0.200 | 250 | Rat plasma |
| STD-4 | STD-3 | 0.250 | 0.050 | 0.200 | 62.5 | Rat plasma |
| STD-5 | STD-4 | 0.063 | 0.050 | 0.200 | 15.6 | Rat plasma |
| STD-6 | STD-5 | 0.016 | 0.050 | 0.200 | 3.91 | Rat plasma |
| STD-7 | STD-6 | 0.004 | 0.050 | 0.200 | 0.977 | Rat plasma |

All stock solutions and working internal standard were stored at 2-8°C. Initial standard was stored frozen at approximatrely -20°C.

**Extraction Procedure**

| | |
|---|---|
| 1 | Include solvent blank, a blank matrix (double blank) and a Control 0 (blank matrix spiked with IS) with the calibration curve. |
| 2 | Aliquot 50.0 µL of control rat plasma, calibration standards or study sample, as appropriate, to a 96-well elution plate. |
| 3 | To Control 0, calibration and study samples, add 200 µL of working-IS solution. To solvent blank and blank matrix, add 200 µL of acetonitrile. |
| 4 | Vortex-mix all tubes for 30 seconds. |
| 5 | Centrifuge at 2800 rpm for 10 minutes. |
| 6 | Transfer the supernatant to a second 96-well elution plate. |
| 7 | Inject 20 µL onto the LC/MS/MS system for analysis. |

**Chromatographic Conditions**

| | | | |
|---|---|---|---|
| Column | Genesis C18, 4 µm, 50 x 2.1 mm | | |
| Mobile Phase A | 0.1% formic acid in water. | | |
| Mobile Phase B | 0.1% formic acid in acetonitrile. | | |
| Flow Rate | 0.5 mL/min | | |
| Injection Volume | 20 µL | | |
| Column Temperature | 35°C | | |

| Gradient | Time | %B | Switching Valve |
|---|---|---|---|
| | 0.01 | 5 | Waste |
| | 0.7 | 5 | Waste |
| | 1.3 | 80 | MS |
| | 1.9 | 80 | MS |
| | 2.0 | 5 | MS |
| | 3.0 | Stop | |
| Run Time | 3 minutes. | | |

**Mass Spectrometric Conditions (Sciex)**

| | | | | | | |
|---|---|---|---|---|---|---|
| Instrument | | API 3000 | | | | |
| Ionization Mode | | TurboIonspray | | | | |
| Polarity | | Positive | | | | |
| Scan Function | | Multiple Reaction Monitoring (MRM) | | | | |

| Parameters | | Oxybutynin | Desethyloxybutynin | Gabapentin | Baclofen | Oxybutynin-D₁₁ |
|---|---|---|---|---|---|---|
| | Precursor Ion | 358.4 | 330.4 | 172.3 | 214.2 | 369.5 |
| | Product Ion | 142.2 | 96.2 | 137.1 | 151.1 | 142.2 |
| | Dwell Time (ms) | 150 | 150 | 150 | 50 | 50 |
| | DP - Declustering Potential (V) | 42 | 32 | 27 | 27 | 42 |
| | FP - Focusing Potential (V) | 115 | 100 | 80 | 80 | 115 |
| | CE - Collision Energy (eV) | 34 | 24 | 23 | 26 | 36 |
| | CXP - Collision Cell Exit Potential (V) | 15 | 16 | 6 | 8 | 10 |
| | IS - Ionspray Voltage (V) | 2200 | | | | |
| | TEM - Turbo Gas Temperature (°C) | 500 | | | | |
| | NEB - Nebulizer Gas | 12 | | | | |
| | CUR - Curtain Gas | 8 | | | | |
| | CAD - Collision Gas | 10 | | | | |
| | Resolution | Unit | | | | |
| Software | | Analyst 1.1 | | | | |
| Regression (weighting) | | 1/x² | | | | |

***Calculations:*** Calculations were perfonned using Excel Version 8.0e. Some reported values may differ in the last reported digit from values calculated directly from the report tables due to the rounding that has been applied.

***Pharmacokinetic Analysis:*** The maximum concentration (Cₘₐₓ) in rat plasma and the time to reach maximum concentration (Tₘₐₓ) were obtained by visual inspection of the raw data. Pharmacokinetic parameters calculated included half-life (t_{1/2}), time to maximum plasma concentration (Tₘₐₓ), area under the concentration-time curve from time 0 to the last time point (AUC₀₋ₜ), area under the concentration-time curve from 0 to infinity (AUC_{0-∞}), volume of distribution (V_{z}), and clearance (CL). Pharmacokinetic parameters were calculated by using WinNonlin Professional Edition (Pharsight Corporation, Version 3.3).

### Results and Conclusions

For gabapentin (Table 2), the elimination phase of the concentration vs. time profiles was not well defined. Based on the comparison of Cₘₐₓ and AUC₀₋ₜ data, there appeared to be no appreciable difference between the oxybutynin (Oxy) group and the combination (Com) group. No evidence of drug-drug interaction between oxybutynin and gabapentin was found with the current study design.

For oxybutynin (Table 3), the pharmacokinetic parameters (Cₘₐₓ, AUC₀₋ₜ, AUC_{0-∞}, t_{1/2}, V_{z} and CL) obtained from the combination (Com) group did not appear to be appreciably different than those from the oxybutynin (Oxy) group. No evidence of drug-drug interaction between oxybutynin and gabapentin was found with the current study design.

For desethyl oxybutynin (Table 4), the elimination phase of the concentration vs. time profile was not well defined. However, based on the comparison of Cₘₐₓ and AUC₀₋ₜ data, there again appeared to be no appreciable difference between the oxybutynin (Oxy) group and the combination (Com) group.

The results of the pharmacokinetic study indicate that pharmacokinetic influences of one drug on the other do not account for the synergistic nature of the oxybutynin-gabapentin combination as seen in Example 1. That is to say that the synergistic nature of the positive effect of the combination on lower urinary tract function is not due to some pharmacokinetic interaction.

**Table 2**

| **Pharmacokinetic parameters for gabapentin in rat plasma** | | | | | | | | | |
|---|---|---|---|---|---|---|---|---|---|
| Treatment | Animal | Dose Level | Cₘₐₓ | Tₘₐₓ | AUC₀₋₁ | AUC_{0-∞} | t_{1/2} | V_{z} | CL |
| | | (mg/kg) | (ng/mL) | (minutes) | (min*ng/mL) | (min*ng/mL) | (minutes) | (mL/kg) | (mL/min/kg) |
| Com | 7 | 100 | 1.13E+05 | 60 | 1.26E+07 | NC | NC | NC | NC |
| Com | 8 | 100 | 1.01E+05 | 30 | 1.08E+07 | 4.59E+07 | 303 | 951 | 2.18 |
| Com | 9 | 100 | 9.33E+04 | 15 | 1.05E+07 | 7.06E+07 | 519 | 1060 | 1.42 |
| Com | 10 | 100 | 1.03E+05 | 15 | 8.76E+06 | 1.51E+07 | 97.3 | 928 | 6.61 |
| Com | 11 | 100 | 1.56E+05 | 60 | 1.40E+07 | NC | NC | NC | NC |
| Com | 20 | 100 | 1.00E+05 | 15 | 1.07E+07 | NC | NC | NC | NC |
| Com | 23 | 100 | 1.12E+05 | 15 | 1.10E+07 | 4.39E+07 | 296 | 975 | 2.28 |
| Com | 24 | 100 | 1.03E+05 | 30 | 1.16E+07 | NC | NC | NC | NC |
| Mean | | | 1.10E+05 | | 1.13E+07 | 4.39E+07 | 304 | 978 | 3.12 |
| SD | | | 1.96E+04 | | 1.56E+06 | 2.27E+07 | 172 | 57.4 | 2.36 |
| Gab | 4 | 100 | 1.07E+05 | 15 | 1.25E+07 | NC | NC | NC | NC |
| Gab | 5 | 100 | 1.12E+05 | 15 | 1.02E+07 | 1.95E+07 | 116 | 857 | 5.12 |
| Gab | 6 | 100 | 1.07E+05 | 15 | 8.56E+06 | 1.37E+07 | 86.2 | 910 | 7.32 |
| Gab | 12 | 100 | 1.10E+05 | 15 | 1.01E+07 | 2.19E+07 | 135 | 890 | 4.57 |
| Gab | 13 | 100 | 9.52E+04 | 15 | 8.19E+06 | 1.44E+07 | 99.4 | 996 | 6.95 |
| Gab | 14 | 100 | 1.23E+05 | 120 | 1.28E+07 | NC | NC | NC | NC |
| Gab | 17 | 100 | *3.45E+01 | 120 | *2.12E+03 | NC | NC | NC | NC |
| Gab | 21 | 100 | 3.59E+04 | 30 | 3.80E+06 | 1.16E+07 | 205 | 2555 | 8.63 |
| Mean | | | 9.86E+04 | | 9.45E+06 | 1.62E+07 | 128 | 1242 | 6.52 |
| SD | | | 2.88E+04 | | 3.05E+06 | 4.32E+06 | 46.7 | 736 | 1.66 |

| | | | | | | | | | |
|---|---|---|---|---|---|---|---|---|---|
| AUC_{0-∞} Area under the plasma concentration-time curve up to infinity. AUC₀₋₁ Area under the plasma concentration-time curve up to the last sampling time with measurable concentrations. CL Clearance. Cₘₐₓ Maximum plasma concentration. NA Not applicable. NC Not calculated due to insufficient elimination phase data. SD Standard deviation. t_{1/2} Observed elimination half-life. Tₘₐₓ Time to maximum concentration. V_{z} Volume of distribution. * Outliers. Excluded from mean and SD calculations. | | | | | | | | | |

**Table 3**

| **Pharmacokinetic parameters for oxybutynin in rat plasma** | | | | | | | | | |
|---|---|---|---|---|---|---|---|---|---|
| Treatment | Animal | Dose Level | Cₘₐₓ | Tₘₐₓ | AUC₀₋ₜ | AUC_{0-∞} | t_{1/2} | V_{z} | CL |
| | | (mg/kg) | (ng/mL) | (minutes) | (min*ng/mL) | (min*ng/mL) | (minutes) | (mL/kg) | (mL/min/kg) |
| Com | 7 | 3 | 320 | 15 | 22152 | 28177 | 24.6 | 3774 | 106 |
| Com | 8 | 3 | 360 | 15 | 20737 | 23114 | 39.3 | 7363 | 130 |
| Com | 9 | 3 | 248 | 15 | 16201 | 19116 | 45.5 | 10301 | 157 |
| Com | 10 | 3 | 316 | 15 | 18387 | 20541 | 39.9 | 8411 | 146 |
| Com | 11 | 3 | 282 | 15 | 16057 | 18295 | 43.3 | 10252 | 164 |
| Com | 20 | 3 | 367 | 15 | 21889 | 26725 | 53.0 | 8590 | 112 |
| Com | 23 | 3 | 342 | 15 | 19405 | 21702 | 41.5 | 8270 | 138 |
| Com | 24 | 3 | 295 | 15 | 17222 | 19529 | 41.2 | 9136 | 154 |
| Mean | | | 316 | | 19006 | 22150 | 41.0 | 8262 | 138 |
| SD | | | 40.4 | | 2435 | 3624 | 7.97 | 2069 | 20.9 |
| Oxy | 1 | 3 | 228 | 15 | 15566 | 21438 | 72.8 | 14701 | 140 |
| Oxy | 2 | 3 | 448 | 15 | 24555 | 28547 | 55.6 | 8425 | 105 |
| Oxy | 3 | 3 | 238 | 15 | 12865 | 14181 | 39.8 | 12158 | 212 |
| Oxy | 15 | 3 | 217 | 15 | 15880 | 20477 | 56.8 | 12004 | 147 |
| Oxy | 16 | 3 | 419 | 15 | 23333 | 24944 | 32.5 | 5632 | 120 |
| Oxy | 18 | 3 | 426 | 15 | 28295 | 38044 | 66.9 | 7612 | 78.9 |
| Mean | | | 329 | | 20082 | 24605 | 54 | 10089 | 134 |
| SD | | | 112 | | 6135 | 8149 | 15.5 | 3405 | 45.3 |

| | | | | | | | | | |
|---|---|---|---|---|---|---|---|---|---|
| AUC_{0-∞} Area under the plasma concentration-time curve up to infinity. AUC₀₋₁ Area under the plasma concentration-time curve up to the last sampling time with measurable concentrations. CL Clearance. Cₘₐₓ Maximum plasma concentration. NA Not applicable. NC Not calculated due to insufficient elimination phase data. SD Standard deviation. t_{1/2} Observed elimination half-life. Tₘₐₓ. Time to maximum concentration. V_{z} Volume of distribution. | | | | | | | | | |

**Table 4**

| | **Pharmacokinetic parameters for desethyl oxybutynin in rat plasma** | | | | | | | | |
|---|---|---|---|---|---|---|---|---|---|
| Treatment | Animal | Dose Level | Cₘₐₓ | Tₘₐₓ | AUC₀₋₁ | AUC_{0-∞} | t_{1/2} | V_{z} | CL |
| | | (mg/kg) | (ng/mL) | (minutes) | (min*ng/mL) | (min*ng/mL) | (minutes) | (mL/kg) | (mL/min/kg) |
| Com | 7 | 3 | 1.19 | 15 | 68.0 | 471 | 266 | 2444603 | 6370 |
| Com | 8 | 3 | 1.15 | 15 | 65.5 | 495 | 292 | 2551693 | 6066 |
| Com | 9 | 3 | 1.57 | 30 | 176 | 877 | 365 | 1801875 | 3420 |
| Com | 10 | 3 | 1.71 | 15 | 163 | 404 | 167 | 1788610 | 7426 |
| Com | 11 | 3 | 1.47 | 15 | 80.9 | 301 | 133 | 1907790 | 9965 |
| Com | 20 | 3 | 3.84 | 15 | 345 | 880 | 158 | 776714 | 3408 |
| Com | 23 | 3 | 3.23 | 15 | 264 | 493 | 113 | 992758 | 6088 |
| Com | 24 | 3 | 1.80 | 15 | 177 | 442 | 160 | 1563846 | 6788 |
| Mean | | | 2.00 | | 168 | 545 | 207 | 1728486 | 6191 |
| SD | | | 0.99 | | 99.1 | 215 | 89.7 | 621739 | 2125 |
| Oxy | 1 | 3 | 3.6 | 15 | 306 | 716 | 158 | 954133 | 4191 |
| Oxy | 2 | 3 | 1.55 | 15 | 47.7 | 99 | 32.0 | 1392698 | 30168 |
| Oxy | 3 | .3 | 1.7 | 15 | 53.4 | 92 | 24.4 | 1142356 | 32463 |
| Oxy | 15 | 3 | 1.18 | 60 | 69.7 | NC | NC | NC | NC |
| Oxy | 16 | 3 | 1.59 | 15 | 83.9 | 247 | 100 | 1754810 | 12124 |
| Oxy | 18 | 3 | 2.81 | 120 | 306 | NC | NC | NC | NC |
| Mean | | | 2.07 | | 144 | 289 | 78.6 | 1310999 | 19737 |
| SD | | | 0.93 | | 126 | 293 | 62.9 | 346139 | 13789 |

| | | | | | | | | | |
|---|---|---|---|---|---|---|---|---|---|
| AUC_{0-∞} Area under the plasma concentration-time curve up to infinity. AUC₀₋₁ Area under the plasma concentration-time curve up to the last sampling time with measurable concentrations. CL Clearance. Cₘₐₓ Maximum plasma concentration. NA Not applicable. NC Not calculated due to insufficient elimination phase data. SD Standard deviation. t_{1/2} Observed elimination half-life. Tₘₐₓ Time to maximum concentration. V_{z} Volume of distribution. | | | | | | | | | |

### Example 3 - Dilute Acetic Acid Model: Pregabalin and Oxybutynin

### Objective and Rationale

The objective of this study was to determine the ability of an α₂δ subunit calcium channel modulator in combination with an antimuscarinic to reverse the reduction in bladder capacity seen following continuous infusion of dilute acetic acid, a commonly used model of overactive bladder. In particular, the current study utilized pregabalin as an exemplary α₂δ subunit calcium channel modulator, and oxybutynin as an exemplary antimuscarinic.

### Materials and Methods

Urethane anesthetized (1.2 g/kg) normal female rats were utilized in this study. Groups of rats were treated with oxybutynin alone, pregabalin alone, and respective dose-matched combinations of oxybutynin and pregabalin.

### Drugs and Preparation

In one series of studies, drugs were dissolved in normal saline at 1, 3 and 10 mg/ml for oxybutynin and 10, 30 and 100 mg/ml for pregabalin. In these studies, individual doses and combinations may be subsequently referred to as Low, Mid and High. Animals were dosed by volume of injection = body weight in kg.

In another series of studies, drugs were dissolved in normal saline at 0.625, 1.25, 2.5, 5.0 and 10 mg/ml for oxybutynin and 3.75, 7.5, 15, 30 and 60 mg/ml for pregabalin. In these studies, individual doses and combinations may be subsequently referred to as Low, Mid Low, Mid, Mid High and High. Animals were dosed by volume of injection = body weight in kg.

### Acute Anesthetized In Vivo Model

***Animal Preparation:*** Female rats (250-300 g body weight) were anesthetized with urethane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording. The abdominal cavity was moistened with saline and closed by covering with a thin plastic sheet in order to maintain access to the bladder for emptying purposes. Fine silver or stainless steel wire electrodes were inserted into the external urethral sphincter (EUS) percutaneously for electromyography (EMG).

***Experimental Design:*** Saline was continuously infused at a rate of 0.055 ml/min via the bladder-filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control saline cystometry period, the third vehicle, and 20 minutes following each subsequent treatment, the infusion pump was stopped, the bladder was emptied by fluid withdrawal via the infusion catheter and a single filling cystometrogram was performed at the same flow rate in order to determine changes in bladder capacity caused by the irritation protocol and subsequent intravenous drug administration.

### Data Analysis

Bladder capacity data for each animal were normalized to "% Recovery from Irritation," and this index was used as the measure of efficacy. Data from experiments in which each of the drugs were administered alone were utilized to create theoretical populations of additive effects for each dose (low, mid and high), and these were compared by one-tailed t-test (individual dose comparisons) and by 2-Way ANOVA (across doses) to the actual combination drug data. The means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N_{α2δ subunit modulator}) - 1. P<0.050 was considered significant. Only rats that showed between a 50-90% reduction in bladder capacity at the third vehicle measurement when compared to pre-irritation saline control values were utilized for numerical analyses.

### Results and Conclusions

The effect of cumulative increasing doses of oxybutynin (n=13), pregabalin (n=7) and matched combinations (e.g. Dose 1 for the combination was 10 mg/kg pregabalin and 1 mg/kg oxybutynin; n=9) on bladder capacity is depicted in Figure 5. Data are normalized to saline controls and are presented as Mean ± SEM.

The effect of cumulative increasing doses of oxybutynin (n=13), pregabalin (n=7) and matched combinations (e.g. Dose 1 for the combination was 10 mg/kg pregabalin and 1 mg/kg oxybutynin; n=9) on bladder capacity (normalized to % Recovery from Irritation) is depicted in Figure 6. Data are presented as Mean ± SEM. Note that the combination of drugs produced a greater than additive effect at the Low (P=0.0386), Mid (P=0.0166) and High doses (P=0.0098), on reduction in bladder capacity caused by continuous intravesical exposure to dilute acetic acid Synergy is also suggested by significant differences between Additive and Combination effects by 2-Way ANOVA (P<0.0004).

The effect of cumulative increasing doses of oxybutynin (n=4), pregabalin (n=7) and matched combinations (e.g. Dose 1 for the combination was 3.75 mg/kg pregabalin and 0.625 mg/kg oxybutynin; n=4) on bladder capacity is depicted in Figure 7. Data are normalized to saline controls and are presented as Mean ± SEM.

The effect of cumulative increasing doses of oxybutynin (n=4), pregabalin (n=7) and their matched combinations (e.g. Dose 1 for the combination was 3.75 mg/kg pregabalin and 0.625 mg/kg oxybutynin; n=4) on bladder capacity (normalized to % Recovery from Irritation) is depicted in Figure 8. Data are presented as Mean ± SEM. Note also that the combination of drugs produced a greater than additive effect at the Mid High (P=0.04) and High doses (P=0.004) on reduction in bladder capacity caused by continuous intravesical exposure to dilute acetic acid. Synergy is also suggested by significant differences between Additive and Combination effects by 2-Way ANOVA (P=0.0037).

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful and associated irritative symptoms lower urinary tract disorders in normal and spinal cord injured patients. Furthermore, the combination of an α₂δ subunit calcium channel modulator and an antimuscarinic produced a synergistic effect that was greater than what would be expected if the effects were simply additive.

### Example 4 - Dilute Acetic Acid Model: Gabapentin and Tolterodine

### Objective and Rationale

The objective of this study was to determine the ability of an α₂δ subunit calcium channel modulator in combination with an antimuscarinic to reverse the reduction in bladder capacity seen following continuous infusion of dilute acetic acid, a commonly used model of overactive bladder. In particular, the current study utilized gabapentin as an exemplary α₂δ subunit calcium channel modulator, and tolterodine as an exemplary antimuscarinic.

### Materials and Methods

Urethane anesthetized (1.2 g/kg) normal female rats were utilized in this study. Groups of rats were treated with tolterodine alone (n=9), gabapentin alone (n=11), and 2 combination studies characterized by single initial dose combinations of tolterodine (Mid and High) together with the Low dose of gabapentin, followed in turn by the Mid and High doses of gabapentin alone (n=4 and n=3, respectively).

### Drugs and Preparation

Drugs were dissolved in normal saline at 1, 3 and 10 mg/ml for tolterodine and 10, 30 and 100 mg/ml for gabapentin. In these studies, individual doses may be subsequently referred to as Low, Mid and High. Combinations are referred to as 3 mg/kg Tolt. Combination and 10 mg/kg Tolt. Combination. Animals were dosed by volume of injection = body weight in kg.

### Acute Anesthetized In Vivo Model

***Animal Preparation:*** Female rats (250-300 g body weight) were anesthetized with urethane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording. The abdominal cavity was moistened with saline and closed by covering with a thin plastic sheet in order to maintain access to the bladder for emptying purposes. Fine silver or stainless steel wire electrodes were inserted into the external urethral sphincter (EUS) percutaneously for electromyography (EMG).

***Experimental Design:*** Saline was continuously infused at a rate of 0.05 ml/min via the bladder-filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control saline cystometry period, the third vehicle, and 20 minutes following each subsequent treatment, the infusion pump was stopped, the bladder was emptied by fluid withdrawal via the infusion catheter and a single filling cystometrogram was performed at the same flow rate in order to determine changes in bladder capacity caused by the irritation protocol and subsequent intravenous drug administration.

### Data Analysis

Bladder capacity data for each animal were normalized to "% Recovery from Irritation," and this index was used as the measure of efficacy. Data from experiments in which each of the drugs were administered alone were utilized to create theoretical populations of additive effects for each dose (low, mid and high), and these were compared by one-tailed t-test (individual dose comparisons) and by 2-Way ANOVA (across doses) to the actual combination drug data. The means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N_{α2δ subunit modulator}) - 1. P<0.050 was considered significant. Only rats that showed between a 50-90% reduction in bladder capacity at the third vehicle measurement when compared to pre-irritation saline control values were utilized for numerical analyses.

### Results and Conclusions

The effect of cumulative increasing doses of tolterodine (n=9), gabapentin (n=11) and the 2 combinations tested (e.g. Dose 1 for the combination 1 was 30 mg/kg gabapentin and 3 mg/kg tolterodine; n=4 and 3 for 3 and 10 mg/kg tolterodine, respectively) on bladder capacity is depicted in Figure 9. Data are normalized to saline controls and are presented as Mean ± SEM.

The effect of cumulative increasing doses of tolterodine (n=9), gabapentin (n=11) and the 2 combinations (e.g. Dose 1 for the combination was 30 mg/kg gabapentin and 3 mg/kg tolterodine; n=4 and 3, for 3 mg/kg and 10 mg/kg tolterodine, respectively) on bladder capacity (normalized to % Recovery from Irritation) is depicted in Figure 10. Data are presented as Mean ± SEM. Note that the combination of drugs produced a greater than additive effect for the 3 mg/kg Tolt. Combination (P=0.0099) and the 10 mg/kg Tolt. Combination (P=0.0104).

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful lower urinary tract disorders and associated irritative symptoms in normal and spinal cord injured patients. Furthermore, the combination of an α₂δ subunit calcium channel modulator and an antimuscarinic produced a synergistic effect that was greater than what would be expected if the effects were simply additive.

### Example 5 - Dilute Acetic Acid Model: Pregabalin and Tolterodine

### Objective and Rationale

The objective of this study was to determine the ability of an α₂δ subunit calcium channel modulator in combination with an antimuscarinic to reverse the reduction in bladder capacity seen following continuous infusion of dilute acetic acid, a commonly used model of overactive bladder. In particular, the current study utilized pregabalin as an exemplary α₂δ subunit calcium channel modulator, and tolterodine as an exemplary antimuscarinic.

### Materials and Methods

Urethane anesthetized (1.2 g/kg) normal female rats were utilized in this study. Groups of rats were treated with tolterodine alone (n=9), pregabalin alone (n=7), and respective dose-matched combinations of tolterodine and pregabalin (n=9).

### Drugs and Preparation

Drugs were dissolved in normal saline at 1, 3 and 10 mg/ml for tolterodine and 10, 30 and 100 mg/ml for pregabalin. In these studies, individual doses and combinations may be subsequently referred to as Low, Mid and High. Animals were dosed by volume of injection = body weight in kg.

### Acute Anesthetized In Vivo Model

***Animal Preparation:*** Female rats (250-300 g body weight) were anesthetized with urethane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording. The abdominal cavity was moistened with saline and closed by covering with a thin plastic sheet in order to maintain access to the bladder for emptying purposes. Fine silver or stainless steel wire electrodes were inserted into the external urethral sphincter (EUS) percutaneously for electromyography (EMG).

***Experimental Design:*** Saline was continuously infused at a rate of 0.055 ml/min via the bladder-filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control saline cystometry period, the third vehicle, and 20 minutes following each subsequent treatment, the infusion pump was stopped, the bladder was emptied by fluid withdrawal via the infusion catheter and a single filling cystometrogram was performed at the same flow rate in order to determine changes in bladder capacity caused by the irritation protocol and subsequent intravenous drug administration.

### Data Analysis

Bladder capacity data for each animal were normalized to "% Recovery from Irritation," and this index was used as the measure of efficacy. Data from experiments in which each of the drugs were administered alone were utilized to create theoretical populations of additive effects for each dose (low, mid and high), and these were compared by one-tailed t-test (individual dose comparisons) and by 2-Way ANOVA (across doses) to the actual combination drug data. The means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N_{α2δ subunit modulator}) - 1. P<0.050 was considered significant. Only rats that showed between a 50-90% reduction in bladder capacity at the third vehicle measurement when compared to pre-irritation saline control values were utilized for numerical analyses.

### Results and Conclusions

The effect of cumulative increasing doses of tolterodine (n=9), pregabalin (n=7) and their matched combinations (e.g. Dose 1 for the combination was 10 mg/kg pregabalin and 1 mg/kg tolterodine; n=9) on bladder capacity is depicted in Ficure 11. Data are normalized to saline controls and are presented as Mean ± SEM.

The effect of cumulative increasing doses of tolterodine (n=9), pregabalin (n=7) and matched combinations (e.g. Dose 1 for the combination was 10 mg/kg pregabalin and 1 mg/kg tolterodine; n=9) on bladder capacity (normalized to % Recovery from Irritation) is depicted in Figure 12. Data are presented as Mean ± SEM. Note also that the combination of drugs produced a greater than additive effect at the Mid doses (P=0.0353) on reduction in bladder capacity caused by continuous intravesical exposure to dilute acetic acid. Synergy is also suggested by significant differences between Additive and Combination effects by 2-Way ANOVA (P<0.0234).

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful lower urinary tract disorders and associated irritative symptoms in normal and spinal cord injured patients. Furthermore, the combination of an α₂δ subunit calcium channel modulator and an antimuscarinic produced a synergistic effect that was greater than what would be expected if the effects were simply additive.

### Example 6 - Dilute Acetic Acid Model: Gabapentin and Propiverine

### Objective and Rationale

The objective of this study was to determine the ability of an α₂δ subunit calcium channel modulator in combination with an antimuscarinic to reverse the reduction in bladder capacity seen following continuous infusion of dilute acetic acid, a commonly used model of overactive bladder. In particular, the current study utilized gabapentin as an exemplary α₂δ subunit calcium channel modulator, and propiverine as an exemplary antimuscarinic.

### Materials and Methods

Urethane anesthetized (1.2 g/kg) normal female rats were utilized in this study. Groups of rats were treated with propiverine alone (n=7), gabapentin alone (n=11), and respective dose-matched combinations of propiverine and gabapentin (n=10).

### Drugs and Preparation

Drugs were dissolved in normal saline at 3, 10 and 30 mg/ml for propiverine and 10, 30 and 100 mg/ml for gabapentin. In these studies, individual doses and combinations may be subsequently referred to as Low, Mid and High. Animals were dosed by volume of injection = body weight in kg.

### Acute Anesthetized In Vivo Model

***Animal Preparation:*** Female rats (250-300 g body weight) were anesthetized with urethane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording. The abdominal cavity was moistened with saline and closed by covering with a thin plastic sheet in order to maintain access to the bladder for emptying purposes. Fine silver or stainless steel wire electrodes were inserted into the external urethral sphincter (EUS) percutaneously for electromyography (EMG).

***Experimental Design:*** Saline was continuously infused at a rate of 0.055 ml/min via the bladder-filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control saline cystometry period, the third vehicle, and 20 minutes following each subsequent treatment, the infusion pump was stopped, the bladder was emptied by fluid withdrawal via the infusion catheter and a single filling cystometrogram was performed at the same flow rate in order to determine changes in bladder capacity caused by the irritation protocol and subsequent intravenous drug administration.

### Data Analysis

Bladder capacity data for each animal were normalized to "%Irritation Control," and this index was used as the measure of efficacy. Data from experiments in which each of the drugs were administered alone were utilized to create theoretical populations of additive effects for each dose (low, mid and high), and these were compared by one-tailed t-test (individual dose comparisons) and by 2-Way ANOVA (across doses) to the actual combination drug data. The means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N_{α2δ subunit modulator}) - 1. P<0.050 was considered significant. Only rats that showed between a 50-90% reduction in bladder capacity at the third vehicle measurement when compared to pre-irritation saline control values were utilized for numerical analyses.

### Results and Conclusions

The effect of cumulative increasing doses of propiverine (n=7), gabapentin (n=11) and their matched combinations (e.g. Dose 1 for the combination was 10 mg/kg gabapentin and 3 mg/kg propiverine; n=10) on bladder capacity is depicted in Figure 13. Data are normalized to saline controls and are presented as Mean ± SEM.

The effect of cumulative increasing doses of propiverine (n=7), gabapentin (n=11) and their matched combinations (e.g. Dose 1 for the combination was 10 mg/kg gabapentin and 3 mg/kg propiverine; n=10) on bladder capacity (normalized to % Recovery from Irritation) is depicted in Figure 14. Data are presented as Mean ± SEM. Note that the combination of drugs produced a greater than additive effect at the Low (P=0.0087) and Mid doses (P=0.0253) on reduction in bladder capacity caused by continuous intravesical exposure to dilute acetic acid. Synergy is also suggested by significant differences between Additive and Combination effects by 2-Way ANOVA (P<0.0067).

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful lower urinary tract disorders and associated irritative symptoms in normal and spinal cord injured patients. Furthermore, the combination of an α₂δ subunit calcium channel modulator and an antimuscarinic produced a synergistic effect that was greater than what would be expected if the effects were simply additive.

### Example 7 - Dilute Acetic Acid Model: Gabapentin and Solifenacin

### Objective and Rationale

The objective of this study was to determine the ability of an α₂δ subunit calcium channel modulator in combination with an antimuscarinic to reverse the reduction in bladder capacity seen following continuous infusion of dilute acetic acid, a commonly used model of overactive bladder. In particular, the current study utilized gabapentin as an exemplary α₂δ subunit calcium channel modulator, and solifenacin as an exemplary antimuscarinic.

### Materials and Methods

Urethane anesthetized (1.2 g/kg) normal female rats were utilized in this study. Groups of rats were treated with solifenacin alone (n=7), gabapentin alone (n=11), and respective dose-matched combinations of solifenacin and gabapentin (n=10).

### Drugs and Preparation

Drugs were dissolved in normal saline at 1, 3 and 10 mg/ml for solifenacin and 10, 30 and 100 mg/ml for gabapentin. In these studies, individual doses and combinations may be subsequently referred to as Low, Mid and High. Animals were dosed by volume of injection = (body weight in kg) * 1.5.

### Acute Anesthetized In Vivo Model

***Animal Preparation:*** Female rats (250-300 g body weight) were anesthetized with urethane (1.2 g/kg) and a saline-filled catheter (PE-50) was inserted into the jugular vein for intravenous drug administration. Via a midline lower abdominal incision, a flared-tipped PE 50 catheter was inserted into the bladder dome for bladder filling and pressure recording. The abdominal cavity was moistened with saline and closed by covering with a thin plastic sheet in order to maintain access to the bladder for emptying purposes. Fine silver or stainless steel wire electrodes were inserted into the external urethral sphincter (EUS) percutaneously for electromyography (EMG).

***Experimental Design:*** Saline was continuously infused at a rate of 0.055 ml/min via the bladder-filling catheter for 60 minutes to obtain a baseline of lower urinary tract activity (continuous cystometry; CMG). Following the control period, a 0.25% acetic acid solution in saline was infused into the bladder at the same flow rate to induce bladder irritation. Following 30 minutes of AA infusion, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control saline cystometry period, the third vehicle, and 20 minutes following each subsequent treatment, the infusion pump was stopped, the bladder was emptied by fluid withdrawal via the infusion catheter and a single filling cystometrogram was performed at the same flow rate in order to determine changes in bladder capacity caused by the irritation protocol and subsequent intravenous drug administration.

### Data Analysis

Bladder capacity data for each animal were normalized to "% Recovery from Irritation," and this index was used as the measure of efficacy. Data from experiments in which each of the drugs were administered alone were utilized to create theoretical populations of additive effects for each dose (low, mid and high), and these were compared by one-tailed t-test (individual dose comparisons) and by 2-Way ANOVA (across doses) to the actual combination drug data. The means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N _{α2δ subunit modulator}) - 1. P<0.050 was considered significant. Only rats that showed between a 50-90% reduction in bladder capacity at the third vehicle measurement when compared to pre-irritation saline control values were utilized for numerical analyses.

### Results and Conclusions

The effect of cumulative increasing doses of solifenacin (n=4), gabapentin (n=11) and their matched combinations (e.g. Dose 1 for the combination was 10 mg/kg gabapentin and 3 mg/kg solifenacin; n=12) on bladder capacity is depicted in Figure 15. Data are normalized to saline controls and are presented as Mean ± SEM.

The effect of cumulative increasing doses of solifenacin (n=4), gabapentin (n=11) and their matched combinations (e.g. Dose 1 for the combination was 10 mg/kg gabapentin and 3 mg/kg solifenacin; n=12) on bladder capacity (normalized to % Irritation Control) is depicted in Figure 16. Data are presented as Mean ± SEM. Note that the combination of drugs produced a greater than additive effect at the Low (P<0.05) and High doses (P<0.05) on reduction in bladder capacity caused by continuous intravesical exposure to dilute acetic acid. Synergy is also suggested by significant differences between Additive and Combination effects by 2-Way ANOVA (P<0.0022).

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful lower urinary tract disorders and associated irritative symptoms in normal and spinal cord injured patients. Furthermore, the combination of an α₂δ subunit calcium channel modulator and an antimuscarinic produced a synergistic effect that was greater than what would be expected if the effects were simply additive.

### Example 8 - Dilute Acetic Acid Model in Cats: Gabapentin and Oxybutynin

### Objective and Rationale

The objective of this study was to determine the ability of an α₂δ subunit calcium channel modulator in combination with an antimuscarinic to reverse the reduction in bladder capacity seen following continuous infusion of dilute acetic acid in a cat model, a commonly used model of overactive bladder. In particular, the current study utilized gabapentin as an exemplary α₂δ subunit calcium channel modulator, and oxybutynin as an exemplary antimuscarinic.

### Materials and Methods

Alpha-chloralose anesthetized (50-100 mg/kg) normal female cats (2.5-3.5 kg; Harlan) were utilized in this study. Groups of cats were treated with oxybutynin alone (n=5), gabapentin alone (n=5), and selected dose-matched combinations of oxybutynin and gabapentin (n=6).

### Drugs and Preparation

Drugs were dissolved in normal saline at 0.01, 0.03, 0.1, 0.3, 1.0, 3.0 and 10mg/ml for oxybutynin and 3.0, 10, 30, 100 and 300 mg/ml for gabapentin. Combinations paired 0.1 mg/kg oxybutynin and 3 mg/kg gabapentin (Low), 0.3 mg/kg, oxybutynin and 10 mg/kg gabapentin (Mid), and 1.0 mg/kg oxybutynin and 30 mg/kg gabapentin (High). Animals were dosed by volume of injection = body weight in kg.

### Acute Anesthetized In Vivo Model

Female cats (2.5-3.5 kg; Harlan) had their food removed the night before the experiment. The following morning, the cat was anesthetized with isoflurane and prepped for surgery using aseptic technique. Polyethylene catheters were surgically placed to permit the measurement of bladder pressure, urethral pressure, arterial pressure, respiratory rate as well as for the delivery of drugs. Fine wire electrodes were implanted alongside the external urethral anal sphincter. Following surgery, the cats were slowly switched from the gas anesthetic isoflurane (2-3.5%) to alpha-chloralose (50-100 mg/kg). During control cystometry, saline was slowly infused into the bladder (0.5-1.0 ml/min) for 1 hour. The control cystometry was followed by 0.5% acetic acid in saline for the duration of the experiment. After assessing the cystometric variables under these baseline conditions, the effects of test drug(s) on micturition were determined via a 3-5 point dose response protocols.

### Data Analysis

For the purposes of assessing synergy using all of the data simultaneously, bladder capacity data for each animal were normalized to % Recovery from Irritation, and this index was used as the measure of efficacy. Data from the experiments in which each of the drugs were administered alone were utilized to create theoretical populations of additive effects for each dose (low, mid and high) and these were compared by one-tailed t-test (individual dose comparisons) and by 2-Way ANOVA (across doses) to the actual combination drug data. For these purposes, the means and standard deviations of each individual treatment's "dose-matched" (low, middle, and high) responses were added together to estimate the mean and standard deviation of the theoretical additive populations for which to compare to the actual data obtained from the combination experiments. The theoretical additive effect population N = (N_{antimuscarinic} + N_{α2δ subunit modulator}) - 1. Because gabapentin alone was not tested at the 3.0 and the 10.0 mg.kg doses, and because there was no significant effect for gabapentin for the 30 mg/kg dose alone, the response at 30 mg/kg was used as a surrogate for the 3.0 and 10.0 mg/kg response in order to calculate the theoretical additive polulation. P<0.050 was considered significant. Additionally, % Voiding Efficiency was determined by the following formula: (Voided Volume / (Voided + Residual Volume)) * 100 for oxybutynin alone, gabapentin alone and the combination.

### Results and Conclusions

The effect of cumulative increasing doses of oxybutynin (n=5), gabapentin (n=5) and their matched combinations (n=6) on bladder capacity is depicted in Figure 17. Data are normalized to saline controls and are presented as Mean ± SEM.

The theoretical additive effect of cumulative increasing doses of oxybutynin (n=5) and gabapentin (n=5), and their matched combinations (e.g. Dose 1 for the combination was 3 mg/kg gabapentin and 0.1 mg/kg oxybutynin; n=6) on bladder capacity (normalized to % Recovery from Irritation) is depicted in Figure 18. Data are presented as Mean ± SEM. Note that the combination of drugs produced a greater than additive effect at the Mid doses (P=0.0490) on reduction in bladder capacity caused by continuous intravesical exposure to dilute acetic acid.

The effect of cumulative increasing doses of oxybutynin (n=5), gabapentin (n=5) on voiding efficiency is depicted in Figure 19 (oxybutynin in Figure 19A, gabapentin in Figure 19B). Note the dose-dependent decrease in voiding efficiency caused by oxybutynin. Also note that gabapentin has no effect.

The effect of cumulative increasing doses of oxybutynin and gabapentin in combination (n=6) on voiding efficiency is depicted in Figure 20. Note that the dose-dependent decrease in voiding efficiency caused by oxybutynin is virtually prevented by co-administration of gabapentin.

At the highest oxybutynin (1 mg/kg) and gabapentin (30 mg/kg) dose combination tested in the cat, voiding efficiency was decreased only 16.7%. This is in striking contrast to the effect of oxybutynin alone at the same dose, which resulted in an 78.4% decrease in voiding efficiency. It is concluded that the addition of gabapentin (which alone at this dose caused a 10.1% increase in voiding efficiency) counteracts the undesirable negative effects of oxybutynin on voiding efficiency while simultaneously providing a positive and desirable synergistic effect on increasing bladder capacity.

The ability of an α₂δ subunit calcium channel modulator in combination with an antimuscarinic to produce a dramatic reversal in acetic acid irritation-induced reduction in bladder capacity strongly indicates efficacy in mammalian forms of painful and non-painful lower urinary tract disorders and associated irritative symptoms in normal and spinal cord injured patients. Furthermore, the combination of an α₂δ subunit calcium channel modulator and an antimuscarinic produced a synergistic effect that was greater than what would be expected if the effects were simply additive. In addition, the ability of an α₂δ subunit calcium channel modulator to counteract negative side effects of an antimuscarinic while simultaneously producing a synergistic positive effect on bladder overactivity strongly suggests efficacy in relieving the irritative symptoms without compromising voiding capability in bladder outlet obstructed patients, such as those suffering from benign prostatic hyperplasia and associated irritative symptoms.

### Example 9 - Spinal Cord Injury Model: Gabapentin and Oxybutynin

### Objective and Rationale

The objective of this study was to determine the ability of an α₂δ subunit calcium channel modulator in combination with an antimuscarinic on the ability to increase bladder capacity in spinal cord injured (SCI) rats, a commonly used model of neurogenic bladder. In particular, the current study utilized gabapentin as an exemplary α₂δ subunit calcium channel modulator, and oxybutynin as an exemplary antimuscarinic.

### Materials and Methods

Awake restrained SCI female rats were treated with combinations of oxybutynin and gabapentin (n=3). Cumulative dose-response protocols were utilized with half log increments for all studies.

### Drugs and Preparation

Drugs were dissolved in normal saline at 1, 3 and 10 mg/ml for oxybutynin and 30, 100 and 300 mg/ml for gabapentin. In these studies, combinations may be subsequently referred to as Low, Mid and High.

### Awake Restrained SCI In Vivo Model

***Animal Preparation:*** Female rats (250-300 g body weight) were anesthetized with 4% isofluorane (2% maintenance) and a laminectomy was performed at the T9-10 spinal level. The spinal cord was completely transected, and the wound was closed in layers. The animals received antibiotic (100 mg/kg ampicillin) immediately thereafter and every third day during recovery until the day of terminal experimentation. SCI rats had their bladders manually expressed twice daily by external crede, and were maintained in single housing for 2-3 weeks until evidence of recovery of voiding function was seen. On the day of the experiment, the animals were anesthetized with 4% isofluorane (2% maintenance) and a saline-filled catheter (PE-50) was inserted into the jugular vein for intravenous drug administration. This catheter was exited via the midscapular region and the ventral wound was closed with silk. Via a midline lower abdominal incision, a flared-tipped PE 50 catheter was inserted into the bladder dome for bladder filling and pressure recording. The abdominal cavity was closed in layers, with the bladder catheter exiting at the apex of the wound. Fine silver or stainless steel wire electrodes were inserted into the external urethral sphincter (EUS) percutaneously for electromyography (EMG). The animal was mounted in a Ballman restraint cage and allowed to recover from anesthesia for 1 hour prior to collection of control data.

***Experimental Design:*** Saline was continuously infused at a rate of 0.100 ml/min via the bladder-filling catheter for 60 minutes to obtain a baseline of lower urinary tract activity (continuous cystometry; CMG). Following the control period, 3 vehicle injections were made at 20 minute intervals to determine vehicle effects, if any. Subsequently, increasing doses of a selected active agent, or combination of agents, at half log increments were administered intravenously at 30 minute intervals in order to construct a cumulative dose-response relationship. At the end of the control cystometry period, the third vehicle (Veh 3), and 20 minutes following each subsequent treatment, the infusion pump was stopped, the bladder was emptied by fluid withdrawal via the infusion catheter and a single filling cystometrogram was performed at the same flow rate in order to determine changes in bladder capacity, as determined by a voiding contraction, caused by the intravenous drug administration.

### Data Analysis

Bladder capacity data for each animal was normalized to % Veh 3, and data were analyzed using a non-parametric repeated measures 1-Way ANOVA (Friedman Test) with the Dunn's Multiple Comparison Post-test. P<0.05 was considered significant.

### Results and Conclusions

The effect of cumulative increasing doses of the combination of oxybutynin and gabapentin (e.g. Dose 1 for the combination was 30 mg/kg gabapentin and 1 mg/kg oxybutynin; n=3) on bladder capacity in chronic SCI rats is depicted in Figure 21. Note the marked dose-dependent increase in bladder capacity (P=0.0278). Data are normalized to vehicle controls and are presented as Mean ± SEM:

The effect of cumulative increasing doses of the combination of oxybutynin and gabapentin (n=3) on bladder instability, as measured by a significant decrease in the number of non-voiding contractions greater than 8 cm H₂O (P=0.0174), is depicted in Figure 22. Data are presented as Mean ± SEM.

The effect of cumulative increasing doses of the combination of oxybutynin and gabapentin (n=3) on bladder instability, as measured by the significant increase in latency to the appearance of non-voiding contractions (P=0.0017), is depicted in Figure 23. Data are presented as Mean ± SEM.

The combination of an α₂δ subunit calcium channel modulator and an antimuscarinic was capable of nearly doubling bladder capacity and significantly reduced bladder instability in a rat model of neurogenic bladder. This finding stands in contrast to the effects of vanilloid agents, such as capsaicin, which have been shown to reduce bladder instability in SCI rats, but not effect bladder capacity to voiding *(Cheng et al.,* 1995, *Brain Res.* 678:40-48). Because both spinal cord injury and benign prostatic hyperplasia are characterized by outlet obstruction, bladder hypertrophy and bladder instability, these findings strongly indicate efficacy for both spinal cord injury and benign prostatic hyperplasia, including irritative symptoms and/or obstructive symptoms associated with benign prostatic hyperplasia.

## Claims

1. Use of an α₂δ subunit calcium channel modulator which is gabapentin or pregabalin or a pharmaceutically acceptable, pharmacologically active acid, salt, ester or amide thereof in the manufacture of a medicament for treating a symptom of a lower urinary tract disorder in combination with an antimuscarinic.

2. Use of an antimuscarinic in the manufacture of a medicament for treating a symptom of a lower urinary tract disorder in combination with an α₂δ subunit calcium channel modulator which is gabapentin or pregabalin or a pharmaceutically acceptable, pharmacologically active acid, salt, ester or amide thereof.

3. Use according to claim 1 or 2, wherein the α₂δ subunit calcium channel modulator is gabapentin or pregabalin.

4. Use according to any of claims 1 to 3, wherein the antimuscarinic is selected from oxybutynin, tolterodine, propiverine, and solifenacin monohydrochloride.

5. Use according to any of claims 1 to 4, wherein said α₂δ subunit calcium channel modulator and said antimuscarinic are contained within a single pharmaceutical formulation.

6. Use according to any of claims 1 to 4, wherein said α₂δ subunit calcium channel modulator and said antimuscarinic are contained within separate pharmaceutical formulations, and wherein said α₂δ subunit calcium channel modulator and said antimuscarinic are for administration concurrently or sequentially.

7. Use according to any of claims 1 to 6 wherein the symptom of a lower urinary tract disorder is urinary frequency, urinary urgency, or nocturia.

8. Use according to any of claims 1 to 6 wherein the symptom of a lower urinary tract disorder is incontinence.

9. Use according to any of claims 1 to 8 wherein at least one detrimental side effect associated with single administration of said α₂δ subunit calcium channel modulator or single administration of said antimuscarinic is lessened by concurrent administration of said α₂δ subunit calcium channel modulator and said antimuscarinic.

10. A pharmaceutical composition comprising an α₂δ subunit calcium channel modulator which is gabapentin or pregabalin or a pharmaceutically acceptable, pharmacologically active acid, salt, ester or amide thereof and an antimuscarinic.

11. A pharmaceutical composition according to claim 10, wherein the α₂δ subunit calcium channel modulator is gabapentin or pregabalin.

12. A pharmaceutical composition according to claim 10 or claim 11, wherein the antimuscarinic is selected from oxybutynin, tolterodine, propiverine, and solifenacin monohydrochloride.

13. A pharmaceutical composition according to any of claims 10 to 12, wherein the α₂δ subunit calcium channel modulator is present in an amount from about 50 mg to about 2400 mg, and wherein the antimuscarinic is present in an amount equal to or less than about 5 mg.

14. A pharmaceutical composition according to claim 13 wherein the α₂δ subunit calcium channel modulator is in an amount of about 200 mg.

15. A pharmaceutical composition according to claim 13 wherein said antimuscarinic is in an amount of about 2.5 mg.

16. A pharmaceutical composition according to claim 13 wherein said antimuscarinic is in an amount of about 1.25 mg.

17. A pharmaceutical composition according to any of claims 10 to 16, wherein said α₂δ subunit calcium channel modulator is gabapentin or a pharmaceutically acceptable, pharmacologically active acid, salt, ester or amide thereof and said antimuscarinic is oxybutynin or a pharmaceutically acceptable acid, salt, ester or amide thereof, and wherein said α₂δ subunit calcium channel modulator and said antimuscarinic are present in a ratio from about 1:1 to about 800:1 or from about 1:1 to about 1:800, respectively, based on a fraction of their respective ED₅₀ values.

18. A pharmaceutical composition according to any of claims 10 to 16, wherein said α₂δ subunit calcium channel modulator is gabapentin or a pharmaceutically acceptable, pharmacologically active acid, salt, ester or amide thereof and said antimuscarinic is oxybutynin or a pharmaceutically acceptable acid, salt, ester or amide thereof, and wherein said α₂δ subunit calcium channel modulator and said antimuscarinic are in a weight/weight ratio of from 1:1 to about 800:1 or from about 1:1 to about 1:800, respectively.

19. A packaged kit for use in the treatment of a symptom of a lower urinary tract disorder, comprising:
(a) an α₂δ subunit calcium channel modulator which is gabapentin or pregabalin or a pharmaceutically acceptable, pharmacologically active acid, salt, ester or amide thereof;
(b) an antimuscarinic;
(c) a container housing said α₂δ subunit calcium channel modulator and said antimuscarinic during storage and prior to administration; and
(d) instructions for carrying out drug administration of said α₂δ subunit calcium channel modulator and said antimuscarinic in a manner effective to treat said symptom of a lower urinary tract disorder.

20. A product containing an α₂δ subunit calcium channel modulator which is gabapentin or pregabalin or a pharmaceutically acceptable, pharmacologically active acid, salt, ester or amide thereof and an antimuscarinic for concurrent or sequential use in the treatment of a symptom of a lower urinary tract disorder.

21. A product according to claim 20 wherein the α2δ subunit calcium channel modulator is gabapentin or pregabalin.

22. A product according to claim 21 wherein the antimuscarinic is selected from oxybutynin, tolterodine, propiverine, and solifenacin monohydrochloride.

23. A product according to any one of claims 20 to 22 wherein the symptom of a lower urinary tract disorder is urinary frequency, urinary urgency, or nocturia.

24. A product according to any one of claims 20 to 22 wherein the symptom of a lower urinary tract disorder is incontinence.

## Patentansprüche

1. Verwendung eines Modulators der α₂δ-Untereinheit des Calciumskanals, der Gabapentin oder Pregabalin oder ein(e) pharmazeutisch verträgliche(s), pharmakologisch wirksame(s) Säure, Salz, Ester oder Amid davon ist, bei der Herstellung eines Medikaments zur Behandlung eines Symptoms einer Erkrankung der unteren Harnwege in Kombination mit einem Antimuskarinikum.

2. Verwendung eines Antimuskarinikums bei der Herstellung eines Medikaments zur Behandlung eines Symptoms einer Erkrankung der unteren Harnwege in Kombination mit einem Modulator der α₂δ-Untereinheit des Calciumskanals, der Gabapentin oder Pregabalin oder ein(e) pharmazeutisch verträgliche(s), pharmakologisch wirksame(s) Säure, Salz, Ester oder Amid davon ist.

3. Verwendung nach Anspruch 1 oder 2, wobei der Modulator der α₂δ-Untereinheit des Calciumskanals Gabapentin oder Pregabalin ist.

4. Verwendung nach einem der Ansprüche 1 bis 3, wobei das Antimuskarinikum aus Oxybutynin, Tolterodin, Propiverin und Solifenacin-Monohydrochlorid ausgewählt ist.

5. Verwendung nach einem der Ansprüche 1 bis 4, wobei der Modulator der α₂δ-Untereinheit des Calciumkanals und das Antimuskarinikum in einer einzigen pharmazeutischen Formulierung enthalten sind.

6. Verwendung nach einem der Ansprüche 1 bis 4, wobei der Modulator der α₂δ-Untereinheit des Calciumkanals und das Antimuskarinikum in getrennten pharmazeutischen Formulierungen enthalten sind und wobei der Modulator der α₂δ-Untereinheit des Calciumkanals und das Antimuskarinikum für gleichzeitige oder sequenzielle Verabreichung sind.

7. Verwendung nach einem der Ansprüche 1 bis 6, wobei das Symptom einer Erkrankung der unteren Harnwege Harnfrequenz, Harndrang oder Nokturie ist.

8. Verwendung nach einem der Ansprüche 1 bis 6, wobei das Symptom einer Erkrankung der unteren Harnwege Inkontinenz ist.

9. Verwendung nach einem der Ansprüche 1 bis 8, wobei wenigstens eine schädliche Nebenwirkung, die mit Einzelverabreichung des Modulators der α₂δ-Untereinheit des Calciumkanals oder Einzelverabreichung des Antimuskarinikums verbunden ist, durch gleichzeitige Verabreichung des Modulators der α₂δ-Untereinheit des Calciumkanals und des Antimuskarinikums verringert wird.

10. Pharmazeutische Zusammensetzung, umfassend einen Modulator der α₂δ-Untereinheit des Calciumkanals, der Gabapentin oder Pregabalin oder ein(e) pharmazeutisch verträgliche(s), pharmakologisch wirksame(s) Säure, Salz, Ester oder Amid davon ist, und ein Antimuskarinikum.

11. Pharmazeutische Zusammensetzung nach Anspruch 10, wobei der Modulator der α₂δ-Untereinheit des Calciumkanals Gabapentin oder Pregabalin ist.

12. Pharmazeutische Zusammensetzung nach Anspruch 10 oder 11, wobei das Antimuskarinikum aus Oxybutynin, Tolterodin, Propiverin und Solifenacin-Monohydrochlorid ausgewählt ist.

13. Pharmazeutische Zusammensetzung nach einem der Ansprüche 10 bis 12, wobei der Modulator der α₂δ-Untereinheit des Calciumkanals in einer Menge von etwa 50 mg bis etwa 2400 mg vorliegt und wobei das Antimuskarinikum in einer Menge vorliegt, die gleich oder weniger als etwa 5 mg ist.

14. Pharmazeutische Zusammensetzung nach Anspruch 13, wobei der Modulator der α₂δ-Untereinheit des Calciumkanals in einer Menge von etwa 200 mg ist.

15. Pharmazeutische Zusammensetzung nach Anspruch 13, wobei das Antimuskarinikum in einer Menge von etwa 2,5 mg ist.

16. Pharmazeutische Zusammensetzung nach Anspruch 13, wobei das Antimuskarinikum in einer Menge von etwa 1,25 mg ist.

17. Pharmazeutische Zusammensetzung nach einem der Ansprüche 10 bis 16, wobei der Modulator der α₂δ-Untereinheit des Calciumkanals Gabapentin oder ein(e) pharmazeutisch verträgliche(s), pharmakologisch wirksame(s) Säure, Salz, Ester oder Amid davon ist, und das Antimuskarinikum Oxybutynin oder ein(e) pharmazeutisch verträgliche(s) Säure, Salz, Ester oder Amid davon ist und wobei der Modulator der α₂δ-Untereinheit des Calciumkanals und das Antimuskarinikum in einem Verhältnis von etwa 1:1 bis etwa 800:1 beziehungsweise von etwa 1:1 bis etwa 1:800, auf der Basis eines Bruchs ihrer jeweiligen ED₅₀-Werte, vorliegen.

18. Pharmazeutische Zusammensetzung nach einem der Ansprüche 10 bis 16, wobei der Modulator der α₂δ-Untereinheit des Calciumkanals Gabapentin oder ein(e) pharmazeutisch verträgliche(s), pharmakologisch wirksame(s) Säure, Salz, Ester oder Amid davon ist und das Antimuskarinikum Oxybutynin oder ein(e) pharmazeutisch verträgliche(s) Säure, Salz, Ester oder Amid davon ist, und wobei der Modulator der α₂δ-Untereinheit des Calciumkanals und das Antimuskarinikum in einem Gewicht/Gewicht-Verhältnis von 1:1 bis etwa 800:1 beziehungsweise von etwa 1:1 bis etwa 1:800 sind.

19. Ein verpackter Kit zur Verwendung bei der Behandlung eines Symptoms einer Erkrankung der unteren Harnwege, umfassend:
(a) einen Modulator der α₂δ-Untereinheit des Calciumkanals, der Gabapentin oder Pregabalin oder ein(e) pharmazeutisch verträgliche(s), pharmakologisch wirksame(s) Säure, Salz, Ester oder Amid davon ist;
(b) ein Antimuskarinikum;
(c) einen Behälter, der den Modulator der α₂δ-Untereinheit des Calciumkanals und das Antimuskarinikum während Lagerung und vor Verabreichung aufnimmt und
(d) Anweisungen zur Durchführung der Arzneimittelverabreichung des Modulators der α₂δ-Untereinheit des Calciumkanals und des Antimuskarinikums in einer Weise, die wirksam ist, um das Symptom einer Erkrankung der unteren Harnwege zu behandeln.

20. Erzeugnis, enthaltend einen Modulator der α₂δ-Untereinheit des Calciumkanals, der Gabapentin oder Pregabalin oder ein(e) pharmazeutisch verträgliche(s), pharmakologisch wirksame(s) Säure, Salz, Ester oder Amid davon ist, und ein Antimuskarinikum zur gleichzeitigen oder sequenziellen Verwendung bei der Behandlung eines Symptoms einer Erkrankung der unteren Harnwege.

21. Erzeugnis nach Anspruch 20, wobei der Modulator der α₂δ-Untereinheit des Calciumkanals Gabapentin oder Pregabalin ist.

22. Erzeugnis nach Anspruch 21, wobei das Antimuskarinikum aus Oxybutynin, Tolterodin, Propiverin und Solifenacin-Monohydrochlorid ausgewählt ist.

23. Erzeugnis nach einem der Ansprüche 20 bis 22, wobei das Symptom einer Erkrankung der unteren Harnwege Harnfrequenz, Harndrang oder Nokturie ist.

24. Erzeugnis nach einem der Ansprüche 20 bis 22, wobei das Symptom einer Erkrankung der unteren Harnwege Inkontinenz ist.

## Revendications

1. Utilisation d'un modulateur d'une sous unité α₂δ du canal calcique, qui est le gabapentin ou la prégabaline ou un acide, sel, ester ou amide pharmaceutiquement acceptable, pharmacologiquement actif de celles-ci, dans la préparation d'un médicament pour le traitement d'un symptôme d'un désordre du tractus urinaire inférieur, en combinaison avec un antimuscarinique.

2. Utilisation d'un antimuscarinique dans la préparation d'un médicament pour le traitement d'un symptôme d'un désordre du tractus urinaire inférieur, en combinaison avec un modulateur d'une sous unité α₂δ du canal calcique, qui est le gabapentin ou la prégabaline ou un acide, sel, ester ou amide pharmaceutiquement acceptable, pharmacologiquement actif de celles-ci.

3. Utilisation selon la revendication 1 ou 2, dans laquelle le modulateur d'une sous unité α₂δ du canal calcique est le gabapentin ou la prégabaline.

4. Utilisation selon l'une quelconque des revendications 1 à 3, dans laquelle l'antimuscarinique est choisi parmi l'oxybutynine, la toltérodine, la propivérine et le monochlorhydrate de solifénacine.

5. Utilisation selon l'une quelconque des revendications 1 à 4, dans laquelle ledit modulateur d'une sous unité α₂δ du canal calcique et ledit antimuscarinique sont présents dans une seule formulation pharmaceutique.

6. Utilisation selon l'une quelconque des revendications 1 à 4, dans laquelle ledit modulateur d'une sous unité α₂δ du canal calcique et ledit antimuscarinique sont présents dans des formulations pharmaceutiques séparées, et dans laquelle ledit modulateur d'une sous unité α₂δ du canal calcique et ledit antimuscarinique sont prévus pour une administration simultanée ou séquentielle.

7. Utilisation selon l'une quelconque des revendications 1 à 6, dans laquelle le symptôme d'un désordre du tractus urinaire inférieur est la fréquence urinaire, l'urgence urinaire ou la polyurie nocturne.

8. Utilisation selon l'une quelconque des revendications 1 à 6, dans laquelle le symptôme d'un désordre du tractus urinaire inférieur est l'incontinence.

9. Utilisation selon l'une quelconque des revendications 1 à 8, dans laquelle au moins un effet secondaire associé à l'administration seule dudit modulateur d'une sous unité α₂δ du canal calcique ou à l'administration seule dudit antimuscarinique est diminué par l'administration simultanée dudit modulateur d'une sous unité α₂δ du canal calcique et dudit antimuscarinique.

10. Composition pharmaceutique comprenant un modulateur d'une sous unité α₂δ du canal calcique, qui est le gabapentin ou la prégabaline, ou un acide, sel, ester ou amide pharmaceutiquement acceptable, pharmacologiquement actif de celles-ci, et un antimuscarinique.

11. Composition pharmaceutique selon la revendication 10, dans laquelle le modulateur d'une sous unité α₂δ du canal calcique est le gabapentin ou la prégabaline.

12. Composition pharmaceutique selon la revendication 10 ou 11, dans laquelle l'antimuscarinique est choisi parmi l'oxybutynine, la toltérodine, la propivérine et le monochlorhydrate de solifénacine.

13. Composition pharmaceutique selon l'une quelconque des revendications 10 à 12, dans laquelle le modulateur d'une sous unité α₂δ du canal calcique est présent en une quantité allant d'environ 50 mg à environ 2400 mg et dans laquelle l'antimuscarinique est présent en une quantité égale ou inférieure à environ 5 mg.

14. Composition pharmaceutique selon la revendication 13, dans laquelle le modulateur d'une sous unité α₂δ du canal calcique est présent en une quantité d'environ 200 mg.

15. Composition pharmaceutique selon la revendication 13, dans laquelle l'antimuscarinique est présent en une quantité d'environ 2,5 mg.

16. Composition pharmaceutique selon la revendication 13, dans laquelle l'antimuscarinique est présent en une quantité d'environ 1,25 mg.

17. Composition pharmaceutique selon l'une quelconque des revendications 10 à 16, dans laquelle ledit modulateur d'une sous unité α₂δ du canal calcique est le gabapentin ou un acide, sel, ester ou amide pharmaceutiquement acceptable, pharmacologiquement actif de celle-ci, et ledit antimuscarinique est l'oxybutynine ou un acide, sel, ester ou amide pharmaceutiquement acceptable de celle-ci et dans laquelle ledit modulateur d'une sous unité α₂δ du canal calcique et ledit antimuscarinique sont présents dans un rapport allant d'environ 1:1 à environ 800:1 ou d'environ 1:1 à environ 1:800, respectivement, sur la base d'une fraction de leurs valeurs DE₅₀ respectives.

18. Composition pharmaceutique selon l'une quelconque des revendications 10 à 16, dans laquelle ledit modulateur d'une sous unité α₂δ du canal calcique est le gabapentin ou un acide, sel, ester ou amide pharmaceutiquement acceptable, pharmacologiquement actif de celle-ci, et ledit antimuscarinique est l'oxybutynine ou un acide, sel, ester ou amide pharmaceutiquement acceptable de celle-ci et dans laquelle ledit modulateur d'une sous unité α₂δ du canal calcique et ledit antimuscarinique sont présents en un rapport poids/poids allant d'environ 1:1 à environ 800:1 ou d'environ 1:1 à environ 1:800, respectivement.

19. Kit emballé à utiliser dans le traitement d'un symptôme d'un désordre du tractus urinaire inférieur, comprenant :
(a) un modulateur d'une sous unité α₂δ du canal calcique, qui est le gabapentin ou la prégabaline, ou un acide, sel, ester ou amide pharmaceutiquement acceptable, pharmacologiquement actif de cellesi-ci ;
(b) un antimuscarinique ;
(c) un récipient contenant ledit modulateur d'une sous unité α₂δ du canal calcique et ledit antimuscarinique pendant le stockage et avant l'administration, et
(d) les instructions pour réaliser l'administration médicamenteuse dudit modulateur d'une sous unité α₂δ du canal calcique et dudit antimuscarinique, de manière efficace pour traiter ledit symptôme d'un désordre du tractus urinaire inférieur.

20. Produit contenant un modulateur d'une sous unité α₂δ du canal calcique, qui est le gabapentin ou la prégabaline, ou un acide, sel, ester ou amide pharmaceutiquement acceptable, pharmacologiquement actif de celles-ci, et un antimuscarinique, pour une utilisation simultanée ou séquentielle dans le traitement d'un symptôme d'un désordre du tractus urinaire inférieur.

21. Produit selon la revendication 20, dans lequel le modulateur d'une sous unité α₂δ du canal calcique est le gabapentin ou la prégabaline.

22. Produit selon la revendication 21, dans lequel l'antimuscarinique est choisi parmi l'oxybutynine, la toltérodine, la propivérine et le monochlorhydrate de solifénacine.

23. Produit selon l'une quelconque des revendications 20 à 22, dans lequel le symptôme d'un désordre du tractus urinaire inférieur est la fréquence urinaire, l'urgence urinaire ou la polyurie nocturne.

24. Produit selon l'une quelconque des revendications 20 à 22, dans lequel le symptôme d'un désordre du tractus urinaire inférieur est l'incontinence.
